(19) Europäisches Patentamt
European Patent Office
Office européen des brevets

(11) Veröffentlichungsnummer : **0 015 523**
**B1**

(12) # EUROPÄISCHE PATENTSCHRIFT

(45) Veröffentlichungstag der Patentschrift : **13.10.82**

(21) Anmeldenummer : **80101037.2**

(22) Anmeldetag : **03.03.80**
**Die Akte enthält technische Angaben, die nach dem Eingang der Anmeldung eingereicht wurden und die nicht in dieser Patentschrift enthalten sind.**

(51) Int. Cl.³ : **C 07 D209/08**, C 07 D209/10, C 07 D209/12, C 07 D401/04, C 07 D401/06, C 07 D471/04, C 07 D487/04, A 61 K 31/40 // (C07D471/04, 221/00, 209/00),(C07D487/04, 223/00, 209/00),(C07D487/04, 209/00, 209/00)

(54) **Neue Indolderivate, deren Herstellung und Verwendung für Arzneimittel.**

(30) Priorität : **13.03.79 DE 2909779**

(43) Veröffentlichungstag der Anmeldung : **17.09.80 (Patentblatt 80/19)**

(45) Bekanntmachung des Hinweises auf die Patenterteilung : **13.10.82 Patentblatt 82/41**

(84) Benannte Vertragsstaaten :
**AT BE CH DE FR GB IT LU NL SE**

(56) Entgegenhaltungen :
**DE A 2 237 361**
**DE A 2 527 937**
**Chemical Abstracts, Band 67, Nr. 19, 6. November 1967 Columbus, Ohio, USA A.N. GRINEV et al. « The synthesis of derivatives of indol-5-yloxyacetic acids » Seite 8 520, Spalte 2, Abstract Nr. 90603j**

(73) Patentinhaber : **Dr. Karl Thomae GmbH**
**Postfach 720**
**D-7950 Biberach (Riss) (DE)**

(72) Erfinder : **Hurnaus, Rudolf, Dr. Dipl.-Chem.**
**Silcherstrasse 19**
**D-7950 Biberach 1 (DE)**
Erfinder : **Griss, Gerhart, Dr. Dipl.-Chem.**
**Schopperweg 1**
**D-7950 Biberach 1 (DE)**
Erfinder : **Grell, Wolfgang, Dr. Dipl.-Chem.**
**Amriswilstrasse 7**
**D-7950 Biberach 1 (DE)**
Erfinder : **Sauter, Robert, Dr. Dipl.-Chem.**
**Albert-Schweitzer Weg 9**
**D-7958 Laupheim (DE)**
Erfinder : **Eisele, Bernhard, Dr. Dipl.-Chem.**
**Beethovenstrasse 12**
**D-7950 Biberach 1 (DE)**
Erfinder : **Kaubisch, Nikolaus, Dr. Dipl.-Chem.**
**Dinglingerstrasse 45**
**D-7950 Biberach 1 (DE)**
Erfinder : **Rupprecht, Eckhard, Dr. Dipl.-Biol.**
**D-7951 Winterstettenstadt (DE)**
Erfinder : **Kähling, Joachim, Dr.**
**Haydnweg 8**
**D-7950 Biberach 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 015 523 B1

Imprimerie Jouve, 18, rue St-Denis, 75001 Paris, France

# Neue Indolderivate, deren Herstellung und Verwendung für Arzneimittel

In der DE-A1-2.237.361 werden bereits (5-Indolyloxy)-essigsäure-Derivate beschrieben, welche in 2-Stellung durch ein Wasserstoffatom, eine Carboxy- oder Alkoxycarbonylgruppe substituiert sein können. Diese Verbindungen senken den Cholesterin- und Triglyceridspiegel im Blutserum.

Es wurde nun gefunden, daß die neuen Indolderivate der allgemeinen Formel

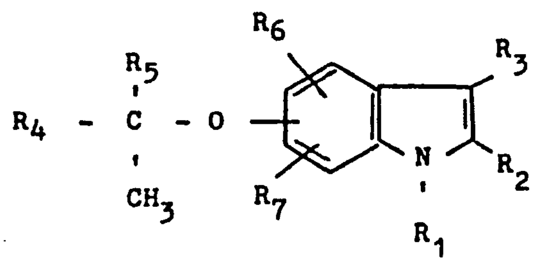

(I)

welche sich durch ihre Substituenten in 2- und/oder 3-Stellung von den in der DE-A1-2.237.361 beschriebenen Indolderivaten unterscheiden, überlegene biologische Eigenschaften aufweisen.

Gegenstand der vorliegenden Anmeldung sind somit die neuen Indolderivate der obigen allgemeinen Formel I und deren physiologisch verträgliche Salze mit anorganischen oder organischen Säuren und Basen, welche wertvolle pharmakologische Eigenschaften aufweisen, insbesondere eine lipidsenkende und antiatherosklerotische Wirkung, Verfahren zu ihrer Herstellung und deren Verwendung als Arzneimittel.

In der obigen allgemeinen Formel I bedeuten

$R_1$ ein Wasserstoffatom, eine geradkettige oder verzweigte Alkylgruppe mit 1 bis 12 Kohlenstoffatomen oder eine Alkenylgruppe mit 3 bis 5 Kohlenstoffatomen,

$R_2$ eine Alkylgruppe mit 1 bis 3 Kohlenstoffatomen, die durch eine Phenylgruppe substituiert sein kann, eine gegebenenfalls durch ein Halogenatom, eine Methoxy-, Amino-, Nitro- oder Acetamidogruppe substituierte Phenylgruppe, eine Pyridylgruppe oder $R_1$ zusammen mit $R_2$ eine Alkylengruppe mit 3 bis 5 Kohlenstoffatomen,

$R_3$ eine Alkylgruppe mit 1 bis 3 Kohlenstoffatomen, die durch eine gegebenenfalls durch eine Methylgruppe, Methoxygruppe, Trifluormethylgruppe oder ein Halogenatom substituierte Phenylgruppe oder eine Pyridylgruppe substituiert sein kann, eine gegebenenfalls durch eine Methylgruppe, Methoxygruppe, Trifluormethylgruppe oder ein Halogenatom substituierte Phenylgruppe, eine Pyridyl-, Pyridyl-N-oxyd- oder Chinolylgruppe oder einer der Reste $R_2$ oder $R_3$ auch ein Wasserstoffatom, wenn der andere der Reste $R_2$ oder $R_3$ jeweils einen der bei der Definition der Reste $R_2$ oder $R_3$ erwähnten aromatischen, araliphatischen, heteroaromatischen oder heteroaraliphatischen Rest darstellt,

$R_4$ eine Carboxyl- oder Nitrilgruppe, eine Trialkoxymethylgruppe, in der jeder Alkoxyrest 1 bis 3 Kohlenstoffatome enthalten kann, eine Carbalkoxygruppe mit insgesamt 2 bis 8 Kohlenstoffatomen, eine gegebenenfalls durch Alkylgruppen mit 1 bis 7 Kohlenstoffatomen, Cycloalkylgruppen mit 3 bis 7 Kohlenstoffatomen oder Alkenylgruppen mit 3 bis 7 Kohlenstoffatomen mono- oder disubstituierte Aminocarbonylgruppe, wobei die Substituenten der Aminogruppe gleich oder verschieden sein können, eine Hydroxymethyl-, Piperidinocarbonyl-, Morpholinocarbonyl- oder Thiomorpholinocarbonylgruppe,

$R_5$ eine Alkylgruppe mit 1 bis Kohlenstoffatomen,

$R_6$ ein Wasserstoffatom, eine Methyl- oder Benzylgruppe und

$R_7$ ein Wasserstoffatom oder die Methylgruppe.

Unter dem bei der Definition der Reste $R_2$ und $R_3$ erwähnten Ausdruck «ein Halogenatom» ist insbesondere ein Fluor-, Chlor-, Brom- oder Jodatom zu verstehen.

Für die bei der Definition der Reste $R_1$ bis $R_5$ eingangs erwähnten Bedeutungen kommen somit

für $R_1$ die Bedeutung des Wasserstoffatoms, der Methyl-, Äthyl-, Propyl-, Isopropyl-, Butyl-, Isobutyl-, sec. Butyl-, tert. Butyl-, Pentyl-, Isopentyl-, sec. Pentyl-, Neopentyl-, Hexyl-, Heptyl-, Octyl-, Decyl-, Dodecyl-, Allyl-, Crotyl- oder 2-Penten-1-yl-gruppe,

für $R_2$ die der Methyl-, Äthyl-, Propyl-, Isopropyl-, Benzyl-, 1-Phenyläthyl-, 1-Phenylpropyl-, 2-Phenyläthyl-, 2-Phenylpropyl-, Pyridyl-(4)-, Pyridyl-(2)-, Phenyl-, Fluorphenyl-, Chlorphenyl-, Bromphenyl-, Jodphenyl-, Methoxyphenyl-, Nitrophenyl-, Aminophenyl- oder Acetamidophenylgruppe,

für $R_1$ zusammen mit $R_2$ die Propylen-, Butylen- oder Pentylengruppe,

für $R_3$ die der Methyl-, Äthyl-, propyl-, Isopropyl-, Benzyl-, Fluorbenzyl-, Chlorbenzyl-, Brombenzyl-, Methoxybenzyl-, 1-Phenyläthyl-, 2-Phenyläthyl-, 2-(Fluorphenyl)äthyl-, 2-(Chlorphenyl)äthyl-, 2-(Bromphenyl)äthyl-, 2-(Methoxyphenyl)äthyl-, 2-(Fluorphenyl)propyl-, 2-(Chlorphenyl)propyl-, 3-(Bromphenyl)-propyl-, 3-(Methoxyphenyl)propyl-, Phenyl-, Methylphenyl-, Methoxyphenyl-, Trifluormethylphenyl-, Fluorphenyl-, Chlorphenyl-, Bromphenyl-, Pyridyl-(2)-, Pyridyl-(4)-, Pyridyl-(4)-N-oxyd-, Chinolyl-(2)-, 4-Pyridylmethyl- oder 2-Pyridylmethylgruppe oder auch für einen der Reste $R_2$ oder $R_3$ die des Wasserstoff atoms, wenn der andere der Reste $R_2$ oder $R_3$ jeweils einen der bei der Definition der Reste $R_2$ oder $R_3$

oben erwähnten aromatischen, araliphatischen, heteroaromatischen oder heteroaraliphatischen Rest darstellt,

für $R_4$ die der Carboxyl-, Nitril-, Tripropoxymethyl-, Trimethoxymethyl-, Triäthoxymethyl-, Methoxycarbonyl-, Äthoxycarbonyl-, Propoxycarbonyl-, Isopropoxycarbonyl-, Butoxycarbonyl-, Isobutoxycarbonyl-, sec. Butoxycarbonyl-, tert. Butoxycarbonyl-, Pentoxycarbonyl-, Isopentoxycarbonyl-, sec. Pentoxycarbonyl-, Neopentoxycarbonyl-, Hexoxycarbonyl-, Heptoxycarbonyl-, Hydroxymethyl-, Aminocarbonyl-, Methylaminocarbonyl-, Dimethylaminocarbonyl-, Äthylaminocarbonyl-, Diäthylaminocarbonyl-, Propylaminocarbonyl-, Pentylaminocarbonyl-, Heptylaminocarbonyl-, Cyclopropylaminocarbonyl-, Cyclohexylaminocarbonyl-, Dicyclohexylaminocarbonyl-, Cycloheptylaminocarbonyl-, Allylaminocarbonyl-, Diallylaminocarbonyl-, Crotonylaminocarbonyl-, pent-2-enylaminocarbonyl-, hept-2-enylaminocarbonyl-, Piperidinocarbonyl-, Morpholinocarbonyl- oder Thiomorpholinocarbonylgruppe und

für $R_5$ die der Methyl-, Äthyl-, Propyl- oder Isopropylgruppe in Betracht.

Bevorzugte Verbindungen der obigen allgemeinen Formel I sind diejenigen, in der

$R_1$ ein Wasserstoffatom, eine Alkylgruppe mit 1 bis 12 Kohlenstoffatomen wie die Methyl-, Äthyl-, Propyl-, Isopropyl-, Butyl-, Isobutyl-, Hexyl- oder Dodecylgruppe oder die Allylgruppe,

$R_2$ ein Wasserstoffatom, eine Alkylgruppe mit 1 bis 3 Kohlenstoffatomen, wie die Methyl-, Äthyl-, Propyl- oder Isorpopylgruppe, oder $R_1$ und $R_2$ zusammen die Propylen-, Butylen- oder Pentylengruppe und

$R_3$ eine gegebenenfalls durch ein Fluor-, Chlor- oder Bromatom, eine Methyl-, Methoxy- oder Trifluormethylgruppe substituierte Phenylgruppe, eine gegebenenfalls durch ein Chloratom oder eine Methoxygruppe substituierte 2-Phenyläthylgruppe, eine Benzyl-, Chlorbenzyl-, Pyridyl-(2)-, Pyridyl-(4)-, Chinolyl-(2)- oder 4-Pyridylmethylgruppe oder

$R_2$ eine gegebenenfalls durch ein Chloratom, eine Methoxy-, Nitro-, Amino- oder Acetaminogruppe substituierte Phenylgruppe, eine Benzyl- oder Pyridyl-(4)-gruppe und

$R_3$ ein Wasserstoffatom oder eine Alkylgruppe mit 1 bis 3 Kohlenstoffatomen, wie die Methyl-, Äthyl-, Propyl- oder Isopropylgruppe,

$R_4$ eine Carboxyl- oder nitrilgruppe, eine Carbalkoxygruppe mit insgesamt 2 bis 6 Kohlenstoffatomen oder eine Hydroxymethylgruppe,

$R_5$ die Methylgruppe,

$R_6$ ein Wasserstoffatom, die Methyl- oder Benzylgruppe und

$R_7$ ein Wasserstoffatom oder die Methylgruppe bedeuten, sowie deren physiologisch verträgliche Salze mit anorganischen und organischen Säuren oder Basen.

Besonders devorzugte Verbindungen der obigen allgemeinen Formel I sind jedoch diegenigen, in der

$R_1$ eine Alkylgruppe mit 1 bis 4 Kohlenstoffatomen,

$R_2$ eine Methylgruppe oder $R_1$ und $R_2$ zusammen die n-Pentylengruppe und

$R_3$ eine gegebenenfalls in 3- oder 4-Stellung durch ein Fluor- oder Chloratom, eine Methoxy- oder Trifluormethylgruppe substituierte Phenylgruppe, eine 2-Phenyläthyl-, Pyridyl-(4)- oder Chinolyl-(2)-gruppe oder

$R_2$ eine gegebênenfalls in 3- oder 4-Stellung durch ein Chloratom, eine Methoxy-, Amino- oder Acetaminogruppe substituierte Phenylgruppe oder eine Pyridyl-(4)-gruppe und

$R_3$ ein Wasserstoffatom oder eine Methylgruppe,

$R_4$ eine Carboxylgruppe oder eine Alkoxycarbonylgruppe mit insgesamt 2 bis 4 Kohlenstoffatomen,

$R_5$ die Methylgruppe,

$R_6$ und $R_7$ je ein Wasserstoffatom bedeuten, sowie deren physiologisch verträgliche Salze mit anorganischen und organischen Säuren oder Basen.

Erfindungsgemäß werden die neuen Indolderivate der allgemeinen Formel I nach folgenden verfahren erhalten :

a) Umsetzung eines Hydroxy-indols der allgemeinen Formel

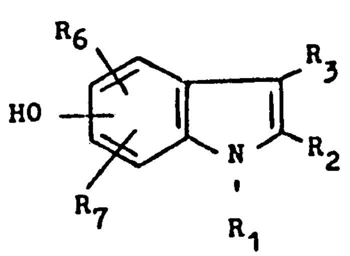

(II)

in der

$R_1$, $R_2$, $R_3$, $R_6$ und $R_7$ wie eingangs definiert sind, bzw. deren Alkalisalze mit eine Verbindung der allgemeinen Formel

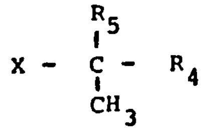 (III)

in der

$R_4$ und $R_5$ wie eingangs definiert sind und

X eine nukleophile Austrittsgruppe wie ein Halogenatom, z.B. ein Chlor-, Brom- oder Jodatom, darstellt.

Die Umsetzung wird zweckmäßigerweise in einem Lösungsmittel wie Aceton, Methyläthylketon, Toluol, Dimethylformamid, Hexamethylphosphorsäure-triamid oder Glykoldimethyläther und vorzugsweise in Gegenwart einer Base wie Kaliumkarbonat oder Natriumhydrid gegebenenfalls in Gegenwart eines Phasentransfer-Katalysators wie einem quartären Ammoniumsalz oder einem Kronenäther, z.B. 18-Krone-6 oder Tetrabutyl-ammoniumchlorid, bei Temperaturen zwischen 0 und 200 °C, vorzugsweise jedoch bei Temperaturen zwischen der Raumtemperatur und der Siedetemperatur des verwendeten Lösungsmittels, beispielsweise bei Temperaturen zwischen 20 und 100 °C, durchgeführt. Die Umsetzung kann jedoch auch in der Schmelze durchgeführt werden.

b) Zur Herstellung von Verbindungen der allgemeinen Formel I, in der $R_4$ eine Carboxylgruppe darstellt :

Umsetzung eines Hydroxy-indols der allgemeinen Formel

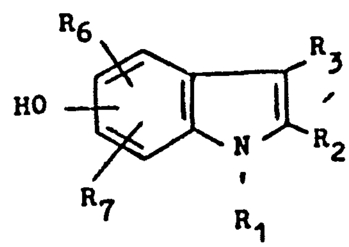 (II)

in der

$R_1$, $R_2$, $R_3$, $R_6$ und $R_7$ wie eingangs definiert sind, bzw. deren Alkalisalze mit einem Alkohol der allgemeinen Formel

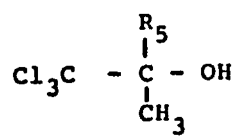 (IV)

in der

$R_5$ wie eingangs definiert ist, in Gegenwart einer anorganischen Base.

Die Umsetzung wird in Gegenwart einer Base wie Kaliumkarbonat, Natriumhydroxid oder Kaliumhydroxid vorzugsweise in einem wasserfreien Lösungsmittel der allgemeinen Formel

$$R_5 — CO — CH_3 \quad (V)$$

in der

$R_5$ wie eingangs definiert ist, zweckmäßigerweise bei Temperaturen zwischen 0 °C und der Siedetemperatur des verwendeten Lösungsmittels, beispielsweise bei Temperaturen zwischen 0 und 100 °C, durchgeführt.

Die Umsetzung kann jedoch auch in der Weise durchgeführt werden, daß eine Verbindung der allgemeinen Formel IV im Reaktionsgemisch durch Umsetzung des entsprechenden Ketons der allgemeinen Formel V mit Chloroform in Gegenwart einer anorganischen Base *in situ* hergestellt wird.

c) Zur Herstellung von Verbindungen der allgemeinen Formel I, in der $R_1$ kein Wasserstoffatom darstellt :

Alkylierung eines Indolderivates der allgemeinen Formel

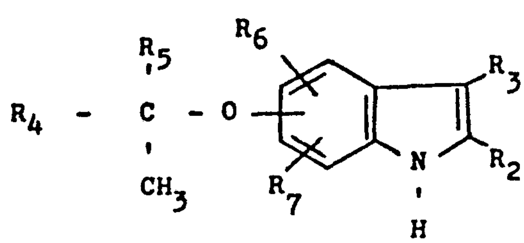 (VI)

in der

$R_2$ bis $R_7$ wie eingangs definiert sind, bzw. dessen Alkalisalze mit einer Verbindung der allgemeinen Formel

$$R_1' - Z \qquad (VII)$$

in der

$R_1'$ mit Ausnahme von Wasserstoff die für $R_1$ eingangs erwähnten Bedeutungen besitzt und

Z eine nukleophile Austrittsgruppe wie ein Halogenatom oder einen Sulfonsäurerest, z.B. ein Chlor-, Brom- oder Jodatom, die p-Toluolsulfonyloxy- oder Methoxysulfonyloxygruppe, darstellt.

Die Alkylierung wird vorzugsweise in Gegenwart einer anorganischen oder tertiären organischen Base wie Natriumkarbonat, Kaliumkarbonat, Kaliumhydroxid, Natriumhydroxid, Natriumhydrid, Pyridin, Triäthylamin oder einem Alkoholat wie Kaliumtert. butylat zweckmäßigerweise in einem Lösungsmittel wie Aceton, Methyl-äthylketon, Dimethylformamid, Dimethylsulfoxid, Hexamethyl-phosphorsäure-triamid oder Glykoldimethyläther bei Temperaturen zwischen 0 und 200 °C, vorzugsweise jedoch bei Temperaturen zwischen Raumtemperatur und der Siedetemperatur des verwendeten Lösungsmittels, z.B. bei Temperaturen zwischen 20 und 100 °C, durchgeführt. Die Umsetzung kann jedoch auch in der Schmelze durchgeführt werden.

d) Zur Herstellung von Verbindungen der allgemeinen Formel I, in der $R_4$ eine Carboxylgruppe darstellt:

Hydrolyse eines Indolderivates der allgemeinen Formel

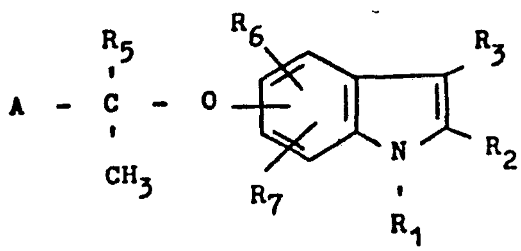

(VIII)

in der

$R_1$ bis $R_3$ und $R_5$ bis $R_7$ wie eingangs definiert sind und A eine durch Hydrolyse in eine Carboxylgruppe überführbare Gruppe darstellt.

Als eine derartige hydrolysierbare Gruppe kommt beispielsweise die Nitrilgruppe, ein funktionelles Derivat der Carboxylgruppe wie deren unsubstituierte oder substituierte Amide, Ester, Thioester, Orthoester, Iminoäther, Amidine oder Anhydride, eine Malonester-(1)-yl- oder Dihydro-1,3-oxazol-(2)-yl-gruppe in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxyd in einem geeigneten Lösungsmittel wie Äthanol, Wasser/Äthanol, Wasser/Isopropanol oder Wasser/Dioxan bei Temperaturen zwischen 50 und 150 °C, vorzugsweise jedoch bei der Siedetemperatur des Reaktionsgemisches, durchgeführt.

Bedeutet in einer verbindung der allgemeinen Formel VIII, A die Cyangruppe, so wird die Umsetzung zweckmäßigerweise in Gegenwart von Äthanol/Chlorwasserstoff durchgeführt, hierbei bildet sich im Reaktionsgemisch der entsprechende Orthoester, welcher nach Zugabe von Wasser zu dem entsprechenden Ester hydrolysiert wird. Ein so erhaltener Ester wird anschließend zu der entsprechenden Carbonsäure verseift.

e) Zur Herstellung von Verbindungen der allgemeinen Formel I, in der $R_4$ eine Carboxylgruppe darstellt:

Oxidation eines Indolderivates der allgemeinen Formel

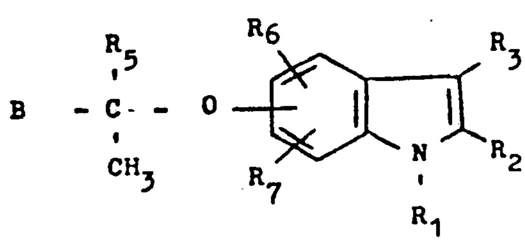

(IX)

in der

$R_1$ bis $R_3$ und $R_5$ bis $R_7$ wie eingangs definiert sind und B eine durch Oxidation in eine Carboxylgruppe überführbare Gruppe bedeutet.

Als eine derartige oxidierbare Gruppe kommt beispielsweise die Formylgruppe und deren Acetale, die Hydroxymethylgruppe und deren Äther, eine unsubstituierte oder substituierte Acylgruppe wie die Acetyl-, Chloracetyl-, Propionyl-, Malonsäure-(1)-yl- oder Malonester-(1)-yl-gruppe in Betracht.

Die Umsetzung wird mit einem Oxidationsmittel wie Chromtrioxid, Kaliumpermanganat, Wasserstoffperoxid oder mit Chlor oder Brom in Gegenwart einer anorganischen Base wie Natrium- oder Kaliumhydroxid in einem geeigneten Lösungsmittel wie Eisessig, Wasser/Eisessig, Pyridin oder Wasser bei Temperaturen zwischen 0 und 100 °C, vorzugsweise jedoch bei Temperaturen zwischen 20 und 50 °C, durchgeführt.

Erhält man erfindungsgemäß eine Verbindung der allgemeinen Formel I, in der $R_4$ eine Carboxylgruppe darstellt, so kann diese mittels Veresterung bzw. Amidierung in eine entsprechende Verbindung der allgemeinen Formel I, in der $R_4$ eine Carbalkoxygruppe mit insgesamt 2 bis 8 Kohlenstoffatomen, eine gegebenenfalls durch Alkylgruppen mit 1 bis 7 Kohlenstoffatomen, Cycloalkylgruppen mit 3 bis 7 Kohlenstoffatomen oder Alkenylgruppen mit 3 bis 7 Kohlenstoffatomen mono- oder disubstituierte Aminocarbonylgruppe, wobei die Substituenten gleich oder verschieden sein können, eine Piperidinocarbonyl-, Morpholinocarbonyl- oder Thiomorpholinocarbonylgruppe darstellt, übergeführt werden oder eine Verbindung der allgemeinen Formel I, in der $R_4$ eine Carboxylgruppe oder eine Carbalkoxygruppe mit insgesamt 2 bis 8 Kohlenstoffatomen darstellt, mittels Reduktion in eine entsprechende Verbindung der allgemeinen Formel I, in der $R_4$ die Hydroxymethylgruppe darstellt, übergeführt werden oder eine Verbindung der allgemeinen Formel I, in der $R_2$ eine Nitrophenylgruppe darstellt, so kann diese mittels Reduktion in eine entsprechende Verbindung der allgemeinen Formel I, in der $R_2$ eine Aminophenylgruppe darstellt, übergeführt werden oder eine Verbindung der allgemeinen Formel I, in der $R_2$ eine Aminophenylgruppe darstellt, so kann diese mittels Acetylierung in eine entsprechende Verbindung der allgemeinen Formel I, in der $R_2$ eine Acetamidophenylgruppe darstellt, übergeführt werden.

Die nachträgliche Überführung einer Carboxyl- oder Estergruppe in die Hydroxymethylgruppe wird vorzugsweise mit einem komplexen Metallhybrid wie Lithiumaluminiumhydrid in einem Lösungsmittel wie Äther, Tetrahydrofuran oder Dioxan bei Temperaturen zwischen 20 und 100 °C, vorzugsweise jedoch bei der Siedetemperatur des verwendeten Lösungsmittels, durchgeführt.

Die nachträgliche Veresterung oder Amidierung wird zweckmäßigerweise in Gegenwart eines wasserentziehenden und/oder säureaktivierenden Mittels wie N,N'-Dicyclohexylcarbodiimid oder Thionylchlorid vorzugsweise in einem geeigneten Lösungsmittel wie äther, Chloroform, Tetrahydrofuran, bei Temperaturen zwischen 0 und 100 °C durchgeführt.

Die nachträgliche Reduktion wird zweckmäßigerweise mit katalytisch angeregtem Wasserstoff, z.B. mit Wasserstoff in Gegenwart von Raney-Nickel oder Palladium/Kohle, oder mit nascierendem Wasserstoff, z.B. mit Zink/Salzsäure, Eisen/Salzsäure oder Zinn(II)-chlorid/Salzsäure, in einem geeigneten Lösungsmittel wie Wasser, Methanol, Wasser/Methanol, Acetanhydrid oder Eisessig bei Temperaturen zwischen 0 und 100 °C durchgeführt.

Die nachträgliche Acetylierung wird zweckmäßigerweise mit Acetylchlorid oder Acetanhydrid gegebenenfalls in Gegenwart einer anorganischen oder tertiären organischen Base und gegebenenfalls in einem Lösungsmittel wie Chloroform, Eisessig oder in einem Überschuß des verwendeten Acetylierungsmittels bei Temperaturen zwischen 0 und 100 °C durchgeführt.

Ferner lassen sich die erhaltenen Verbindungen der allgemeinen Formel I, in der $R_4$ eine Carboxylgruppe darstellt, in ihre physiologisch verträglichen Salze mit anorganischen und organischen Basen bzw. die erhaltenen Verbindungen der allgemeinen Formel I in ihre physiologisch verträglichen Salze mit anorganischen und organischen Säuren überführen. Hierfür kommen als Basen beispielsweise Natriumhydroxid, Kaliumhydroxid oder Cyclohexylamin und als Säuren Salzsäure, Bromwasserstoffsäure, Schwefelsäure, Phosphorsäure, Milchsäure, Zitronensäure, Weinsäure, Maleinsäure oder Fumarsäure in betracht.

Die als Ausgangsstoffe verwendeten Verbindungen der allgemeinen Formeln III-V und VII sind literaturbekannt bzw. werden nach an sich bekannten verfahren erhalten.

Die als Ausgangsstoffe verwendeten Verbindungen der allgemeinen Formeln VI, VIII und IX erhält man zweckmäßigerweise durch Umsetzung einer entsprechenden α-Halogenverbindung mit einem entsprechenden Hydroxyindol der allgemeinen Formel II.

Die als Ausgangsstoffe verwendeten Verbindungen der allgemeinen Formel II sind teilweise neu und lassen sich nach folgenden an sich bekannten Verfahren herstellen :

Durch Kondensation eines Benzyläthers der allgemeinen Formel

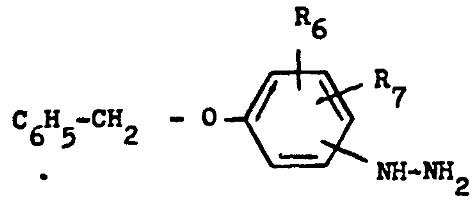

(X)

mit einem Keton der allgemeinen Formel

$$R_2 — CO — CH_2 — R_3 \quad (XI)$$

in Gegenwart von Äthanol/Schwefelsäure, Eisessig, Salzsäure/Äthanol, Salzsäure/Methanol oder Salzsäure/Isopropanol, anschließende Alkylierung des erhaltenen Benzyloxyindols in 1-Stellung und Entbenzylierung mittels katalytischer Hydrierung bzw. Bromwasserstoff oder, zur Herstellung von Hydroxyindolen der allgemeinen Formel II, in der $R_2$ einen aromatischen Rest darstellt, durch Kondensation eines Ketons der allgemeinen Formel

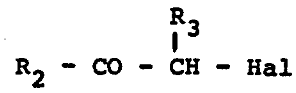

(XII)

mit einem Benzyläther der allgemeinen Formel

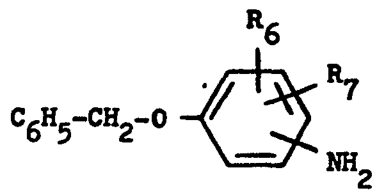

(XIII)

anschließende Alkylierung des erhaltenen Benzyloxyindols in 1-Stellung und Entbenzylierung mittels katalytischer Hydrierung bzw. Bromwasserstoff oder durch Anlagerung eines 1,4-Benzochinons an eine ungesättigte Verbindung der allgemeinen Formel

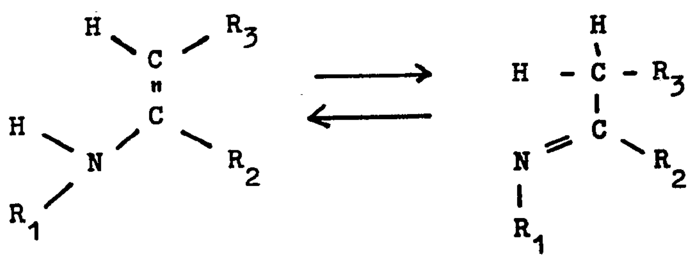

(XIV)

oder durch Aromatisierung eines Indolons der allgemeinen Formel

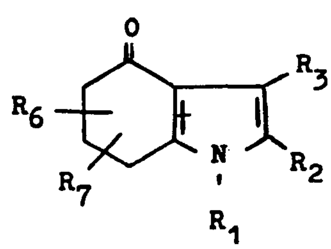

(XV)

mittels Palladium/Kohle oder durch Alkylierung eines Benzyloxyindols der allgemeinen Formel

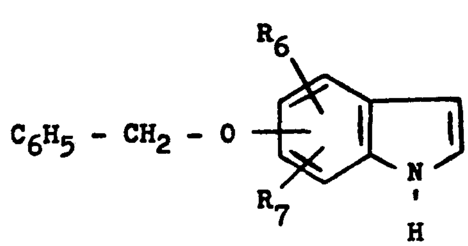

(XVI)

in 3- bzw. 3- und 4-Stellung, anschließende Alkylierung des erhaltenen Benzyloxyindol in 1-Stellung und Entbenzylierung mittels katalytischer Hydrierung bzw. Bromwasserstoff oder, zur Herstellung einer Verbindung der allgemeinen Formel I, in der $R_1$ und $R_2$ zusammen eine Alkylengruppe mit 3 bis 5 Kohlenstoffatomen bedeuten, durch Umsetzung eines entsprechenden Benzyloxyphenyldiazoniumsalzes mit einem entsprechend substituierten Acetessigsäureäthylester, anschließende O-Benzylierung des erhaltenen Hydroxyindols der allgemeinen Formel

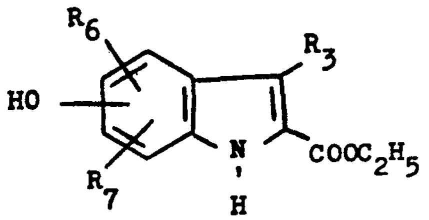

(XVII)

Alkylierung des erhaltenen Benzyloxyindols in 1-Stellung mit einem entsprechenden ω-Halogenalkansäureäthylester, Cyclisierung des erhaltenen Dicarbonsäureesters nach Dieckmann, Reduktion des nach Verseifung und Decarboxylierung erhaltenen cyclischen Ketons und Entbenzylierung des erhaltenen Benzyloxyderivates mittels katalytischer Hydrierung.

Wie bereits eingangs erwähnt, weisen die neuen Verbindungen der allgemeinen Formel I und deren physiologisch verträgliche Salze wertvolle pharmakologische Eigenschaften auf. Sie wirken triglycerid- und cholesterinsenkend, insbesondere erniedrigen sie die Low-density-lipoproteine und erhöhen die High-density-lipoproteine.

Beispielsweise wurden die Verbindungen

A = 2-[3-(4-Chlorphenyl)-1,2-dimethyl-1H-indol-5-yloxy]-2-methyl-propansäure,
B = 2-[1-Butyl-3-(4-fluorphenyl)-2-methyl-1H-indol-5-yloxy]-2-methyl-propansäure,
C = 2-Methyl-2-[2-methyl-1-propyl-3-(4-pyridyl)-1H-indol-5-yloxy] propansäure-hydrochlorid und
D = 2-[2-(4-Acetaminophenyl)-1-propyl-1H-indol-6-yloxy]-2-methyl-propansäure-äthylester

auf ihre biologischen Eigenschaften wie folgt untersucht :

1. Lipidsenkende Wirkung

Literatur : P.E. Schurr et al. in Atherosclerosis Drug Discovery (1976), Herausgeber ; C.E. Day ; Plenum, New-York, Seite 215.

Junge männliche Ratten mit einem durchschnittlichen Gewicht von 100 g wurden durch viertägige Gabe einer Diät (bestehend aux 10 % Kokosfett, 1,5 % Cholesterin, 0,5 % Cholsäure, 0,2 % Cholinchlorid und 15 % Sucrose) hyperlipämisch gemacht. Unter Beibehaltung der Diät appliziert man an zwei aufeinanderfolgenden Tagen die zu untersuchenden Substanzen in Methylcellulose-Suspension per Schlundsonde. Anschließend wurden die Tiere über Nacht nüchtern gehalten, und 24 Stunden nach der letzten Substanzapplikation wurde Blut zur Serumgewinnung entnommen.

Im Serum wurden gesamtcholesterin (Boehringer Mannheim Test-kombination 187.313) und Triglyceride (Boehringer Mannheim Testkombination 126.039) enzymatisch bestimmt. Die β-Lipoproteine wurden nach Fällung mit $Ca^{++}$ und Heparin im Autoanalyzer nephelometrisch bestimmt.

Die Berechnung der prozentualen Senkung erfolgte gegen eine Kontrollgruppe.

Die folgende Tabelle enthält die gefundenen Werte :

| Substanz | Dosis [mg/kg] | Senkung in % gegenüber Kontrolle nach zweimaliger Applikation: Serum-Gesamt-cholesterin | Serum-tri-glyceride | Serum-ß-Lipo-proteine |
|---|---|---|---|---|
| A | 1,25 | – 38,2 | – 46,3 | – 45,8 |
| | 5,0 | – 60,9 | – 62,6 | – 68,2 |
| | 20,0 | – 56,8 | – 54,7 | – 75,7 |
| B | 1,25 | – 37,5 | – 44,4 | – 43,7 |
| | 5,0 | – 49,8 | – 59,3 | – 55,2 |
| | 20,0 | – 49,4 | – 56,5 | – 53,4 |

(Fortsetzung)

| Substanz | Dosis [mg/kg] | Senkung in % gegenüber Kontrolle nach zweimaliger Applikation: Serum-Gesamt-cholesterin | Serum-tri-glyceride | Serum-β-Lipo-proteine |
|---|---|---|---|---|
| C | 1,25 | – 30,2 | – 45,6 | – 37,5 |
| | 5,0 | – 40,1 | – 48,0 | – 57,5 |
| | 20,0 | – 60,0 | – 63,6 | – 82,5 |
| D | 1,25 | – 34,9 | – 35,4 | – 42,3 |
| | 20,0 | – 65,6 | – 33,3 | – 84,9 |

2. Akute Toxizität :

Die akute Toxizität wurde orientierend an Gruppen von 6 weiblichen Mäusen (Beobachtungszeit : 7 Tage) bzw. 10 Mäusen (5 weibliche und 5 männliche Mäuse ; Beobachtungszeit : 14 Tage) nach Gabe einer Dosis von 1 000 oder 2 000 mg/kg pro Tier in Methylcellulose-Suspension per Schlundsonde bestimmt :

| Substanz | perorale Toxizität |
|---|---|
| A | > 2 000 mg/kg (1 von 10 Tieren gestorben) |
| B | 2 000 mg/kg (3 von 6 Tieren gestorben) |
| C | > 1 000 mg/kg (0 von 6 Tieren gestorben) |
| D | > 2 000 mg/kg (0 von 6 Tieren gestorben) |

Aufgrund ihrer pharmakologischen Eigenschaften eignen sich die erfindungsgemäß hergestellten Verbindungen zur Behandlung von Hyperlipidämien, insbesondere des Typ IIA, IIB und IV, und dadurch bedingten atherosklerotischen Veränderungen des Gefäßsystems und lassen sich zur pharmazeutischen Anwendung, gegebenenfalls in Kombination mit anderen Wirksubstanzen, in die üblichen pharmazeutischen Zubereitungene wie Dragées, Tabletten, Kapseln, Suppositorien, Suspensionen oder Lösungen einarbeiten, die Einzeldosis beträgt hierbei 5 bis 100 mg, vorzugsweise jedoch 5 bis 30 mg, und die Tagesdosis 10 bis 300 mg, vorzugsweise 15-90 mg.

Die nachfolgenden Beispiele sollen die Erfindung näher erläutern :

## Herstellung der Ausgangsstoffe

### Beispiel A

5-Benzyloxy-3-(3-chlor-phenyl)-2-methyl-1H-indol

11,7 g (47 mMol) 4-Benzyloxy-phenylhydrazin-hydrochlorid und 6,39 g (38 mMol) (3-Chlorphenyl)aceton werden in 35 ml absolutem Äthanol gelöst und nach Zugabe von 2,7 ml konzentrierter Schwefelsäure 5 Stunden auf Rückflußtemperatur erhitzt. Nach dem Abdestillieren des Äthanols wird der Rückstand mit Wasser versetzt und mit Chloroform extrahiert. Die Extrakte werden über Natriumsulfat getrocknet und eingeengt. Der Einengungsrückstand wird zur Reinigung an Kieselgel mit Toluol Fließmittel chromatographiert.

Ausbeute : 6,6 g (50 % der Theorie),
Schmelzpunkt : < 20 °C

### Beispiel B

5-Benzyloxy-3-(3-chlor-phenyl)-1,2-dimethyl-1H-indol

3,21 g (9,25 mMol) 5-Benzyloxy-3-(3-chlor-phenyl)-2-methyl-1H-indol werden in 50 ml absolutem Dimethylformamid gelöst und mit 406 mg (9,3 mMol) 55 %igem Natriumhydrid in Paraffinöl versetzt. Nach einer Stunde werden 1,45 g (10,2 mMol) Methyljodid zugegeben und über Nacht gerührt. Anschließend wird eingeengt, mit Wasser zersetzt und mit Chloroform extrahiert. Die über Natriumsulfat getrockneten

Extrakte werden eingeengt und an Kieselgel mit Toluol als Fließmittel chromatographiert.

Ausbeute : 2,56 g (77 % der Theorie),

Schmelzpunkt : 143 °C.

Beispiel C

3-(3-Chlor-phenyl)-1,2-dimethyl-1H-indol-5-ol

4 g (0,011 Mol) 5-Benzyloxy-3-(3-chlor-phenyl)-1,2-dimethyl-1H-indol werden in 100 ml Dioxan und 50 ml Methanol gelöst und bei Raumtemperatur und 1 bar mit 0,5 g Palladium auf Kohle bis zur Beendigung der Wasserstoffaufnahme hydriert.

Nach Abfiltrieren des Katalysators wird eingeengt und mit Toluol als Fließmittel an Kieselgel chromatographiert.

Ausbeute : 2,3 g (68 % der Theorie),

Schmelzpunk : 68 °C.

Analog den Beispielen A bis C wurden folgende Verbindungen hergestellt :

1,2-Dimethyl-3-phenyl-1H-indol-5-ol
3-(2-Methoxyphenyl)-1,2-dimethyl-1H-indol-5-ol
3-(4-Methoxy-phenyl)-1,2dimethyl-1H-indol-5-ol
3-(4-Methoxy-phenyl)-2-methyl-1H-indol-5-ol
3-(4-Methoxy-phenyl)-2-Methyl-1-propyl-1H-indol-5-ol
3-(2-Chlor-phenyl)-2-methyl-1H-indol-5-ol
3-(2-Chlor-phenyl)-2-methyl-1-propyl-1H-indol-5-ol
3-(4-Chlor-phenyl)-2-methyl-1-propyl-1H-indol-5-ol
3-(4-Chlor-phenyl)-1-hexyl-2-methyl-1H-indol-5-ol
3-(4-Chlor-phenyl)-1-dodecyl-2-methyl-1H-indol-5-ol
3-(4-Chlor-phenyl)-1,2-dimethyl-1H-indol-5-ol
3-(2-Chlor-phenyl)-1,2-dimethyl-1H-indol-5-ol
1-Äthyl-3-(2-brom-phenyl)-2-methyl-1H-indol-5-ol
3-(4-Fluor-phenyl)-1,2-dimethyl-1H-indol-5-ol
1-Butyl-3-(4-fluor-phenyl)-2-methyl-1H-indol-5-ol
3-(4-Fluor-phenyl)-1-hexyl-2-methyl-1H-indol-5-ol
3-(3-Trifluormethyl-phenyl)-1,2-dimethyl-1H-indol-5-ol
2-Methyl-3-(2-methyl-phenyl)-1-propyl-1H-indol-5-ol
2-Methyl-3-(3-methyl-phenyl)-1H-indol-5-ol
1,2-Dimethyl-3-(3-methyl-phenyl)-1H-indol-5-ol
2-Methyl-3-(4-methyl-phenyl)-1H-indol-5-ol
1,2-Dimethyl-3-(4-methyl-phenyl)-1H-indol-5-ol
1,2-Dimethyl-3-(2-phenyl-äthyl)-1H-indol-5-ol
2-Methyl-3-(2-phenyl-äthyl)-1-propyl-1H-indol-5-ol
3-[2-(Chlor-phenyl)äthyl]-2-methyl-1-propyl-1H-indol-5-ol
3-[2-(4-Chlor-phenyl)äthyl]-2-methyl-1H-indol-5-ol
3-[2-(4-Methoxy-phenyl)äthyl]-2-methyl-1-propyl-1H-indol-5-ol
2-Methyl-3-(4-pyridyl)-1H-indol-5-ol
2-Isopropyl-3-(4-pyridyl)-1H-indol-5-ol
2-Benzyl-3-(4-pyridyl)-1H-indol-5-ol
1,2-Dimethyl-3-(4-pyridyl)-1H-indol-5-ol
2-Isopropyl-1-methyl-3-(4-pyridyl)-1H-indol-5-ol
1-Äthyl-2-methyl-3-(4-pyridyl)-1H-indol-5-ol
2-Methyl-1-propyl-3-(4-pyridyl)-1H-indol-5-ol
1-Isopropyl-2-methyl-3-(4-pyridyl)-1H-indol-5-ol
1-Isobutyl-2-methyl-3-(4-pyridyl)-1H-indol-5-ol
1-Butyl-2-methyl-3-(4-pyridyl)-1H-indol-5-ol
1,2-Dimethyl-3-(2-pyridyl)-1H-indol-5-ol
2-Methyl-1-propyl-3-(4-pyridyl)-1H-indol-6-ol
2-Methyl-1-propyl-3-(4-pyridyl)-1H-indol-4-ol
2-Methyl-3-phenyl-1-propyl-1H-indol-6-ol
3-Benzyl-2-methyl-1-propyl-1H-indol-5-ol
3-(4-Chlor-benzyl)-2-methyl-1-propyl-1H-indol-5-ol
3-(4-Methoxy-phenyl)-1,2-dimethyl-1H-indol-7-ol
2-(4-Amino-phenyl)-3-methyl-1-propyl-1H-indol-5-ol
2-(4-Amino-phenyl)-1-propyl-1H-indol-6-ol
5-Benzyloxy-2-(4-nitro-phenyl)-1-propyl-1H-indol
2-(4-Acetamino-phenyl)-1-propyl-1H-indol-5-ol

Beispiel D

5-Benzyloxy-3-methyl-2-phenyl-1H-indol

3,6 g (0,015 Mol) 2-Brom-propiophenon und 6 g (0,03 Mol) 4-Benzyloxy-anilin werden in 15 ml Triäthylamin 2 Stunden am Rückfluß erhitzt. Dann engt man zur Trockne ein und erhitzt den Rückstand 2 Stunden auf 180 °C. Nach dem Abkühlen versetzt man mit 10 %iger Salzsäure und extrahiert mit Chloroform. Die Extrakte werden über Natriumsulfat getrocknet, filtriert, eingeengt und an Kieselgel mit Toluol als Fließmittel säulenchromatographisch gereinigt.

Ausbeute : 2,8 g (59,5 % der Theorie),
Schmelzpunkt : 130 °C.

In analoger Weise werden nach Beispiele D, B und C erhalten :

3-Methyl-2-phenyl-1-propyl-1H-indol-5-ol
2-(4-Chlorphenyl)-3-methyl-1-propyl-1H-indol-5-ol
2-(4-Methoxy-phenyl)-3-methyl-1-propyl-1H-indol-5-ol
1,3-Dimethyl-2-(4-pyridyl)-1H-indol-5-ol
3-Methyl-1-propyl-2-(4-pyridyl)-1H-indol-5-ol

Beispiel E

7,8,9,10-Tetrahydro-11-(4-pyridyl)-6H-azepino[1,2-a]indol-2-ol
7,8,9,10-Tetrahydro-11-(4-pyridyl)-6H-azepino[1,2-a]indol-3-ol

141 g (0,75 Mol) 2-(4-Picolinyliden-1,3,4,5,6,7-hexahydro-azepin werden in 1 000 ml Chloroform gelöst und unter Eiskühlung mit einer Lösung von 89 g (0,83 Mol) 1,4-Benzochinon in 1 000 ml Chloroform tropfenweise versetzt. Nach 24 Stunden bei Raumtemperatur filtriert man den gebildeten Niederschlag ab, suspendiert ihn in 500 ml Dimethylformamid und erwärmt 2 Stunden auf 100 °C. Nach dem Abkühlen wird abgesaugt und mit Dimethylformamid und Aceton gewaschen. Man erhält reines 2-Hydroxy-Isomeres.

Ausbeute : 38,1 g (18,2 % der Theorie),
Schmelzpunkt : 345-348 °C (Zers.)

Nach Einengen aller angefallenen Mutterlagen wird im Fließmittel Chloroform/Methanol 20 : 1 an Kieselgel chromatographiert. Nach dem Einengen der Eluate erhält man durch Verreiben mit Aceton reines 3-Hydroxy-Isomeres.

Ausbeute : 19,8 g (9,5 % der Theorie),
Schmelzpunkt : 266-270 °C. (Zers.)

In analoger Weise werden erhalten :

11-(2-Chinolyl)-7,8,9,10-tetrahydro-6H-azepino[1,2-a]indol-2-ol
7,8,9,10-Tetrahydro-11-(2-pyridyl)-6H-azepino[1,2-a]indol-2-ol
7,8,9,10-Tetrahydro-1,3-dimethyl-11-(4-pyridyl)-6H-azepino[1,2-a]indol-2-ol
6,7,8,9-Tetrahydro-10-(4-pyridyl)-pyrido[1,2-a]indol-2-ol
7,8-Dihydro-9-(4-pyridyl)-6H-pyrrolo[1,2-a]indol-2-ol
2-(4-Nitro-phenyl)-1-propyl-1H-indol-6-ol

Beispiel F

2-Benzyloxy-7,8,9,10-tetrahydro-11-phenyl-6H-azepino[1,2-a]indol

a) 5-Hydroxy-3-phenyl-1H-2-indolcarbonsäure-äthylester

193 g (0,82 Mol) 4-Benzyloxyanilin-hydrochlorid werden in 650 ml halbkonzentrierter Salzsäure mit 65,9 g (0,95 Mol) Natriumnitrit diazotiert. Die so erhaltene Diazoniumsalzlösung wird bei − 5 °C zur Lösung von 198,2 g (0,9 Mol) 2-Benzyl-acetessigsäureäthylester und 140 g Kaliumhydroxid in wässrigem Äthanol gegeben und anschließend 2 Stunden bei Raumteperatur gerührt. Dann extrahiert man mit Toluol, trocknet die Toluolextrakte und engt ein. Der Einengungsrückstand wird in absolutem Äthanol gelöst und in der Siedehitze mit Salzsäuregas gesättigt.

Nach Filtration und Einengen der Reaktionslösung wird an Kieselgel mit Toluol/Essigester (7 : 1) als Fließmittel chromatographiert.

Ausbeute : 56,4 g (24,5 % der Theorie),
Schmelzpunkt : 133-135 °C.

b) 5-Benzyloxy-3-phenyl-1H-2-indolcarbonsäure-äthylester

26 g (92,4 mMol) 5-Hydroxy-3-phenyl-1H-2-indolcarbonsäure-äthylester werden mit 27,6 g Kaliumcarbonat und 11 ml (92,4 Mol) Benzylbromid in 700 ml Methyl-äthyl-keton 4 Tage am Rückfluß gekocht. Nach Filtration und Einengen des Filtrats wird an Kieselgel mit Toluol als Fließmittel chromatographiert.

Ausbeute : 20,6 g (60 % der Theorie),
Schmelzpunkt : < 20 °C.

c) 5-(2-Äthoxycarbonyl-5-benzyloxy-3-phenyl-1H-indol-1-yl)-valeriansäure-äthylester

23,5 g (63,3 mMol) 5-Benzyloxy-3-phenyl-1H-2-indolcarbonsäure-äthylester werden in absolutem Dimethylformamid mit 3,0 g (63,3 mMol) 50 %igem Natriumhydrid versetzt. Nach 30 Minuten fügt man 13,2 g (63,3 mMol) 5-Brom-valeriansäure-äthylester zu und rührt 3 Tage. Anschließend wird eingeengt und an Kieselgel mit Toluol/Aceton (20 : 1) als fließmittel chromatographiert.

Ausbeute : 33,9 g (92,6 % der Theorie),
Schmelzpunkt : < 20 °C.

d) 2-Benzyloxy-7,8,9,10-tetrahydro-10-oxo-11-phenyl-6H-9-azepino[1,2-a] indolcarbonsäure-äthylester

23 g (46 mMol) 5-(2-Äthoxycarbonyl-5-benzyloxy-3-phenyl-1H-indol-1-yl) valeriansäure-äthylester werden in 150 ml Toluol gelöst und innerhalb von 4 Stunden tropfenweise einer siedenden Suspension von 5,2 g (46 mMol) Kalium-tert.-butylat in 150 ml Toluol zugefügt. Dabei wird gebildetes Äthanol mit Toluol abdestilliert. Nach Abkühlung wird mit eiskalter verdünnter Salzsäure versetzt und mit Toluol extrahiert. Die Toluolextrakte werden eingeengt und durch Kieselgelchromatographie mit Toluol als Fließmittel gereinigt.

Ausbeute : 3,8 g (18,2 % der Theorie),
Schmelzpunkt : 108-111 °C.

e) 2-Benzyloxy-7,8,9,10-tetrahydro-10-oxo-11-phenyl-6H-azepino[1,2-a] indol

2,8 g (6,17 mMol) 2-Benzyloxy-7,8,9,10-tetrahydro-10-oxo-11-phenyl-6H-9-azepino[1,2-a] indolcarbonsäure-äthylester und 0,36 g (6,1 mMol) Natriumchlorid werden in 3,5 ml Dimethylsulfoxid und 0,35 ml Wasser 4 Stunden am Rückfluß gekocht. Nach dem Abkühlen nimmt man in Essigester auf, trocknet über Natriumsulfat, saugt ab und engt ein. Der Rückstand wird mit Petroläther verrieben und abgesaugt.

Ausbeute : 2,3 g (97,7 % der Theorie),
Schmelzpunkt : 126-128 °C.

f) 2-Benzyloxy-7,8,9,10-tetrahydro-11-phenyl-6H-azepino[1,2-a]indol

2,3 g (6 mMol) 2-Benzyloxy-7,8,9,10-tetrahydro-10-oxo-11-phenyl-6H-azepino[1,2-a]indol, 4,5 ml 80 %iges Hydrazinhydrat und 1,5 g Kaliumhydroxid werden in 6 ml Triäthylenglykol 2 Stunden auf 180 °C und dann 4 Stunden auf 220 °C erhitzt. Nach dem Abkühlen versetzt man mit Wasser und extrahiert mit Chloroform. Nach Säulenchromatographie der eingeengten Chloroformextrakte an Kieselgel mit Toluol als Fließmittel erhält man farblose Kristalle.

Ausbeute : 0,8 g (36,3 % der Theorie),
Schmelzpunkt : 147-149 °C.

Durch Umsetzung des erhaltenen Produkts analog Beispiel C wird 7,8,9,10-Tetrahydro-11-phenyl-6H-azepino[1,2-a] indol-2-ol erhalten.

## Beispiel G

5-Benzyloxy-3-(4-pyridylmethyl)-1H-indol

Zu einer aus 0,27 Mol Magnesium, 0,30 Mol Methyljodid und 200 ml Äther hergestellten Grignardlösung tropft man eine Lösung von 33,8 g (0,152 Mol)-5-Benzyloxy-1H-indol in 800 ml Äther. Anschließend gibt man bei 0-5 °C 13,2 g (0,08 Mol) 4-Chlormethyl-pyridin-hydrochlorid zu. Nach weiterer Zugabe von 500 ml absolutem Benzol wird 2 Stunden am Rückfluß gekocht, wobei man vorher den Äther abdestillieren läßt. Nach Stehen über Nacht wird mit Eis und Salzsäure zersetzt. Nach Abdekantieren wird der zähe Rückstand mit wässrigem Ammoniak versetzt und in Chloroform aufgenommen. Nach Trocknen und Einengen der Chloroformextrakte wird mit Essigester als Fließmittel an Kieselgel chromatographiert.

Ausbeute : 11,6 g (46 % der Theorie),
Schmelzpunkt : 124-126 °C.

Analog Beispiel G, B und C werden erhalten :

3,4-Dibenzyl-1-propyl-1H-indol-5-ol

1-Propyl-3-(4-pyridylmethyl)-1H-indol-5-ol

Beispiel H

3-Methyl-2-(4-nitro-phenyl)-1-propyl-1H-indol-5-ol

4 g (10 mMol) 5-Benzyloxy-3-methyl-2-(4-nitro-phenyl)-1-propyl-1H-indol und 40 ml einer gesättigten Lösung von Bromwasserstoff in Eisessig werden 20 Minuten unter Rückfluß gekocht. Nach dem Einengen wird an Kieselgel mit Toluol/Aceton (20 : 1) als Fließmittel chromatographisch gereinigt.
Ausbeute : 0,85 g (27,4 % der Theorie),
Schmelzpunkt : < 20 °C.

Beispiel J

2-Methyl-3-phenyl-1H-indol-4-ol

22,5 g (0,1 Mol) 4,5,6,7-Tetrahydro-2-methyl-3-phenyl-1H-4-indolon und 5 g Palladium auf Kohle werden 6 Stunden auf 200 °C erhitzt. Nach dem Abkühlen wird mit Äther verrührt und filtriert. Das Ätherfiltrat wird eingeengt und an Kieselgel mit Toluol als Fließmittel chromatographiert.
Ausbeute : 6,7 g (30 % der Theorie),
Schmelzpunkt : < 20 °C.

Beispiel 1

2-Methyl-2-[1,2-dimethyl-3-phenyl-1H-indol-5-yloxy] propansäureäthylester

1 g (42 mMol) 1,2-Dimethyl-3-phenyl-1H-indol-5-ol werden in 10 ml absolutem Dimethylformamid mit 223 mg (5 mMol) 55 %iger Natrium-hybrid-immersion in öl in das Natriumsalz überführt und bei Raumtemperatur mit 990 mg (5 mMol) 2-Brom-2-methyl-propansäure-äthyl-ester versetzt. Nach einer Reaktionszeit von 6 Stunden wird das Lösungsmittel im Vakuum abdestilliert und das Reaktionsprodukt über eine Kieselgelsäule mit Toluol : Essigsäureäthylester (9 : 1) als Laufmittel chromatographisch gereinigt. Der Trockenrückstand der vereinigten Fraktionen, die den gereinigten Ester enthalten, wird mit Petroläther behandelt.
Ausbeute : 330 mg (34 % der Theorie),
Schmelzpunkt : 115 °C.
Ber. : C 75,1 H 7,19 N 3,99
Gef. : C 74,6 H 7,09 N 4,01

Beispiel 2

2-[3-(2-Methoxy-phenyl)-1,2-dimethyl-1H-indol-5-yloxy]-2-methylpropansäure-äthylester

Hergestellt aus 3-(2-Methoxy-phenyl)-1,2-dimethyl-1H-indol-5-ol und 2-Brom-2-methyl-propansäure-äthylester analog Beispiel 1 (Lösungsmittel : Hexamethylphosphorsäuretriamid).
Ausbeute : 68 % der Theorie,
Schmelzpunkt : < 20 °C.
Ber. : C 72,5 H 7,13 N 3,67
Gef. : C 72,4 H 7,07 N 3,97

Beispiel 3

2-[3-(4-Methoxy-phenyl)-1,2-dimethyl-1H-indol-5-yloxy]-2-methylpropansäure-äthylester

Hergestellt aus 3-(4)Methoxy-phenyl)-1,2-dimethyl-1H-indol-5-ol und 2-Brom-2-methyl-propansäure-äthylester analog Beispiel 1.
Ausbeute : 58 % der Theorie,
Schmelzpunkt : 82 °C.
Ber. : C 72,5 H 7,15 N 3,67
Gef. : C 72,2 H 7,12 N 3,87

Beispiel 4

2-[3-(4-Methoxy-phenyl)-2-methyl-1H-indol-5-yloxy]-2-methyl-propansäure-äthylester

Hergestellt aus 3-(4-Methoxy-phenyl)-2-methyl-1H-indol-5-ol und 2-Brom-2-methyl-propansäure-

äthylester analog Beispiel 1.
Ausbeute : 34 % der Theorie,
Schmelzpunkt : 108 °C.
Ber. : C 71,9 H 6,86 N 3,82
Gef. : C 71,7 H 6,91 N 3,87

Beispiel 5

2-[3-(4-Methoxy-phenyl)-2-methyl-1-propyl-1H-indol-5-yloxy]-2-methyl-propansäure-äthylester

Hergestellt aus 3-(4-Methoxy-phenyl)-2-methyl-1-propyl-1H-indol-5-ol und 2-Brom-2-methyl-propansäure-äthylester analog Beispiel 1.
Ausbeute : 68 % der Theorie,
Schmelzpunkt ; 85 °C.
Ber. : C 73,3 H 7,63 N 3,42
Gef. : C 73,6 H 7,86 N 3,45

Beispiel 6

2-[3-(2-Chlor-phenyl)-2-methyl-1H-indol-5-yloxy]-2-methyl-propansäure-äthylester

Hergestellt aus 3-(2-Chlor-phenyl)-2-methyl-1H-indol-5-ol und 2-Brom-2-methyl-propansäure-äthylester analog Beispiel 1.
Ausbeute : 58 % der Theorie,
Schmelzpunkt : 102 °C.
Ber. : C 67,95 H 5,98 N 3,77 Cl 9,57
Gef. : C 67,80 H 6,03 N 3,90 Cl 9,77

Beispiel 7

2-[3-(2-Chlor-phenyl)-2-methyl-1-propyl-1H-indol-5-yloxy]-2-methyl-propansäure-äthylester

Hergestellt aus 3-(2-Chlor-phenyl)-2-methyl-1-propyl-1H-indol-5-ol und 2-Brom-2-methyl-propansäure-äthylester analog Beispiel 1.
Ausbeute : 32 % der Theorie,
Schmelzpunkt : 82 °C.
Ber. : C 69,55 H 6,81 N 3,38 Cl 8,58
Gef. : C 69,60 H 6,97 N 3,55 Cl 8,62

Beispiel 8

2-[3-(4-Chlor-phenyl)-1,2-dimethyl-1H-indol-5-yloxy]-2-methyl-propansäure-äthylester

Hergestellt aus 3-(4-Chlor-phenyl)-1,2-dimethyl-1H-indol-5-ol und 2-Brom-2-methyl-propansäure-äthylester analog Beispiel 1.
Ausbeute : 48 % der Theorie,
Schmelzpunkt : 120 °C.
Ber. : C 68,1 H 6,26 N 3,61
Gef. : C 69,1 H 6,65 N 4,02

Beispiel 9

2-[3-(3-Chlor-phenyl)-1,2-dimethyl-1H-indol-5-yloxy]-2-methyl-propansäure-äthylester

Hergestellt aus 3-(3-Chlor-phenyl)-1,2-dimethyl-1H-indol-5-ol und 2-Brom-2-methyl-propansäure-äthylester analog Beispiel 1.
Ausbeute : 80 % der Theorie,
Schmelzpunkt : 90 °C.
Ber. : C 68,1 H 6,26 N 3,61
Gef. : C 68,8 H 6,50 N 3,58

Beispiel 10

2-[1-Äthyl-3-(2-Brom-phenyl)-2-mehtyl-1H-indol-5-yloxy]-2-methyl-propansäure-äthylester

Hergestellt aus 1-Äthyl-3-(2-Brom-phenyl)-2-methyl-1H-indol-5-ol und 2-Brom-2-methyl-propansäure-äthylester analog Beispiel 1.
Ausbeute : 8 % der Theorie,
Schmelzpunkt : 88 °C.

Beispiel 11

2-[3-(4-Fluor-phenyl)-1,2-dimethyl-1H-indol-5-yloxy]-2-methyl-propansäure-äthylester

Hergestellt aus 3-(4-Fluor-phenyl)-1,2-dimethyl-1H-indol-5-ol und 2-Brom-2-methyl-propansäure-äthylester analog Beispiel 1.
Ausbeute : 70 % der Theorie,
Schmelzpunkt : 108 °C
Ber. : C 71,6 H 6,54 N 3,79
Gef. : C 71,5 H 6,69 N 3,74

Beispiel 12

2-[1-Butyl-3-(4-Fluor-phenyl)-2-methyl-1H-indol-5-yloxy]-2-methyl-propansäure-äthylester

Hergestellt aus 1-Butyl-3-(4-Fluor-phenyl)-2-methyl-1H-indol-5-ol und 2-Brom-2-methyl-propansäure-äthylester analog Beispiel 1.
Ausbeute : 74 % der Theorie,
Schmelzpunkt : < 20 °C.

Beispiel 13

2-[3-(4-Fluor-phenyl)-1-hexyl-2-methyl-1H-indol-5-yloxy]-2-methyl-propansäure-äthylester

Hergestellt aus 3-(4-Fluor-phenyl)-1-hexyl-2-methyl-1H-indol-5-ol und 2-Brom-2-methyl-propansäure-äthylester analog Beispiel 1.
Ausbeute : 74 % der Theorie,
Schmelzpunkt : < 20 °C.
Ber. : C 73,9 H 7,81 N 3,19
Gef. : C 73,5 H 7,85 N 3,43

Beispiel 14

2-[3-(3-Trifluormethyl-phenyl)-1,2-dimethyl-1H-indol-5-yloxy]-2-methyl-propansäure-äthylester

Hergestellt aus 3-(3-Trifluormethyl-phenyl)-1,2-dimethyl-1H-indol-5-ol und 2-Brom-2-methyl-propansäure-äthylester analog Beispiel 1.
Ausbeute : 30 % der Theorie,
Schmelzpunkt : < 20 °C.

Beispiel 15

2-Methyl-2-[2-methyl-3-(2-methyl-phenyl)-1-propyl-1H-indol-5-yloxy] propansäure-äthylester

Hergestellt aus 2-Methyl-3-(2-methyl-phenyl)-1-propyl-1H-indol-5-ol und 2-Brom-2-methyl-propansäure-äthylester analog Beispiel 1.
Ausbeute : 42 % der Theorie,
Schmelzpunkt : 70 °C.
Ber. : C 76,4 H 7,95 N 3,50
Gef. : C 76,6 H 8,04 N 3,56

Beispiel 16

2-Methyl-2-[2-methyl-3-(3-methyl-phenyl)-1H-indol-5-yloxy]-propansäure-äthylester

Hergestellt aus 2-Methyl-3-(3-methyl-phenyl)-1H-indol-5-ol und 2-Brom-2-methyl-propansäure-äthylester analog Beispiel 1.
Ausbeute : 62 % der Theorie,
Schmelzpunkt : < 20 °C.

Ber.: C 75,3 H 7,20 N 4,00
Gef.: C 75,6 H 7,40 N 3,97

Beispiel 17

2-Methyl-2-[1,2-dimethyl-3-(3-methyl-phenyl)-1H-indol-5-yloxy]-propansäure-äthylester

Hergestellt aus 1,2-Dimethyl-3-(3-methyl-phenyl)-1H-indol-5-ol und 2-Brom-2-methyl-propansäure-äthylester analog Beispiel 1.
Ausbeute: 47 % der Theorie,
Schmelzpunkt: 87 °C.
Ber.: C 75,53 H 7,45 N 3,83
Gef.: C 75,40 H 7,54 N 3,79

Beispiel 18

2-Methyl-2-[2-methyl-3-(4-methyl-phenyl)-1H-indol-5-yloxy]-propansäure-äthylester

Hergestellt aus 2-Methyl-3-(4-methyl-phenyl)-1H-indol-5-ol und 2-Brom-2-methyl-propansäure-äthylester analog Beispiel 1.
Ausbeute: 24 % der Theorie,
Schmelzpunkt: 121 °C.
Ber.: C 75,3 H 7,20 N 4,00
Gef.: C 75,6 H 7,44 N 4,03

Beispiel 19

2-Methyl-2-[1,2-dimethyl-3-(4-methyl-phenyl)-1H-indol-5-yloxy]-propansäure-äthylester

Hergestellt aus 1,2-Dimethyl-3-(4-methyl-phenyl)-1H-indol-5-ol und 2-Brom-2-methyl-propansäure-äthylester analog Beispiel 1.
Ausbeute: 41 % der Theorie,
Schmelzpunkt: 83 °C.
Ber.: C 75,53 H 7,45 N 3,83
Gef.: C 75,40 H 7,63 N 3,87

Beispiel 20

2-Methyl-2-[1,2-dimethyl-3-(2-phenyl-äthyl)-1H-indol-5-yloxy]-propansäure-äthylester

Hergestellt aus 1,2-Dimethyl-3-(2-phenyl-äthyl)-1H-indol-5-ol und 2-Brom-2-methyl-propansäure-äthylester analog Beispiel 1.
Ausbeute: 59 % der Theorie,
Schmelzpunkt: 71 °C.
Ber.: C 76,0 H 7,71 N 3,69
Gef.: C 76,0 H 7,69 N 3,68

Beispiel 21

2-Methyl-2-[2-methyl-3-(2-phenyl-äthyl)-1-propyl-1H-indol-5-yloxy]-propansäure-äthylester

Hergestellt aus 2-Methyl-3-(2-phenyl-äthyl)-1-propyl-1H-indol-5-ol und 2-Brom-2-methyl-propansäure-äthylester analog Beispiel 1.
Ausbeute: 69 % der Theorie,
Schmelzpunkt: $< 20$ °C.
Ber.: C 76,5 H 8,16 N 3,44
Gef.: C 76,5 H 8,19 N 3,54

Beispiel 22

2-{3-[2-(4-Chlor-phenyl)-äthyl]-2-methyl-1-propyl-1H-indol-5-yloxy}-2-methyl-propansäure-äthylester

Hergestellt aus 3-[2-(4-Chlor-phenyl)-äthyl]-2-methyl-1-propyl-1H-indol-5-ol und 2-Brom-2-methyl-propansäure-äthylester analog Beispiel 1.
Ausbeute: 74 % der Theorie,

Schmelzpunkt : < 20 °C.
Ber. : C 70,70 H 7,30 N 3,17
Gef. : C 71,33 H 7,41 N 3,25

## Beispiel 23

2-{3-[2-(4-Chlor-phenyl)-äthyl]-2-methyl-1H-indol-5-yloxy}-2-methyl-propansäure-äthylester

Hergestellt aus 3-[2-(4(Chlor-phenyl)äthyl]-2-methyl-1H-indol-5-ol und 2-Brom-2-methyl-propansäure-äthylester analog Beispiel 1.
Ausbeute : 47 % der Theorie,
Schmelzpunkt : 90 °C.
Ber. : C 69,2 H 6,55 N 3,50
Gef. : C 70,0 H 6,75 N 3,53

## Beispiel 24

2-{3-[2-(4-Methoxy-phenyl)äthyl]-2-methyl-1-propyl-1H-indol-5-yloxy}-2-methyl-propansäure-äthylester

Hergestellt aus 3-[2-(4-Methoxy-phenyl)äthyl]-2-methyl-1-propyl-1H-indol-5-ol und 2-Brom-2-methyl-propansäure-äthylester analog Beispiel 1.
Ausbeute : 74 % der Theorie,
Schmelzpunkt : < 20 °C.
Ber. : C 74,10 H 8,06 N 3,20
Gef. : C 74,30 H 8,05 N 3,43

## Beispiel 25

2-Methyl-2-[2-methyl-3-(4-pyridyl)-1H-indol-5-yloxy] propansäure-äthylester

Hergestellt aus 2-Methyl-3-(4-pyridyl)-1H-indol-5-ol und 2-Brom-2-methyl-propansäure-äthylester analog Beispiel 1.
Ausbeute : 35 % der Theorie,
Schmelzpunkt : 115 °C.
Ber. : C 72,8 H 6,54 N 8,26
Gef. : C 72,5 H 6,50 N 8,06

## Beispiel 26

2-[2-Isopropyl-3-(4-pyridyl)-1H-indol-5-yloxy]-2-methyl-propansäure-äthylester

Hergestellt aus 2-Isopropyl-3-(4-pyridyl)-1H-indol-5-ol und 2-Brom-2-methyl-propansäure-äthylester analog Beispiel 1.
Ausbeute : 25 % der Theorie,
Schmelzpunkt : 210 °C.
Ber. : C 72,1 H 7,25 N 7,55
Gef. : C 71,7 H 7,84 N 7,11

## Beispiel 27

2-[2-Benzyl-3-(4-pyridyl)-1H-indol-5-yloxy]-2-methyl-propansäure-äthylester

Hergestellt aus 2-Benzyl-3-(4-pyridyl)-1H-indol-5-ol und 2-Brom-2-methyl-propansäure-äthylester analog Beispiel 1.
Ausbeute : 21 % der Theorie,
Schmelzpunkt : 156 °C.
Ber. : C 75,4 H 6,33 N 6,76
Gef. : C 74,8 H 5,94 N 6,56

## Beispiel 28

2-Methyl-2-[1,2-dimethyl-3-(4-pyridyl)-1H-indol-5-yloxy]propansäure-äthylester

Hergestellt aus 1,2-Dimethyl-1,3-(4-pyridyl)-1H-indol-5-ol und 2-Brom-2-methyl-propansäure-äthylester analog Beispiel 1.

Ausbeute : 55 % der Theorie,
Schmelzpunkt : 104 °C.
Ber. : C 71,5 H 6,87 N 7,85
Gef. : C 70,7 H 6,61 N 7,75

Beispiel 29

2-[2-Isopropyl-1-methyl-3-(4-pyridyl)-1H-indol-5-yloxy]-2-methyl-propansäure-äthylester

Hergestellt aus 2-Isopropyl-1-methyl-3-(4-pyridyl)-1H-indol-5-ol und 2-Brom-2-methyl-propansäure-äthylester analog Beispiel 1.
Ausbeute : 70 % der Theorie,
Schmelzpunkt : 102 °C.
Ber. : C 72,55 H 7,42 N 7,37
Gef. : C 72,85 H 7,53 N 7,40

Beispiel 30

2-[1-Äthyl-2-methyl-3-(4-pyridyl)-1H-indol-5-yloxy]-2-methyl-propansäure-äthylester

Hergestellt aus 1-Äthyl-2-methyl-3-(4-pyridyl)-1H-indol-5-ol und 2-Brom-2-methyl-propansäure-äthylester analog Beispiel 1.
Ausbeute : 55 % der Theorie,
Schmelzpunkt : 78 °C.
Ber. : C 72,1 H 7,25 N 7,35
Gef. : C 72,2 H 7,30 N 7,73

Beispiel 31

2-Methyl-2-[2-methyl-1-propyl-3-(4-pyridyl)-1H-indol-5-yloxy]-propansäure-äthylester

Hergestellt aus 2-Methyl-1-propyl-3-(4-pyridyl)-1H-indol-5-ol und 2-Brom-2-methyl-propansäure-äthylester analog Beispiel 1.
Ausbeute : 36 % der Theorie,
Schmelzpunkt : 90 °C.
Ber. : C 72,6 H 7,43 N 7,36
Gef. : C 72,9 H 7,47 N 7,43

Beispiel 32

2-[1-Isopropyl-2-methyl-3-(4-pyridyl)-1H-indol-5-yloxy]-2-methyl-propansäure-äthylester

Hergestellt aus 1-Isopropyl-2-methyl-3-(4-pyridyl)-1H-indol-5-ol und 2-Brom-2-methyl-propansäure-äthylester analog Beispiel 1.
Ausbeute : 53 % der Theorie,
Schmelzpunkt : 128 °C.
Ber. : C 72,7 H 7,44 N 7,38
Gef. : C 72,8 H 7,57 N 7,20

Beispiel 33

2-[1-Isobutyl-2-methyl-3-(4-pyridyl)-1H-indol-5-yloxy]-2-methyl-propansäure-äthylester

Hergestellt aus 1-Isobutyl-2-methyl-3-(4-pyridyl)-1H-indol-5-ol und 2-Brom-2-methyl-propansäure-äthylester analog Beispiel 1.
Ausbeute : 49 % der Theorie,
Schmelzpunkt : < 20 °C.

Beispiel 34

2-[1-Butyl-2-methyl-3-(4-pyridyl)-1H-indol-5-yloxy]-2-methyl-propansäure-äthylester

Hergestellt aus 1-Butyl-2-methyl-3-(4-pyridyl)-1H-indol-5-ol und 2-Brom-2-methyl-propansäure-äthylester analog Beispiel 1.
Ausbeute : 44 % der Theorie,

Schmelzpunkt : < 20 °C.

Beispiel 35

2-Methyl-2-[1,2-dimethyl-3-(2-pyridyl)-1H-indol-5-yloxy]propansäure-äthylester

Hergestellt aus 1,2-Dimethyl-3-(2-pyridyl)-1H-indol-5-ol und 2-Brom-2-methyl-propansäure-äthylester analog Beispiel 1.
Ausbeute : 52 % der Theorie,
Schmelzpunkt : 80 °C.
Ber. : C 71,60 H 6,86 N 7,95
Gef. : C 71,65 H 7,00 N 8,13

Beispiel 36

2-Methyl-2-[3-methyl-2-phenyl-1-propyl-1H-indol-5-yloxy]propansäure-äthylester

Hergestellt aus 3-Methyl-2-phenyl-1-propyl-1H-indol-5-ol und 2-Brom-2-methyl-propansäure-äthylester analog Beispiel 1.
Ausbeute : 78 % der Theorie,
Schmelzpunkt : < 20 °C.
Ber. : C 76,1 H 7,72 N 3,69
Gef. : C 76,1 H 7,82 N 3,81

Beispiel 37

2-[2-(4-Chlor-phenyl)-3-methyl-1-propyl-1H-indol-5-yloxy]-2-methyl-propansäure-äthylester

Hergestellt aus 2-(4-Chlorphenyl)-3-methyl-1-propyl-1H-indol-5-ol und 2-Brom-2-methyl-propansäure-äthylester analog Beispiel 1.
Ausbeute : 84 % der Theorie,
Schmelzpunkt : 75 °C.
Ber. : C 69,75 H 6,83 N 3,38
Gef. : C 70,00 H 6,97 N 3,38

Beispiel 38

2-[2-(4-Methoxy-phenyl)-3-methyl-1-propyl-1H-indol-5-yloxy]-2-methyl-propansäure-äthylester

Hergestellt aus 2-(4-Methoxy-phenyl)-3-methyl-1-propyl-1H-indol-5-ol und 2-Brom-2-methyl-propansäure-äthylester analog Beispiel 1.
Ausbeute : 70 % der Theorie,
Schmelzpunkt : 93 °C.
Ber. : C 73,4 H 7,64 N 3,42
Gef. : C 73,6 H 7,67 N 3,27

Beispiel 39

2-Methyl-2-[1,3-dimethyl-2-(4-pyridyl)-1H-indol-5-yloxy]propansäure-äthylester

Hergestellt aus 1,3-Dimethyl-2-(4-pyridyl)-1H-indol-5-ol und 2-Brom-2-methyl-propansäure-äthylester analog Beispiel 1.
Ausbeute : 55 % der Theorie,
Schmelzpunkt : < 20 °C.
Ber. : C 71,50 H 6,86 N 7,95
Gef. : C 71,30 H 6,92 N 8,02
Das Hydrochlorid ist aus ätherischer Lösung mit isopropanolischer Salzsäure fällbar.
Ausbeute : 85 % der Theorie,
Schmelzpunkt : 164 °C.
Ber. : C 64,80 H 6,48 N 7,20
Gef. : C 64,70 H 6,54 N 7,42

Beispiel 40

2-Methyl-2-[3-methyl-1-propyl-2-(4-pyridyl)-1H-indol-5-yloxy]-propansäure-äthylester

Hergestellt aus 3-Methyl-1-propyl-2-(4-pyridyl)-1H-indol-5-ol und 2-Brom-2-methyl-propansäure-äthylester analog Beispiel 1.

Ausbeute : 70 % der Theorie,
Schmelzpunkt : 106 °C.
Ber. : C 72,5 H 7,42 N 7,35
Gef. : C 72,2 H 7,45 N 7,48

Beispiel 41

2-[11-(2-Chinolyl)-7,8,9,10-tetrahydro-6H-azepino[1,2-a]indol-2-yloxy]-2-methyl-propansäure-äthylester

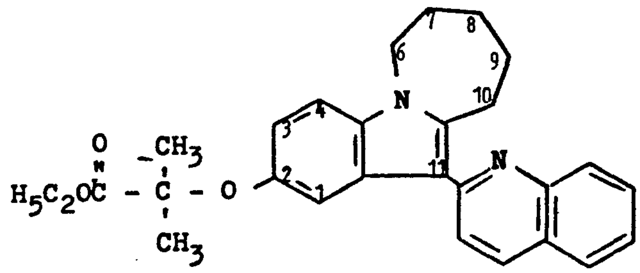

Hergestellt aus 11-(2-Chinolyl)-7,8,9,10-tetrahydro-6H-azepino-[1,2-a]indol-2-ol und 2-Brom-2-methyl-propansäure-äthylester analog Beispiel 1.

Ausbeute : 36 % der Theorie,
Schmelzpunkt : 96-98 °C.
Ber. : C 76,00 H 6,83 N 6,33
Gef. : C 75,80 H 6,97 N 6,39

Beispiel 42

2-[7,8,9,10-Tetrahydro-11-(4-pyridyl)-6H-azepino[1,2-a]indol-2-yloxy]-2-methyl-propansäure-äthylester

Hergestellt aus 7,8,9,10-Tetrahydro-11-(4-pyridyl)-6H-azepino-[1,2-a]indol-2-ol und 2-Brom-2-methyl-propansäure-äthylester analog Beispiel 1.

Ausbeute : 79,3 % der Theorie,
Schmelzpunkt : 118-119 °C.
Ber. : 73,45 H 7,19 N 7,14
Gef. : C 73,40 H 7,22 N 7,10

Beispiel 43

2-[7,8,9,10-Tetrahydro-11-(4-pyridyl)-6H-azepino[1,2-a]indol-3-yloxy]-2-methyl-propansäure-äthylester

Hergestellt aus 7,8,9,10-Tetrahydro-11-(4-pyridyl)-6H-azepino-[1,2-a]indol-3-ol und 2-Brom-2-methyl-propansäure-äthylester analog Beispiel 1.

Ausbeute : 65,5 % der Theorie,
Schmelzpunkt : 145-146 °C.
Ber. : C 73,45 H 7,19 N 7,14
Gef. : C 73,70 H 7,29 N 7,12

Beispiel 44

2-[7,8,9,10-Tetrahydro-1,3-dimethyl-11-(4-pyridyl)-6H-azepino-[1,2-a]indol-2-yloxy]-2-methyl-propansäure-äthylester

Hergestellt aus 7,8,9,10-Tetrahydro-1,3-dimethyl-11-(4-pyridyl)-6H-azepino[1,2-a]indol-2-ol und 2-Brom-2-methyl-propansäure-äthylester analog Beispiel 1.

Ausbeute : 35 % der Theorie,
Schmelzpunkt : 115-117 °C.
Ber. : C 74,26 H 7,67 N 6,66
Gef. : C 74,00 H 7,89 N 6,74

Beispiel 45

2-[7,8,9,10-Tetrahydro-11-(2-pyridyl)-6H-azepino[1,2-a]indol-2-yloxy]-2-methyl-propansäure-äthylester

Hergestellt aus 7,8,9,10-Tetrahydro-11-(2-pyridyl)-6H-azepino-[1,2-a]indol-2-ol und 2-Brom-2-methyl-propansäure-äthylester analog Beispiel 1.
Ausbeute : 45 % der Theorie,
Schmelzpunkt : 98-99 °C.
Ber. : C 73,45 H 7,19 N 7,14
Gef. : C 73,20 H 7,17 N 7,32

Beispiel 46

2-[6,7,8,9-Tetrahydro-10-(4-pyridyl)-pyrido[1,2-a]indol-2-yloxy]-2-methyl-propansäure-äthylester

Hergestellt aus 6,7,8,9-Tetrahydro-10-(4-pyridyl)-pyrido[1,2-a]-indol-2-ol und 2-Brom-2-methyl-propansäure-äthylester analog Beispiel 1.
Ausbeute : 34,9 % der Theorie,
Schmelzpunkt : < 20 °C.
Ber. : C 72,99 H 6,92 N 7,40
Gef. : C 72,40 H 6,90 N 7,48

Beispiel 47

2-[7,8-Dihydro-9-(4-pyridyl)-6H-pyrrolo[1,2-a]indol-2-yloxy]-2-methyl-propansäure-äthylester

Hergestellt aus 7,8-Dihydro-9-(4-pyridyl)-6H-pyrrolo[1,2-a]indol-2-ol und 2-Brom-2-methyl-propansäure-äthylester analog Beispiel 1.
Ausbeute : 51 % der Theorie,
Schmelzpunkt : < 20 °C.
Ber. : C 72,51 H 6,64 N 7,69
Gef. : C 72,40 H 6,66 N 7,75

Beispiel 48

2-Methyl-2-[2-methyl-1-propyl-3-(4-pyridyl)-1H-indol-6-yloxy]-propansäure-äthylester

Hergestellt aus 2-Methyl-1-propyl-3-(4-pyridyl)-1H-indol-6-ol und 2-Brom-2-methyl-propansäure-äthylester analog Beispiel 1.
Ausbeute : 29,2 % der Theorie,
Schmelzpunkt : < 20 °C.
Ber. : C 72,60 H 7,42 N 7,36
Gef. : C 72,40 H 7,66 N 7,51

Beispiel 49

2-Methyl-2-[2-methyl-1-propyl-3-(4-pyridyl)-1H-indol-4-yloxy]-propansäure-äthylester

Hergestellt aus 2-Methyl-1-propyl-3-(4-pyridyl)-1H-indol-4-ol und 2-Brom-2-methyl-propansäure-äthylester analog Beispiel 1.
Ausbeute : 21,9 % der Theorie,
Schmelzpunkt : 122-124 °C.
Ber. : C 72,60 H 7,42 N 7,36
Gef. : C 72,80 H 7,55 N 7,32

Beispiel 50

2-Methyl-2-[2-methyl-3-phenyl-1-propyl-1H-indol-6-yloxy]propansäure-äthylester

Hergestellt aus 2-Methyl-3-phenyl-1-propyl-1H-indol-6-ol und 2-Brom-2-methyl-propansäure-äthylester analog Beispiel 1 (Lösungsmittel : Glykoldimethyläther).
Ausbeute : 36,7 % der Theorie,
Schmelzpunkt : < 20 °C.
Ber. : C 75,96 H 7,70 N 3,69
Gef. : C 76,30 H 7,72 N 3,86

Beispiel 51

2-Methyl-2-[2-methyl-3-phenyl-1H-indol-4-yloxy]propansäure-äthylester

Hergestellt aus 2-Methyl-3-phenyl-1H-indol-4-ol und 2-Brom-2-methyl-propansäure-äthylester analog Beispiel 1.
Ausbeute : 40 % der Theorie,
Schmelzpunkt : < 20 °C.

Beispiel 52

2-(3-Benzyl-2-methyl-1-propyl-1H-indol-5-yloxy)-2-methyl-propansäure-äthylester

Hergestellt aus 3-Benzyl-2-methyl-1-propyl-1H-indol-5-ol und 2-Brom-2-methyl-propansäure-äthylester analog Beispiel 1.
Ausbeute : 55 % der Theorie,
Schmelzpunkt : < 20 °C.
Ber. : C 76,30 H 7,94 N 3,56
Gef. : C 76,40 H 7,94 N 3,57

Beispiel 53

2-[3-(4-Chlorbenzyl)-2-methyl-1-propyl-1H-indol-5-yloxy]-2-methyl-propansäure-äthylester

Hergestellt aus 3-(4-Chlorbenzyl)-2-methyl-1-propyl-1H-indol-5-ol und 2-Brom-2-methyl-propansäure-äthylester analog Beispiel 1.
Ausbeute : 57 % der Theorie,
Schmelzpunkt : < 20 °C.
Ber. : C 70,16 H 7,07 N 3,27
Gef. : C 70,25 H 7,24 N 3,35

Beispiel 54

2-{3-[2-(4-Chlor-phenyl)äthyl]-2-methyl-1-allyl-1H-indol-5-yloxy}-2-methyl-propansäure-äthylester

2 g (5 mMol) 2-{3-[2-(4-Chlor-phenyl)äthyl]-2-methyl-1H-indol-5-yloxy}-2-methyl-propansäure-äthylester werden in 20 ml absolutem Dimethylformamide mit 0,24 g (~ 5 mMol) 55 %iger Natriumhydrid-Immersion in Öl in das Natriumsalz überführt und mit 1,2 g (10 mMol) Allylbromid bei Raumtemperatur alkyliert. Nach Abdestillieren des Lösungsmittels im Vakuum wird der Trockenrückstand in Toluol gelöst, filtriert und über eine Kieselgelsäule mit Toluol als Laufmittel gereinigt.
Ausbeute : 36 % der Theorie,
Schmelzpunkt : < 20 °C.
Ber. : C 71,0 H 6,88 N 3,18
Gef. : C 71,5 H 7,20 N 3,42

Beispiel 55

2-Methyl-2-[2-methyl-3-phenyl-1-propyl-1H-indol-4-yloxy]propansäure-äthylester

Hergestellt aus 2-Methyl-2-[2-methyl-3-phenyl-1H-indol-4-yloxy]-propansäure-äthylester und Propylbromid analog Beispiel 54.
Ausbeute : 23 % der Theorie,
Schmelzpunkt : < 20 °C.
Ber. : C 75,96 H 7,70 N 3,69
Gef. : C 76,20 H 7,72 N 3,57

Beispiel 56

2-[3-(4-Methoxy-phenyl)-1,2-dimethyl-1H-indol-7-yloxy]-2-methyl-propansäure-äthylester

Hergestellt aus 3-(4-Methoxy-phenyl)-1,2-dimethyl-1H-indol-7-ol und 2-Brom-2-methyl-propansäure-äthylester analog Beispiel 1.
Ausbeute : 58 % der Theorie,
Schmelzpunkt : 108 °C.
Ber. : C 72,5 H 7,15 N 3,67
Gef. : C 72,2 H 7,27 N 3,66

Beispiel 57

2-[3-(2-Methoxy-phenyl)-1,2-dimethyl-1H-indol-5-yloxy]-2-methyl-propansäure

1 g (2,6 mMol) 2-[3-(2-Methoxy-phenyl)-1,2-dimethyl-1H-indol-5-yloxy]-2-methyl-propansäure-äthylester werden in 18 ml Methanol gelöst und mit einer Lösung von 0,35 g Kaliumhydroxid in 2 ml Wasser versetzt. Bei Raumtemperatur werden innerhalb von 3-4 Stunden 6 ml Wasser so langsam zugetropft, daß es zu keiner bleibenden Ausfällung des Esters kommt. Nach 2-3 stündigem Rühren wird das Methanol am Rotationsverdampfer abdestilliert. Dann werden 20 ml Wasser zugefügt und die alkalische Lösung zweimal mit Äther extrahiert. Nach dem Ansäuern der wässrigen Phase mit Salzsäure, Extraktion mit Äther, Trocknung der Ätherextrakte über Natriumsulfat, Abdestillieren des Äthers wird nach Behandeln des erhaltenen Rückstandes mit Petroläther die Säure erhalten.

Ausbeute : 0,9 g (97 % der Theorie),
Schmelzpunkt : 120 °C,
Ber. : C 71,20 H 6,76 N 4,05
Gef. : C 71,30 H 6,57 N 3,90

Beispiel 58

2-[3-(4-Methoxy-phenyl)-1,2-dimethyl-1H-indol-5-yloxy]-2-methyl-propansäure

Hergestellt aus 2-[3-(4-Methoxy-phenyl)-1,2-dimethyl-1H-indol-5-yloxy]-2-methyl-propansäure-äthylester durch alkalische hydrolyse analog Beispiel 57.

Ausbeute : 60 % der Theorie,
Schmelzpunkt : 106 °C.
Ber. : C 71,20 H 6,56 N 3,97
Gef. : C 70,50 H 6,58 N 3,81

Beispiel 59

2-[3-(4-Methoxy-phenyl)-2-methyl-1-propyl-1H-indol-5-yloxy]-2-methyl-propansäure

Hergestellt aus 2-[3-(4-Methoxy-phenyl)-2-methyl-1-propyl-1H-indol-5-yloxy]-2-methyl-propansäure-äthylester durch alkalische Hydrolyse analog Beispiel 57.

Ausbeute : 44 % der Theorie,
Schmelzpunkt : 99 °C.
Ber. : C 72,45 H 7,14 N 3,67
Gef. : C 73,00 H 7,10 N 3,81

Beispiel 60

2-[3-(2-Chlor-phenyl)-2-methyl-1-propyl-1H-indol-5-yloxy]-2-methyl-propansäure

Hergestellt aus 2-[3-(2-Chlor-phenyl)-2-methyl-1-propyl-1H-indol-5-yloxy]-2-methyl-propansäure-äthylester durch alkalische Hydrolyse analog Beispiel 57.

Ausbeute : 49 % der Theorie,
Schmelzpunkt : 140 °C.
Ber. : C 68,47 H 6,28 N 3,63
Gef. : C 68,3 H 6,09 N 3,38

Beispiel 61

2-[3-(4-Chlor-phenyl)-1,2-dimethyl-1H-indol-5-yloxy]-2-methyl-propansäure

Hergestellt aus 2-[3-(4-Chlor-phenyl)-1,2-dimethyl-1H-indol-5-yloxy]-2-methyl-propansäure-äthylester durch alkalische Hydrolyse analog Beispiel 57.

Ausbeute : 80 % der Theorie,
Schmelzpunkt : 150 °C.
Ber. : C 67,0 H 5,64 N 3,92
Gef. : C 66,7 H 5,81 N 3,75

Beispiel 62

2-[1-Äthyl-3-(2-brom-phenyl)-2-methyl-1H-indol-5-yloxy]-2-methyl-propansäure

Hergestellt aus 2-[1-Äthyl-3-(2-brom-phenyl)-2-methyl-1H-indol-5-yloxy]-2-methyl-propansäure-äthylester durch alkalische Hydrolyse analog Beispiel 57.

Ausbeute : 58 % der Theorie,
Schmelzpunkt : 114 °C.

Ber.: C 60,6 H 5,33 N 3,36
Gef.: C 60,8 H 5,60 N 3,22

Beispiel 63

2-Methyl-2-[2-methyl-3-(2-methyl-phenyl)-1-propyl-1H-indol-5-yloxy]propansäure

Hergestellt aus 2-Methyl-2-[2-methyl-3-(2-methyl-phenyl)-1-propyl-1H-indol-5-yloxy]propansäure-äthylester durch alkalische Hydrolyse analog Beispiel 57.
Ausbeute: 54 % der Theorie,
Schmelzpunkt: 100 °C.
Ber.: C 75,5 H 7,43 N 3,84
Gef.: C 75,2 H 7,32 N 4,04

Beispiel 64

2-Methyl-2-[2-methyl-3-(3-methyl-phenyl)-1H-indol-5-yloxy]-propansäure

Hergestellt aus 2-Methyl-2-[2-methyl-3-(3-methyl-phenyl)-1H-indol-5-yloxy]propansäure-äthylester durch alkalische Hydrolyse analog Beispiel 57.
Ausbeute: 59 % der Theorie,
Schmelzpunkt: 85 °C.
Ber.: C 74,4 H 6,56 N 4,35
Gef.: C 74,3 H 6,50 N 4,21

Beispiel 65

2-Methyl-2-[1,2-dimethyl-3-(3-methyl-phenyl)-1H-indol-5-yloxy]-propansäure

Hergestellt aus 2-Methyl-2-[1,2-dimethyl-3-(3-methyl-phenyl)-1H-indol-5-yloxy]propansäure-äthylester durch alkalische Hydrolyse analog Beispiel 57.
Ausbeute: 77 % der Theorie,
Schmelzpunkt: 124 °C.
Ber.: C 74,7 H 6,86 N 4,15
Gef.: C 74,6 H 6,92 N 4,20

Beispiel 66

2-[3-(3-Trifluormethyl-phenyl)-1,2-dimethyl-1H-indol-5-yloxy]-2-methyl-propansäure

Hergestellt aus 2-[3-(3-Trifluormethyl-phenyl)-1,2-dimethyl-1H-indol-5-yloxy]-2-methyl-propansäure-äthylester durch alkalische Hydrolyse analog Beispiel 57.
Ausbeute: 80 % der Theorie,
Schmelzpunkt: 130 °C.
Ber.: C 64,55 H 5,15 N 3,58
Gef.: C 64,20 H 5,28 N 3,61

Beispiel 67

2-[1-Butyl-3-(4-fluor-phenyl)-2-methyl-1H-indol-5-yloxy]-2-methyl-propansäure

Hergestellt aus 2-[1-Butyl-3-(4-fluor-phenyl)-2-methyl-1H-indol-5-yloxy]-2-methyl-propansäure-äthylester durch alkalische Hydrolyse analog Beispiel 57.
Ausbeute: 69 % der Theorie,
Schmelzpunkt: 120 °C.
Ber.: C 72,1 H 6,85 N 3,65
Gef.: C 72,0 H 6,87 N 3,56

Beispiel 68

2-[3-(3-Chlor-phenyl)-1,2-dimethyl-1H-indol-5-yloxy]-2-methyl-propansäure

Hergestellt aus 2-[3-(3-Chlor-phenyl)-1,2-dimethyl-1H-indol-5-yloxy]-2-methyl-propansäure-äthylester durch alkalische Hydrolyse analog Beispiel 57.

Ausbeute : 79 % der Theorie,
Schmelzpunkt : 162 °C.
Ber. : C 67,15 H 5,6 N 3,92
Gef. : C 67,2 H 5,68 N 3,72

Beispiel 69

2-{3-[2-(4-Methoxy-phenyl)äthyl]-2-methyl-1-propyl-1H-indol-5-yloxy}-2-methyl-propansäure

Hergestellt aus 2-{3-[2-(4-Methoxy-phenyl)äthyl]-2-methyl-1-propyl-1H-indol-5-yloxy}-2-methyl-propansäure-äthylester durch alkalische Hydrolyse analog Beispiel 57.
Ausbeute : 86 % der Theorie,
Schmelzpunkt : < 20 °C.
Ber. : C 73,50 H 7,65 N 3,43
Gef. : C 73,57 H 7,98 N 3,50

Beispiel 70

2-{3-[2-(4-Chlor-phenyl)äthyl]-2-methyl-1-propyl-1H-indol-5-yloxy}-2-methyl-propansäure

Hergestellt aus 2-{3-[2-(4-Chlor-phenyl)äthyl]-2-methyl-1-propyl-1H-indol-5-yloxy}-2-methyl-propansäure-äthylester durch alkalische Hydrolyse analog Beispiel 57.
Ausbeute : 92 % der Theorie,
Schmelzpunkt : < 20 °C.
Ber. : C 70,0 H 6,85 N 3,39
Gef. : C 70,5 H 6,74 N 3,43

Beispiel 71

2-Methyl-2-[1,2-dimethyl-3-(2-phenyl-äthyl)-1H-indol-5-yloxy]-propansäure

Hergestellt aus 2-Methyl-2-[1,2-dimethyl-3-(2-phenyl-äthyl)-1H-indol-5-yloxy]propansäure-äthylester durch alkalische Hydrolyse analog Beispiel 57.
Ausbeute : 84 % der Theorie,
Schmelzpunkt : 68 °C.
Ber. : C 75,00 H 7,15 N 3,98
Gef. : C 75,00 H 7,31 N 3,72

Beispiel 72

2-Methyl-2-[2-methyl-3-(2-phenyl-äthyl)-1-propyl-1H-indol-5-yloxy]propansäure

Hergestellt aus 2-Methyl-2-[2-methyl-3-(2-phenyl-äthyl)-1-propyl-1H-indol-5-yloxy]propansäure-äthylester durch alkalische Hydrolyse analog Beispiel 57.
Ausbeute : 75 % der Theorie,
Schmelzpunkt : < 20 °C.
Ber. : C 76,00 H 7,50 N 3,70
Gef. : C 75,50 H 7,55 N 3,44

Beispiel 73

2-{3-[2-(4-Chlor-phenyl)äthyl]-2-methyl-1-allyl-1H-indol-5-yloxy}-2-methyl-propansäure

Hergestellt aus 2-{3-[2-(4-Chlor-phenyl)äthyl]-2-methyl-1-allyl-1H-indol-5-yloxy}-2-methyl-propansäure-äthylester durch alkalische Hydrolyse analog Beispiel 57.
Ausbeute : 67 % der Theorie,
Schmelzpunkt : < 20 °C.
Ber. : C 70,0 H 6,37 N 3,40
Gef. : C 70,0 H 6,80 N 3,38

Beispiel 74

2-Methyl-2-[3-methyl-2-phenyl-1-propyl-1H-indol-5-yloxy]propansäure

Hergestellt aus 2-Methyl-2-[3-methyl-2-phenyl-1-propyl-1H-indol-5-yloxy]propansäure-äthylester

durch alkalische Hydrolyse analog Beispiel 57.
Ausbeute : 73 % der Theorie,
Schmelzpunkt : 135 °C.
Ber. : C 75,35 H 7,17 N 3,98
Gef. : C 75,00 H 7,33 N 4,06

Beispiel 75

2-[2-(4-Chlorphenyl)-3-methyl-1-propyl-1H-indol-5-yloxy]-2-methyl-propansäure

Hergestellt aus 2-[2-(4-Chlorphenyl)-3-methyl-1-propyl-1H-indol-5-yloxy]-2-methyl-propansäure-äthylester durch alkalische durch alkalische Hydrolyse analog Beispiel 57.
Ausbeute : 63 % der Theorie,
Schmelzpunkt : 110 °C.
Ber. : C 68,55 H 6,26 N 3,65
Gef. : C 68,45 H 6,31 N 3,61

Beispiel 76

2-[2-(4-Methoxy-phenyl)-3-methyl-1-propyl-1H-indol-5-yloxy]-2-methyl-propansäure

Hergestellt aus 2-[2-(4-Methoxy-phenyl)-3-methyl-1-propyl-1H-indol-5-yloxy]-2-methyl-propansäure-äthylester durch alkalische Hydrolyse analog Beispiel 57.
Ausbeute : 84 % der Theorie,
Schmelzpunkt : 138 °C.
Ber. : C 72,5 H 7,15 N 3,67
Gef. : C 72,8 H 7,23 N 3,56

Beispiel 77

2-[7,8-Dihydro-9-(4-pyridyl)-6H-pyrrolo[1,2-a]indol-2-yloxy]-2-methyl-propansäure-hydrochlorid

Hergestellt aus 2-[7,8-Dihydro-9-(4-pyridyl)-6H-pyrrolo[1,2-a]-indol-2-yloxy]-2-methyl-propansäure-äthylester durch alkalische Hydrolyse analog Beispiel 57. Beim Ansäuern der wässrigen alkalischen Lösung mit 2N Salzsäure fällt das Hydrochlorid aus, das aus Äthanol umkristallisiert wird.
Ausbeute : 62 % der Theorie,
Schmelzpunkt : 150 °C (Zers.).
Ber. : C 64,43 H 5,68 N 7,52
Gef. : C 64,20 H 5,88 N 7,66

Beispiel 78

2-[7,8,9,10-Tetrahydro-11-(4-pyridyl)-6H-azepino[1,2-a]indol-2-yloxy]-2-methyl-propansäure

Hergestellt aus 2-[7,8,9,10-Tetrahydro-11-(4-pyridyl)-6H-azepino-[1,2-a]indol-2-yloxy]-2-methyl-propansäure-äthylester durch alkalische Hydrolyse analog Beispiel 57.
Ausbeute : 89 % der Theorie,
Schmelzpunkt : 204-206 °C.
Ber. : C 72,51 H 6,64 N 7,69
Gef. : C 72,40 H 6,49 N 7,57

Beispiel 79

2-[7,8,9,10-Tetrahydro-11-(4-pyridyl)-6H-azepino[1,2-a]indol-3-yloxy]-2-methyl-propansäure

Hergestellt aus 2-[7,8,9,10-Tetrahydro-11-(4-pyridyl)-6H-azepino-[1,2-a]indol-3-yloxy]-2-methyl-propansäure-äthylester durch alkalische Hydrolyse analog Beispiel 57.
Ausbeute : 95 % der Theorie,
Schmelzpunkt : 224-225 °C (Zers.).
Ber. : C 72,51 H 6,64 N 7,69
Gef. : C 72,25 H 6,76 N 7,99

Beispiel 80

2-Methyl-2-[3-methyl-1-propyl-2-(4-pyridyl)-1H-indol-5-yloxy]-propansäure-hydrochlorid

Hergestellt aus 2-Methyl-2-[3-methyl-1-propyl-2-(4-pyridyl)-1H-indol-5-yloxy]propansäure-äthylester durch Hydrolyse mit verdünnter Salzsäure bei Siedetemperatur. Nach Abdestillieren der Salzsäure im Vakuum wird aus Aceton umkristallisiert.

Ausbeute : 50 % der Theorie,
Schmelzpunkt : 200 °C.
Ber. : C 64,85 H 6,42 N 7,20 Cl 9,12
Gef. : C 64,60 H 6,58 N 7,15 Cl 9,40

## Beispiel 81

2-Methyl-2-[1,3-dimethyl-2-(4-pyridyl)-1H-indol-5-yloxy]propansäure-hydrochlorid

Hergestellt aus 2-Methyl-2-[1,3-dimethyl-2-(4-pyridyl)-1H-indol-5-yloxy]propansäure-äthylester analog Beispiel 80.

Ausbeute : 60 % der Theorie,
Schmelzpunkt : 202 °C.
Ber. : C 63,2 H 5,85 N 7,75
Gef. : C 63,5 H 5,95 N 7,58

## Beispiel 82

2-Methyl-2-[1,2-dimethyl-3-(4-pyridyl)-1H-indol-5-yloxy]propansäure-hydrochlorid

Hergestellt aus 2-Methyl-2-[1,2-dimethyl-3-(4-pyridyl)-1H-indol-5-yloxy]propansäure-äthylester durch saure Hydrolyse analog Beispiel 80.

Ausbeute : 56 % der Theorie,
Schmelzpunkt : 220 °C.
Ber. : C 63,3 H 5,87 N 7,75 Cl 9,82
Gef. : C 63,2 H 6,06 N 7,75 Cl 9,98

## Beispiel 83

2-Methyl-2-[2-isopropyl-1-methyl-3-(4-pyridyl)-1H-indol-5-yloxy]-propansäure-hydrochlorid

Hergestellt aus 2-Methyl-2-[2-isopropyl-1-methyl-3-(4-pyridyl)-1H-indol-5-yloxy]propansäure-äthylester durch saure Hydrolyse analog Beispiel 80.

Ausbeute : 82 % der Theorie,
Schmelzpunkt : 235 °C.
Ber. : C 64,8 H 6,50 N 7,20 Cl 9,14
Gef. : C 63,9 H 6,54 N 7,29 Cl 9,33

## Beispiel 84

2-Methyl-2-[1-methyl-2-propyl-3-(4-pyridyl)-1H-indol-5-yloxy]-propansäure-hydrochlorid

Hergestellt aus 2-Methyl-2-[1-methyl-2-propyl-3-(4-pyridyl)-1H-indol-5-yloxy]propansäure-äthylester durch saure Hydrolyse analog Beispiel 80.

Ausbeute : 74 % der Theorie,
Schmelzpunkt : 219 °C.
Ber. : C 64,8 H 6,50 N 7,2 Cl 9,14
Gef. : C 64,5 H 6,68 N 7,0 Cl 9,16

## Beispiel 85

2-[2-Benzyl-1-methyl-3-(4-pyridyl)-1H-indol-5-yloxy]-2-methyl-propansäure-hydrochlorid

Hergestellt aus 2-[2-Benzyl-1-methyl-3-(4-pyridyl)-1H-indol-5-yloxy]-2-methyl-propansäure-äthylester durch saure Hydrolyse analog Beispiel 80.

Ausbeute : 36 % der Theorie,
Schmelzpunkt : ~ 185 °C.

## Beispiel 86

2-Methyl-2-[2-methyl-1-propyl-3-(4-pyridyl)-1H-indol-5-yloxy]-propansäure-hydrochlorid

Hergestellt aus 2-Methyl-2-[2-methyl-1-propyl-3-(4-pyridyl)-1H-indol-5-yloxy]propansäure-äthylester durch saure Hydrolyse analog Beispiel 80.

Ausbeute : 60 % der Theorie,
Schmelzpunkt : 240 °C.
Ber. : C 64,6 H 6,50 N 7,22 Cl 9,16
Gef. : C 64,3 H 6,65 N 7,04 Cl 9,54

Beispiel 87

2-[1-Isobutyl-2-methyl-3-(4-pyridyl)-1H-indol-5-yloxy]-2-methyl-propansäure-hydrochlorid

Hergestellt aus 2-[1-Isobutyl-2-methyl-3-(4-pyridyl)-1H-indol-5-yloxy]-2-methyl-propansäure-äthylester durch saure Hydrolyse analog Beispiel 80.

Ausbeute : 79 % der Theorie,
Schmelzpunkt : 226 °C.
Ber. : C 65,3 H 6,75 N 6,95 Cl 8,80
Gef. : C 64,8 H 6,99 N 6,77 Cl 8,65

Beispiel 88

2-[1-Butyl-2-methyl-3-(4-pyridyl)-1H-indol-5-yloxy]-2-methyl-propansäure-hydrochlorid

Hergestellt aus 2-[1-Butyl-2-methyl-3-(4-pyridyl)-1H-indol-5-yloxy]-2-methyl-propansäure-äthylester durch saure Hydrolyse analog Beispiel 80.

Ausbeute : 75 % der Theorie,
Schmelzpunkt : 210 °C.
Ber. : C 65,3 H 6,75 N 6,95
Gef. : C 64,5 H 6,81 N 6,95

Beispiel 89

2-[3-(4-Chlorphenyl)-1,2-dimethyl-1H-indol-5-yloxy]-2-methyl-propansäure

8,13 g (0,03 Mol) 3-(4-Chlorphenyl)-1,2-dimethyl-1H-indol-5-ol werden in 100 ml wasserfreiem Aceton mit 10,1 g (0,18 Mol) gepulvertem Kaliumhydroxid 30 Minuten bei Raumtemperatur gerührt. Dann kühlt man im Eisbad und tropft eine Lösung von 8,0 g (0,045 Mol) 1,1,1-Trichlor-tert.butanol-semihydrat in 30 ml Aceton zu. Man rührt 3 Stunden bei Raumtemperatur nach und erhitzt schließlich 2 Stunden am Rückfluß. Nach Einengen im Vakuum wird mit 2N Salzsäure angesäuert und mit Essigester extrahiert. Nach dem Trocknen der Extrakte über Natriumsulfat engt man ein und chromatographiert an Kieselgel mit Chloroform/Methanol 8 : 2 als Laufmittel.

Ausbeute : 4,4 g (41 % der Theorie),
Schmelzpunkt : 150 °C.
Ber. : C 67,00 H 5,64 N 3,92
Gef. : C 67,10 H 5,55 N 3,66

Beispiel 90

2-[3-(4-Methoxyphenol)-2-methyl-1-propyl-1H-indol-5-yloxy]-2-methyl-propansäure

11,5 g (0,03 Mol) 3-(4-Methoxy-phenyl)-2-methyl-1-propyl-1H-indol-5-ol werden in 40 g (0,69 Mol) Aceton mit 10 g (0,25 Mol) gepulvertem Natriumhydroxid versetzt. Dann erhitzt man auf Rückflußtemperatur und tropft langsam 10 g (0,083 Mol) Chloroform zu. Nach 4 Stunden Nacherhitzen versetzt man mit Eiswasser, säuert mit 2N Salzsäure an und extrahiert mit Chloroform. Die über Natriumsulfat getrockneten Extrakte werden eingeengt und an Kieselgel im Laufmittel Chloroform/Methanol 9 : 1 chromatographiert.

Ausbeute : 7,0 g (47,5 % der Theorie),
Schmelzpunkt : 97 °C.

Beispiel 91

2-[3-(4-Methoxy-phenyl)-2-methyl-1-propyl-1H-indol-5-yloxy]-2-methyl-propansäure

5,9 g (0,02 Mol) 3-(4-Methoxy-phenyl)-2-methyl-1-propyl-1H-indol-5-ol, 5,1 g (0,03 Mol) 2-Brom-2-methyl-propansäure und 15 g Kaliumcarbonat werden in 100 ml Aceton 48 Stunden am Rückfluß

gekocht. Dann entfernt man das Aceton im Vakuum und säuert den Rückstand mit 2N Salzsäure an. Nach Extraktion mit Essigester wird über Natriumsulfat getrocknet und eingeengt. Zur Reinigung wird an Kieselgel mit Chloroform/Methanol 9 : 1 als Laufmittel chromatographiert.

Ausbeute : 2,2 g (29 % der Theorie),
Schmelzpunkt : 99 °C.
Ber. : C 72,45 H 7,14 N 3,67
Gef. : C 72,80 H 7,20 N 3,41

Beispiel 92

2-[3-(4-Chlorphenyl)-1,2-dimethyl-1H-indol-5-yloxy]-2-methyl-propansäure

5,4 g (0,02 Mol) 3-(4-Chlorphenyl)-1,2-dimethyl-1H-indol-5-ol und 5,1 g (0,03 Mol) 2-Brom-2-methylpropansäure werden 2 Stunden auf 90-130 °C erhitzt. Anschließend wird in Chloroform/Methanol 8 : 2 aufgenommen und an Kieselgel chromatographiert. Der Eindampfrückstand des Eluats wird mit Cyclohexan verrührt und abgesaugt.

Ausbeute : 0,78 g (11 % der Theorie),
Schmelzpunkt : 150 °C.

Beispiel 93

2-[3-(2-Methoxy-phenyl)-1,2-dimethyl-1H-indol-5-yloxy]-2-methyl-propansäure-methylester

5,34 g (0,02 Mol) 3-(2-Methoxy-phenyl)-1,2-dimethyl-1H-indol-5-ol, 5,4 g (0,03 Mol) 2-Brom-2-methylpropansäure-methylester und 14 g (0,1 Mol) Kaliumcarbonat werden in 250 ml Methyl-äthylketon 16 Stunden am Rückfluß gekocht. Dann filtriert man vom Niederschlag ab, engt das Filtrat ein und chromatographiert zur Reinigung an Kieselgel mit Toluol/Aceton 30 : 1.

Ausbeute : 2,25 g (31 % der Theorie),
Schmelzpunkt : < 20 °C.
Ber. : C 71,91 H 6,85 N 3,81
Gef. : C 71,68 H 6,73 N 3,56

Beispiel 94

2-[3-(3-Chlor-phenyl)-1,2-dimethyl-1H-indol-5-yloxy]-2-methyl-propansäure-propylester

Hergestellt aus 3-(3-Chlor-phenyl)-1,2-dimethyl-1H-indol-5-ol und 2-Brom-2-methyl-propansäure-propylester analog Beispiel 93.

Ausbeute : 52 % der Theorie,
Schmelzpunkt : < 20 °C.
Ber. : C 69,07 H 6,35 N 3,50
Gef. : C 68,89 H 6,20 N 3,25

Beispiel 95

2-[3-(4-Chlor-phenyl)-1,2-dimethyl-1H-indol-5-yloxy]-2-methyl-propansäure-butylester

Hergestellt aus 3-(4-Chlor-phenyl)-1,2-dimethyl-1H-indol-5-ol und 2-Brom-2-methyl-propansäure-butylester analog Beispiel 93.

Ausbeute : 41 % der Theorie,
Schmelzpunkt : < 20 °C.
Ber. : C 69,63 H 6,81 N 3,38
Gef. : C 69,85 H 6,66 N 3,21

Beispiel 96

2-[3-(4-Chlor-phenyl)-1,2-dimethyl-1H-indol-5-yloxy]-2-methyl-propansäure-amylester

Hergestellt aus 3-(4-Chlor-phenyl)-1,2-dimethyl-1H-indol-5-ol und 2-Brom-2-methyl-propansäure-amylester analog Beispiel 93.

Ausbeute : 63 % der Theorie,
Schmelzpunkt : < 20 °C.
Ber. : C 70,16 H 7,06 N 3,27
Gef. : C 70,25 H 7,13 N 3,06

Beispiel 97

2-[7,8,9,10-Tetrahydro-11-(4-pyridyl)-6H-azepino[1,2-a]indol-2-yloxy]-2-methyl-propansäure-natriumsalz

364 mg (1 mMol) 2-[7,8,9,10-Tetrahydro-11-(4-pyridyl)-6H-azepino[1,2-a]indol-2-yloxy]-2-methyl-propansäure werden in 20 ml Äthanol suspendiert und mit 1 ml einer Lösung von 460 mg (20 mMol) Natrium in 20 ml Äthanol versetzt. Dabei wird eine klare Lösung erhalten. Anschließend versetzt man mit 50 ml Äther und saugt den gebildeten gallertigen Niederschlag ab. Nach dem Waschen mit Petroläther erhält man ein weißes Kristallpulver.

Ausbeute : 0,3 g (78 % der Theorie),
Schmelzpunkt : 326-330 °C (Zers.).
Ber. : C 68,38 H 5,99 N 7,25
Gef. : C 68,51 H 6,10 N 7,13

Beispiel 98

2-[3-(4-Chlor-phenyl)-2-methyl-1-propyl-1H-indol-5-yloxy]-2-methyl-propansäure-äthylester

Hergestellt aus 3-(4-Chlor-phenyl)-2-methyl-1-propyl-1H-indol-5-ol und 2-Brom-2-methyl-propansäure-äthylester analog Beispiel 1.

Ausbeute : 73 % der Theorie,
Schmelzpunkt : < 20 °C.

Beispiel 99

2-[3-(4-Chlor-phenyl)-1-hexyl-2-methyl-1H-indol-5-yloxy]-2-methyl-propansäure-äthylester

Hergestellt aus 3-(4-Chlor-phenyl)-1-hexyl-2-methyl-1H-indol-5-ol und 2-Brom-2-methyl-propansäure-äthylester analog Beispiel 1.

Ausbeute : 56 % der Theorie,
Schmelzpunkt : < 20 °C.

Beispiel 100

2-[3-(4-Chlor-phenyl)-1-dodecyl-2-methyl-1H-indol-5-yloxy]-2-methyl-propansäure-äthylester

Hergestellt aus 3-(4-Chlor-phenyl)-1-dodecyl-2-methyl-1H-indol-5-ol und 2-Brom-2-methyl-propansäure-äthylester analog Beispiel 1.

Ausbeute : 57 % der Theorie,
Schmelzpunkt : < 20 °C.

Beispiel 101

2-[3-(4-Chlor-phenyl)-2-methyl-1-propyl-1H-indol-5-yloxy]-2-methyl-propansäure

Hergestellt aus 2-[3-(4-Chlor-phenyl)-2-methyl-1-propyl-1H-indol-5-yloxy]-2-methyl-propansäure-äthylester durch alkalische Hydrolyse analog Beispiel 57.

Ausbeute : 68 % der Theorie,
Schmelzpunkt : 124 °C.
Ber. : C 68,47 H 6,27 N 3,63
Gef. : C 68,80 H 6,44 N 3,56

Beispiel 102

2-[3-(4-Chlor-phenyl)-1-hexyl-2-methyl-1H-indol-5-yloxy]-2-methyl-propansäure

Hergestellt aus 2-[3-(4-Chlor-phenyl)-1-hexyl-2-methyl-1H-indol-5-yloxy]-2-methyl-propansäure-äthylester durch Hydrolyse analog Beispiel 57.

Ausbeute : 43 % der Theorie,
Schmelzpunkt : 112 °C.
Ber. : C 70,16 H 7,07 N 3,27
Gef. : C 70,21 H 7,28 N 3,36

Beispiel 103

2-[3-(4-Chlor-phenyl)-1-dodecyl-2-methyl-1H-indol-5-yloxy]-2-methyl-propansäure

Hergestellt aus 2-[3-(4-Chlor-phenyl)-1-dodecyl-2-methyl-1H-indol-5-yloxy]-2-methyl-propansäure-äthylester durch alkalische Hydrolyse analog Beispiel 57.

Ausbeute : 57 % der Theorie,
Schmelzpunkt : < 20 °C.
Ber. : C 72,7 H 8,27 N 2,74
Gef. : C 72,0 H 8,02 N 2,54

Beispiel 104

2-Methyl-2-[2-(4-nitro-phenyl)-1-propyl-1H-indol-6-yloxy]-propansäure-äthylester

Hergestellt aus 2-(4-Nitrophenyl)-1-propyl-1H-indol-6-ol und 2-Brom-2-methyl-propansäure-äthylester analog Beispiel 1.

Ausbeute : 38,4 % der Theorie,
Schmelzpunkt : < 20 °C.
Ber. : C 67,30 H 6,38 N 6,83
Gef. : C 66,95 H 6,01 N 6,41

Beispiel 105

2-[2-(4-Acetamido-phenyl)-1-propyl-1H-indol-6-yloxy]-2-methyl-propansäure-äthylester

5,4 g (13 mMol) 2-Methyl-2-[2-(4-nitro-phenyl)-1-propyl-1H-indol-6-yloxy]propansäure-äthylester und 2,5 g Raney-Nickel werden in 150 ml Acetanhydrid suspendiert und bei 20 °C unter einem Druck von 5 bar mit Wasserstoff hydriert. Anschließend wird vom Katalysator abfiltriert und eingeengt. Nach Chromatographie an Kieselgel mit Toluol/Aceton (3 : 1) als Fließmittel wird mit Petroläther/Äther (3 : 1) verrieben und abgesaugt.

Ausbeute : 1,45 g (26,2 % der Theorie),
Schmelzpunkt : 109-112 °C.
Ber. : C 71,06 H 7,16 N 6,63
Gef. : C 70,74 H 7,26 N 6,68

Beispiel 106

2-[2-(4-Amino-phenyl)-3-methyl-1-propyl-1H-indol-5-yloxy]-2-methyl-propansäure-äthylester

Hergestellt aus 2-(4-Amino-phenyl)-3-methyl-1-propyl-1H-indol-5-ol und 2-Brom-2-methyl-propansäure-äthylester analog Beispiel 1.

Ausbeute : 78,6 % der Theorie,
Schmelzpunkt : < 20 °C.
Ber. : C 73,06 H 7,67 N 7,10
Gef. : C 72,50 H 7,59 N 7,06

Beispiel 107

2-[2-(4-Acetamido-phenyl)-3-methyl-1-propyl-1H-indol-5-yloxy]-2-methyl-propansäure-äthylester

1 g (2,53 mMol) 2-[2-(4-Amino-phenyl)-3-methyl-1-propyl-1H-indol-5-yloxy]-2-methyl-propansäure-äthylester und 0,25 g (2,5 mMol) Triäthylamin werden in 15 ml Chloroform gelöst und unter Eiskühlung tropfenweise mit 0,2 g (2,55 mMol) Acetylchlorid versetzt. Nach einigen Stunden bei Raumtemperatur wird mit Wasser versetzt und mit Chloroform extrahiert. Die Chloroformextrakte werden getrocknet, eingeengt und der Rückstand zur Reinigung an Kieselgel mit Toluol/Aceton (5 : 1) als Fließmittel chromatographiert.

Ausbeute : 0,75 g (68,7 % der Theorie),
Schmelzpunkt : < 20 °C.
Ber. : C 71,53 H 7,39 N 6,42
Gef. : C 71,20 H 7,15 N 6,40

Beispiel 108

2-Methyl-2-[3-methyl-2-(4-nitro-phenyl)-1-propyl-1H-indol-5-yloxy]propansäure-äthylester

Hergestellt aus 3-Methyl-2-(4-nitro-phenyl)-1-propyl-1H-indol-5-ol und 2-Brom-2-methyl-propansäure-äthylester analog Beispiel 1.
Ausbeute : 45,3 % der Theorie,
Schmelzpunkt : < 20 °C.
Ber. : C 67,90 H 6,65 N 6,60
Gef. : C 68,20 H 6,88 N 6,35

Beispiel 109

2-[7,8,9,10-Tetrahydro-11-phenyl-6H-azepino[1,2-a]indol-2-yloxy]-2-methyl-propansäure-äthylester

Hergestellt aus 7,8,9,10-Tetrahydro-11-phenyl-6H-azepino[1,2-a]-indol-2-ol und 2-Brom-2-methyl-propansäure-äthylester analog Beispiel 1.
Ausbeute : 50 % der Theorie,
Schmelzpunkt : 84-86 °C.
Ber. : C 76,69 H 7,47 N 3,58
Gef. : C 76,22 H 7,25 N 3,54

Beispiel 110

2-Methyl-2-[1-propyl-3-(4-pyridylmethyl)-1H-indol-5-yloxy]-propansäure-äthylester

Hergestellt aus 1-Propyl-3-(4-pyridylmethyl)-1H-indol-5-ol und 2-Brom-2-methyl-propansäure-äthylester analog Beispiel 1.
Ausbeute : 46 % der Theorie,
Schmelzpunkt : < 20 °C.
Ber. : C 72,62 H 7,42 N 7,36
Gef. : C 72,40 H 7,50 N 7,29

Beispiel 111

2-(3,4-Dibenzyl-1-propyl-1H-indol-5-yloxy)-2-methyl-propansäure-äthylester

Hergestellt aus 3,4-Dibenzyl-1-propyl-1H-indol-5-ol und 2-Brom-2-methyl-propansäure-äthylester analog Beispiel 1.
Ausbeute : 37 % der Theorie,
Schmelzprunkt : < 20 °C.
Ber. : C 79,28 H 7,51 N 2,98
Gef. : C 78,89 H 7,53 N 3,00

Beispiel 112

2-Methyl-2-[2-phenyl-1-propyl-1H-indol-5-yloxy]propansäure-äthylester

Hergestellt aus 2-Phenyl-1-propyl-1H-indol-5-ol und 2-Brom-2-methyl-propansäure-äthylester analog Beispiel 1.
Ausbeute : 41 % der Theorie,
Schmelzpunkt : 58 °C.
Ber. : C 75,58 H 7,46 N 3,83
Ger. : C 75,90 H 7,75 N 3,79

Beispiel 113

2-Methyl-2-[2-phenyl-1-propyl-1H-indol-5-yloxy]propansäure

Hergestellt aus 2-Methyl-2-[2-phenyl-1-propyl-1H-indol-5-yloxy]propansäure-äthylester durch alkalische Hydrolyse analog Beispiel 57.
Ausbeute : 65 % der Theorie,
Schmelzpunkt : 110 °C
Ber. : C 74,75 H 6,86 N 4,15
Gef. : C 74,60 H 6,86 N 4,17

Beispiel 114

2-[2-(4-Acetamino-phenyl)-1-propyl-1H-indol-5-yloxy]-2-methyl-propansäure-äthylester

Hergestellt aus 2-(4-Acetamino-phenyl)-1-propyl-1H-indol-5-ol und 2-Brom-2-methyl-propansäure-äthylester analog Beispiel 1.
Ausbeute : 45 % der Theorie,
Schmelzpunkt : 113-115 °C.
Ber. : C 71,06 H 7,16 N 6,63
Gef. : C 71,23 H 7,09 N 6,62

Beispiel 115

2-[2-(4-Acetamino-phenyl)-1-propyl-1H-indol-6-yloxy]-2-methyl-propansäure

Hergestellt durch alkalische Verseifung von 2-[2-(4-Acetamino-phenyl)-1-propyl-1H-indol-6-yloxy]-2-methyl-propansäure-äthyl-ester analog Beispiel 57.
Ausbeute : 95 % der Theorie,
Schmelzpunkt : 144-146 °C.
Ber. : C 70,03 H 6,64 N 7,10
Gef. : C 69,70 H 6,93 N 7,08

Beispiel 116

2-Methyl-2-[2-methyl-1-propyl-3-(4-pyridyl)-1H-indol-5-yloxy]-propanol

Zu 1,0 g (26 mMol) Lithiumaluminiumhydrid in 50 ml absolutem Tetrahydrofuran gibt man unter Rühren tropfenweise eine Suspension von 1,0 g (2,6 mMol) 2-Methyl-2-[2-methyl-1-propyl-3-(4-pyridyl)-1H-indol-5-yloxy]propansäure-hydrochlorid. Dann rührt man eine Stunde bei 60 °C. Anschließend zersetzt man mit 2 N Natronlauge. Nach Abfiltrieren des Natriumaluminatniederschlags wird eingeengt und an Kieselgel säulenchromatographisch gereinigt (Fließmittel : Essigsäureäthylester).
Ausbeute : 600 mg (69 % der Theorie),
Schmelzpunkt : < 20 °C.
Ber. : C 74,52 H 7,74 N 8,27
Gef. : C 74,30 H 7,84 N 8,20

Beispiel 117

2-[3-(4-Chlorphenyl)-1,2-dimethyl-1H-indol-5-yloxy]-2-methyl-propanol

Hergestellt aus 2-[3-(4-Chlorphenyl)-1,2-dimethyl-1H-indol-5-yloxy]-2-methyl-propansäure und Lithiumaluminiumhybrid analog Beispiel 116.
Ausbeute : 31 % der Theorie,
Schmelzpunkt : 105 °C.
Ber. : C 69,86 H 6,45 N 4,07
Gef. : C 70,10 H 6,72 N 4,56

Beispiel 118

2-Methyl-2-[2-methyl-1-propyl-3-(4-pyridyl)-1H-indol-5-yloxy]-propansäure

3,38 g (10 mMol) 2-Methyl-2-[2-methyl-1-propyl-3-(4-pyridyl)-1H-indol-5-yloxy]propanol in 50 ml Dioxan werden nacheinander mit 300 mg Natriumhydroxid in 5 ml Wasser und 3,4 g Kaliumpermanganat, gelöst in 30 ml Wasser, versetzt. Man rührt 12 Stunden nach, filtriert, engt ein und neutralisiert mit verdünnter Schwefelsäure. Anschließend wird mit Chloroform extrahiert, über Natriumsulfat getrocknet und eingeengt. Der Einengungsrückstand wird aus Äthanol umkristallisiert.
Schmelzpunkt : 210 °C.

Beispiel 119

2-Methyl-2-]2-methyl-1-propyl-3-(4-pyridyl)-1H-indol-5-yloxy]-propansäure-propylamid

0,5 g (1,3 mMol) 2-Methyl-2-[2-methyl-1-propyl-3-(4-pyridyl)-1H-indol-5-yloxy]propansäure-hydrochlorid werden mit 4 ml Thionylchlorid 15 Minuten auf dem Dampfbad erwärmt. Anschließend wird überschüssiges Thionylchlorid im Vakuum abgezogen, der Rückstand in 100 ml Chloroform gelöst und unter Rühren tropfenweise mit 4 ml Propylamin versetzt. Nach einer Stunde wird eingeengt und an Kieselgel mit Essigsäureäthylester als Fließmittel säulenchromatographisch gereinigt.
Ausbeute : 0,2 g (39,5 % der Theorie),

Schmelzpunkt : 103-105 °C.
Ber. : C 73,24 H 7,94 N 10,68
Gef. : C 73,20 H 7,89 N 10,27

Beispiel 120

2-Methyl-2-[2-methyl-1-propyl-3-(4-pyridyl)-1H-indol-5-yloxy]-propansäure-morpholid

Hergestellt aus 2-Methyl-2-[2-methyl-1-propyl-3-(4-pyridyl)-1H-indol-5-yloxy]propansäure-hydrochlorid und Morpholin analog Beispiel 119.
Ausbeute : 83 % der Theorie,
Schmelzpunkt : 114-116 °C.
Ber. : C 71,23 H 7,41 N 9,97
Gef. : C 71,00 H 7,57 N 9,42

Beispiel 121

2-[3-(4-Chlorphenyl)-1,2-dimethyl-1H-indol-5-yloxy]-2-methyl-propansäure-piperidid

0,5 g (1,4 mMol) 2-[3-(4-Chlorphenyl)-1,2-dimethyl-1H-indol-5-yloxy]-2-methyl-propansäure werden in 25 ml Tetrahydrofuran gelöst und mit 0,24 g (1,4 mMol) N,N'-Carbonyl-diimidazol 4 Stunden auf dem Dampfbad erwärmt. Anschließend fügt man 0,24 g (2,8 mMol) Piperidin zu und erhitzt 12 stunden am Rückfluß. Nach dem Einengen wird mit verdünnter Salzsäure versetzt und mit Äther extrahiert. Der eingedampfte Ätherrückstand wird mit Toluol/Essigester (8 : 2) an Kieselgel säulenchromatographisch gereinigt.
Ausbeute : 400 mg (67 % der Theorie),
Schmelzpunkt : 165 °C.
Ber. : C 70,65 H 6,88 N 6,59
Gef. : C 70,30 H 6,82 N 6,51

Beispiel 122

2-[3-(4-Chlorphenyl)-1,2-dimethyl-1H-indol-5-yloxy]-2-methyl-propansäure-thiomorpholid

Hergestellt aus 2-[3-(4-Chlorphenyl)-1,2-dimethyl-1H-indol-5-yloxy]-2-methyl-propansäure und Thiomorpholin analog Beispiel 121.
Ausbeute : 70 % der Theorie,
Schmelzpunkt : 168 °C.
Ber. : C 65,06 H 6,14 N 6,32
Gef. : C 64,75 H 6,18 N 6,09

Beispiel 123

2-[3-(4-Chlorphenyl)-1,2-dimethyl-1H-indol-5-yloxy]-2-methyl-propansäure-dipropylamid

Hergestellt aus 2-[3-(4-Chlorphenyl)-1,2-dimethyl-1H-indol-5-yloxy]-2-methyl-propansäure und Dipropylamin analog Beispiel 121.
Ausbeute : 21 % der Theorie,
Schmelzpunkt : < 20 °C.
Ber. : C 70,81 H 7,54 N 6,35
Gef. : C 70,50 H 7,73 N 6,53

Beispiel 124

2-[3-(4-Chlorphenyl)-1,2-dimethyl-1H-indol-5-yloxy]-2-methyl-propansäuren-N-methyl-cyclohexylamid

Hergestellt aus 2-[3-(4-Chlorphenyl)-1,2-dimethyl-1H-indol-5-yloxy]-2-methyl-propansäure und N-Methyl-cyclohexylamin analog Beispiel 121.
Ausbeute : 25 % der Theorie,
Schmelzpunkt : 158 °C.
Ber. : C 71,58 H 7,34 N 6,19
Gef. : C 71,75 H 7,35 N 5,95

Beispiel 125

2-[3-(4-Chlorphenyl)-1,2-dimethyl-1H-indol-5-yloxy]-2-methyl-propansäure-diallylamid

Hergestellt aus 2-[3-(4-Chlorphenyl)-1,2-dimethyl-1H-indol-5-yloxy]-2-methyl-propansäure und Diallylamin analog Beispiel 121.

Ausbeute : 24 % der Theorie,
Schmelzpunkt : 82 °C.
Ber. : C 71,46 H 6,69 N 6,41
Gef. : C 71,20 H 6,86 N 6,47

Beispiel 126

2-[3-(4-Chlorphenyl)-1,2-dimethyl-1H-indol-5-yloxy]-2-methyl-propansäureamid

Hergestellt aus 2-[3-(4-Chlorphenyl)-1,2-dimethyl-1H-indol-5-yloxy]-2-methyl-propansäure und einer gesättigten Lösung von Ammoniak in absolutem Tetrahydrofuran analog Beispiel 121.

Ausbeute : 90 % der Theorie,
Schmelzpunkt : 198 °C.
Ber. : C 67,31 H 5,93 N 7,85
Gef. : C 67,00 H 6,08 N 7,50

Beispiel 127

2-Methyl-2-[2-methyl-1-propyl-3-(4-pyridyl)-1H-indol-5-yloxy]-propansäureamid

Hergestellt aus 2-Methyl-2-[2-methyl-1-propyl-3-(4-pyridyl)-1H-indol-5-yloxy]propansäure-hydrochlorid und wässrigem konz. Ammoniak in Dioxan als Lösungsmittel analog Beispiel 119.

Ausbeute : 66 % der Theorie,
Schmelzpunkt : 145 °C.
Ber. : C 71,77 H 7,17 N 11,96
Gef. : C 71,52 H 7,35 N 11,78

Beispiel 128

2-Methyl-2-[2-methyl-1-propyl-3-(4-pyridyl)-1H-indol-5-yloxy]-propansäure-methylamid

Hergestellt aus 2-Methyl-2-[2-methyl-1-propyl-3-(4-pyridyl)-1H-indol-5-yloxy]propansäure-hydrochlorid und Methylamin in Dioxan analog Beispiel 119.

Ausbeute : 74 % der Theorie,
Schmelzpunkt : 123-125 °C.
Ber. : C 72,30 H 7,45 N 11,50
Gef. : C 72,42 H 7,75 N 11,08

Beispiel 129

2-Methyl-2-[2-methyl-1-propyl-3-(4-pyridyl)-1H-indol-5-yloxy]-propansäure-cyclohexylamid

Hergestellt aus 2-Methyl-2-[2-methyl-1-propyl-3-(4-pyridyl)-1H-indol-5-yloxy]propansäure-hydrochlorid und Cyclohexylamin analog Beispiel 119.

Ausbeute : 53 % der Theorie,
Schmelzpunkt : 124 °C.
Ber. : C 74,79 H 8,14 N 9,69
Gef. : C 74,91 H 8,24 N 9,50

Beispiel 130

2-Methyl-2-[2-methyl-1-propyl-3-(4-pyridyl)-1H-indol-5-yloxy]-propansäure-diäthylamid

Hergestellt aus 2-Methyl-2-[2-methyl-1-propyl-3-(4-pyridyl)-1H-indol-5-yloxy]propansäure-hydrochlorid und Diäthylamin analog Beispiel 119.

Ausbeute : 40 % der Theorie,
Schmelzpunkt : 134-136 °C.
Ber. : C 73,68 H 8,16 N 10,31
Gef. : C 73,60 H 8,60 N 9,97

Beispiel 131

2-Methyl-2-[2-methyl-1-propyl-3-(4-pyridyl)-1H-indol-5-yloxy]-propansäurenitril-hydrochlorid

Hergestellt aus 2-Methyl-1-propyl-3-(4-pyridyl)-1H-indol-5-ol und 2-Brom-2-methyl-propansäurenitril analog Beispiel 93 in Toluol als Lösungsmittel und unter Zusatz von katalytischen Mengen 18-Krone-6 als Phasen-transfer-Katalysator.

Ausbeute : 31,5 % der Theorie,
Schmelzpunkt : 236-240 °C (Zers.).
Ber. : C 68,18 H 6,54 N 11,36 Cl 9,59
Gef. : C 68,50 H 6,56 N 11,65 Cl 9,74

Beispiel 132

2-[3-(4-Chlor-phenyl)-1,2-dimethyl-1H-indol-5-yloxy]-2-methyl-propansäurenitril

Hergestellt aus 3-(4-Chlor-phenyl)-1,2-dimethyl-1H-indol-5-ol und 2-Brom-2-methyl-propansäurenitril analog Beispiel 1.

Ausbeute : 33,3 % der Theorie,
Schmelzpunkt : 151-153 °C.
Ber. : C 70,89 H 5,65 N 8,27 Cl 10,46
Gef. : C 70,87 H 5,88 N 8,14 Cl 10,75

Beispiel 133

2-[3-(4-Chlor-phenyl)-1,2-dimethyl-1H-indol-5-yloxy]-2-methyl-propansäure

Eine Lösung von 6,0 g (60 mMol) Chromtrioxid in 50 ml Eisessig und 5 ml Wasser wird tropfenweise zu einer Mischung aus 19,0 g (55 mMol) 2-[3-(4-Chlor-phenyl)-1,2-dimethyl-1H-indol-5-yloxy]-2-methyl-propanol, 50 ml Eisessig, 10 ml Wasser und 5 ml konz. Schwefelsäure unter Kühlen und Rühren getropft. Nach Stehen über Nacht wird auf dem Dampfbad eine Stunde erwärmt. Anschließend wird mit Chloroform extrahiert. Die Chloroformextrakte werden getrocknet, eingeengt und an Kieselgel mit Chloroform/Methanol (8 : 2) als Fließmittel chromatographiert.

Schmelzpunkt : 149 °C.

Beispiel 134

2-[3-(4-Chlor-phenyl)-1,2-dimethyl-1H-indol-5-yloxy]-2-methyl-propansäureäthylester

3,4 g (10 mMol) 2-[3-(4-Chlor-phenyl)-1,2-dimethyl-1H-indol-5-yloxy]-2-methyl-propansäurenitril werden in 100 ml absolutem Äthanol gelöst und mit Chlorwasserstoffgas gesättigt. Nach sechstägigem Stehen bei Raumtemperatur wird eingeengt, mit Wasser versetzt und mit Essigester extrahiert. Die Extrakte werden eingeengt und an Kieselgel mit Toluol/Essigester (9 : 1) als Fließmittel säulenchromatographisch gereinigt.

Ausbeute : 2,2 g (58 % der Theorie),
Schmelzpunkt : 120 °C.
Ber. : C 68,10 H 6,26 N 3,61
Gef. : C 68,50 H 6,47 N 3,51

Beispiel 135

2-Methyl-2-[2-methyl-1-propyl-3-(4-pyridyl)-1H-indol-5-yloxy]-propansäureäthylester

Hergestellt aus 2-Methyl-2-[2-methyl-1-propyl-3-(4-pyridyl)-1H-indol-5-yloxy]propansäurenitril und äthanolischer Salzsäure analog Beispiel 134.

Ausbeute : 47 % der Theorie,
Schmelzpunkt : 90 °C.

Beispiel 136

2-[3-(4-Chlor-phenyl)-1,2-dimethyl-1H-indol-5-yloxy]-2-methyl-propansäure

3,4 g (10 mMol) 2-[3-(4-Chlor-phenyl)-1,2-dimethyl-1H-indol-5-yloxy]-2-methyl-propansäurenitril werden in 40 ml Äthylenglykol mit 1,2 g (21,1 mMol) Kaliumhydroxid auf 120 °C erhitzt. Nach 4 Stunden verdünnt man mit Wasser und extrahiert mit Essigester. Die wässrig alkalische Phase wird anschließend angesäuert und mit Chloroform extrahiert. Nach dem Trocknen und Einengen der Extrakte erhält man die gewünschte Verbindung.

Ausbeute : 2,18 g (61 % der Theorie),
Schmelzpunkt : 148-149 °C.

Beispiel 137

2-[3-(4-Chlor-phenyl)-1,2-dimethyl-1H-indol-5-yloxy]-2-methyl-orthopropansäure-äthylester

3,4 g (10 mMol) 2-[3-(4-Chlor-phenyl)-1,2-dimethyl-1H-indol-5-yloxy]propansäurenitril werden in 50 ml absolutem Äther gelöst. Nach Zugabe von 0,51 g (11 mMol) absolutem Äthanol wird Chlorwasserstoffgas bei 0 °C bis zur Sättigung eingeleitet. Nach Stehen über Nacht wird vom gebildeten Niederschlag abgesaugt. Das so erhaltene Imidoäthylesterhydrochlorid wird anschließend in 150 ml absolutem Äthanol 2 Tage bei Raumtemperatur gerührt. Nach Neutralisieren der Reaktionslösung mit Natriumäthylat wird eingeengt. Der Einengungsrückstand wird in Äther aufgenommen, filtriert und erneut eingeengt.
Schmelzpunkt : 20 °C.

Beispiel 138

2-[3-(4-Chlorphenyl)-1,2-dimethyl-1H-indol-5-yloxy]-2-methyl-propansäure-piperidid

Hergestellt aus 3-(4-Chlorphenyl)-1,2-dimethyl-1H-indol-5-ol und 2-Brom-2-methyl-propansäure-piperidid analog Beispiel 1.
Schmelzpunkt : 165 °C.

Beispiel I

Suppositorien zu 30 mg 2-Methyl-2-[3-methyl-1-propyl-2-(4-pyridyl)-1H-indol-5-yloxy]propansäure-hydrochlorid

Zusammensetzung :
1 Zäpfchen enthält :

| | |
|---|---|
| Wirksubstanz | 0,030 g |
| Zäpfchenmasse (z.B. Witepsol W 45 | 1,336 g |
| Witepsol E 75) | 0,334 g |
| | 1,700 g |

Herstellung :

Der gemahlene Wirkstoff wird unter Rühren in das aud 40 °C temperierte aufgeschmolzene Gemisch der Zäpfchenmassen eingetragen und die Schmelze in gekühlte Gießformen ausgegossen. Nach dem völligen Erstarren werden die Suppositorien den Formen entnommen und in geeigneter Weise verpackt.

Beispiel II

Gelatine-Steckkapseln zu 5 mg 2-Methyl-2-[3-methyl-1-propyl-2-(4-pyridyl)-1H-indol-5-yloxy]propansäure-hydrochlorid

1 Kapsel enthält :

| | |
|---|---|
| Wirksubstanz | 5,0 mg |
| Maisstärke getr. | 100,0 mg |
| Maisstärke pulv. | 93,0 mg |
| Magnesiumstearat | 2,0 mg |
| | 200,0 mg |

Herstellung :

Wirkstoff und Hilfsstoffe werden gemischt, durch ein Sieb der Maschenweite 0,75 mm gegeben und in einem geeignetem Mischer homogen verteilt. Das Pulver wird auf einem Kapselfüll- und Schließbautomaten in Hartgelatine-Steckkapseln der Größe 3 (Parke Davis) abgefüllt.

Beispiel III

Tabletten zu 25 mg 2-Methyl-2-[3-methyl-1-propyl-2-(4-pyridyl)-1H-indol-5-yloxy]propansäure-hydrochlorid

1 Tablette enthält :

| | |
|---|---|
| Wirksubstanz | 25,0 mg |
| Milchzucker | 35,0 mg |
| Maisstärke | 15,0 mg |

| | |
|---|---|
| Polyvinylpyrrolidon | 4,5 mg |
| Magnesiumstearat | 0,5 mg |
| | 80,0 mg |

Herstellung :

Der Wirkstoff wird mit Milchzucker und Stärke gemischt und danach mit der wäßrigen Polyvinylpyrrolidon-Lösung gleichmäßig befeuchtet.

| | |
|---|---|
| Feuchtsiebung : | 1,5 mm-Maschenweite |
| Trocknung : | Umlufttrockenschrank bei 45 °C |
| Trockensiebung : | 1,0 mm-Maschenweite |

Das trockne Granulat wird nach Zumischung des Schmiermittels zu Tabletten verpreßt. Tabletten : 6 mm ∅, beidseitige Facette, einseitige Teilkerbe, biplan.

Beispiel IV

Dragées zu 25 mg 2-Methyl-2-[3-methyl-1-propyl-2-(4-pyridyl)-1H-indol-5-yloxy]propansäure-hydrochlorid

Herstellung der preßfertigen Mischung analog Beispiel II.

Verpressung zu bikonvexen Dragéekernen von 80,0 mg Gewicht, 6 mm ∅ und Wölbungsradius 5 mm.

Die Kerne werden mit einer praxisüblichen Zuckerdragiersuspension auf ein Gewicht von 110 mg im Dragierkessel dragiert und anschließend mit einer Poliersuspension poliert.

**Ansprüche** (für die Vertragsstaaten BE, CH, DE, FR, GB, IT, LU, NL, SE)

1. Neue Indolderivate der allgemeinen Formel

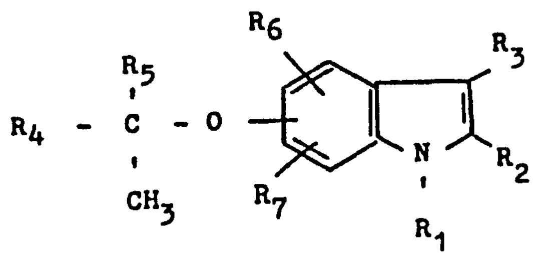

(I)

in der

$R_1$ ein Wasserstoffatom, eine geradkettige oder verzweigte Alkylgruppe mit 1 bis 12 Kohlenstoffatomen oder eine Alkenylgruppe mit 3 bis 5 Kohlenstoffatomen,

$R_2$ eine Alkylgruppe mit 1 bis 3 Kohlenstoffatomen, die durch eine Phenylgruppe substituiert sein kann, eine gegebenenfalls durch ein Halogenatom, eine Methoxy-, Amino-, Nitro-oder Acetamidogruppe substituierte Phenylgruppe, eine Pyridylgruppe oder $R_1$ zusammen mit $R_2$ ein Alkylengruppe mit 3 bis 5 Kohlenstoffatomen,

$R_3$ eine Alkylgruppe mit 1 bis 3 Kohlenstoffatomen, die durch eine gegebenenfalls durch eine Methylgruppe, Methoxygruppe, Trifluormethylgruppe oder ein Halogenatom substituierte Phenylgruppe oder eine Pyridylgruppe substituiert sein kann, eine gegebenenfalls durch eine Methylgruppe, Methoxygruppe, Trifluormethylgruppe oder ein Halogenatom substituierte Phenylgruppe, eine Pyridyl-, Pyridyl-N-oxid-oder Chinolylgruppe oder einer der Reste $R_2$ oder $R_3$ auch ein Wasserstoffatom, wenn der andere der Reste jeweils einen der bei der Definition der Reste $R_2$ oder $R_3$ erwähnten aromatischen, araliphatischen, heteroaromatischen oder heteroaraliphatischen Rest darstellt,

$R_4$ eine Carboxyl- oder Nitrilgruppe, eine Trialkoxymethylgruppe, in der jeder Alkoxyrest 1 bis 3 Kohlenstoffatome enthalten kann, eine Carbalkoxygruppe mit insgesamt 2 bis 8 Kohlenstoffatomen, eine gegebenenfalls durch Alkylgruppen mit 1 bis 7 Kohlenstoffatomen, Cycloalkylgruppen mit 3 bis 7 Kohlenstoffatomen oder Alkenylgruppen mit 3 bis 7 Kohlenstoffatomen mono- oder disubstituierte Aminocarbonylgruppe, wobei die Substituenten der Aminogruppe gleich oder verschieden sein können, eine Hydroxymethyl-, Piperidinocarbonyl-, Morpholinocarbonyl- oder Thiomorpholinocarbonylgruppe,

$R_5$ eine Alkylgruppe mit 1 bis 3 Kohlenstoffatomen,

$R_6$ ein Wasserstoffatom, eine Methyl- oder Benzylgruppe und

$R_7$ ein Wasserstoffatom oder die Methylgruppe bedeuten, und deren physiologisch verträgliche Salze mit anorganischen oder organischen Säuren und auch Basen, wenn $R_4$ die Carboxylgruppe darstellt.

2. Neue Indolderivate der allgemeinen Formel I gemäß Anspruch 1, in der

$R_1$ ein Wasserstoffatom, eine Alkylgruppe mit 1 bis 12 Kohlenstoffatomen oder die Allylgruppe,

$R_2$ ein Wasserstoffatom, eine Alkylgruppe mit 1 bis 3 Kohlenstoffatomen oder $R_1$ und $R_2$ zusammen die Propylen-, Butylen- oder Pentylengruppe und

$R_3$ eine gegebenenfalls durch ein Fluor-, Chlor- oder Bromatom, eine Methyl-, Methoxy- oder Trifluormethylgruppe substituierte Phenylgruppe, eine gegebenenfalls durch ein Chloratom oder eine Methoxygruppe substituierte 2-Phenyläthylgruppe, eine Benzyl-, Chlorbenzyl-, Pyridyl-(2)-, Pyridyl-(4)-, Chinolyl-(2)- oder 4-Pyridylmethylgruppe oder

$R_2$ eine gegebenenfalls durch ein Chloratom, eine Methoxy-, Nitro-, Amino- oder Acetaminogruppe substituierte Phenylgruppe, eine Benzyl- oder Pyridyl-(4)-gruppe und

$R_3$ ein Wasserstoffatom oder eine Alkylgruppe mit 1 bis 3 Kohlenstoffatomen,

$R_4$ eine Carboxyl- oder Nitrilgruppe, eine Carbalkoxygruppe mit insgesamt 2 bis 6 Kohlenstoffatomen oder eine Hydroxymethylgruppe,

$R_5$ die Methylgruppe,

$R_6$ ein Wasserstoffatom, die Methyl- oder Benzylgruppe und

$R_7$ ein Wasserstoffatom oder die Methylgruppe bedeuten, und deren physiologisch verträgliche Salze mit anorganischen oder organischen Säuren und auch Basen, wenn $R_4$ die Carboxylgruppe darstellt.

3. Neue Indolderivate der allgemeinen Formel I gemäß Anspruch 1, in der

$R_1$ eine Alkylgruppe mit 1 bis 4 Kohlenstoffatomen,

$R_2$ eine Methylgruppe oder $R_1$ und $R_2$ zusammen die n-Pentylengruppe und

$R_3$ eine gegebenenfalls in 3- oder 4-Stellung durch ein Fluor- oder Chloratom, eine Methoxy- oder Trifluormethylgruppe substituierte Phenylgruppe, eine 2-Phenyläthyl-, Pyridyl-(4)- oder Chinolyl-(2)-gruppe oder

$R_2$ eine gegebenenfalls in 3- oder 4-Stellung durch ein Chloratom, eine Methoxy-, Amino- oder Acetaminogruppe substituierte Phenylgruppe oder eine Pyridyl-(4)-gruppe und

$R_3$ ein Wasserstoffatom oder eine Methylgruppe,

$R_4$ eine Carboxylgruppe oder eine Alkoxycarbonylgruppe mit insgesamt 2 bis 4 Kohlenstoffatomen,

$R_5$ die Methylgruppe,

$R_6$ und $R_7$ je ein Wasserstoffatom bedeuten, und deren physiologisch verträgliche Salze mit anorganischen oder organischen Säuren und auch Basen, wenn $R_4$ die Carboxylgruppe darstellt.

4. Neue Indolderivate der allgemeinen Formel I gemäß Anspruch 1, in der

$R_1$ eine Alkylgruppe mit 1 bis 4 Kohlenstoffatomen,

$R_2$ eine Methylgruppe oder $R_1$ und $R_2$ zusammen die n-Pentylengruppe,

$R_3$ eine gegebenenfalls in 3- oder 4-Stellung durch ein Fluor- oder Chloratom, eine Methoxy- oder Trifluormethylgruppe substituierte Phenylgruppe, eine 2-Phenyläthyl-, Pyridyl-(4)- oder Chinolyl-(2)-gruppe,

$R_4$ eine Carboxylgruppe oder eine Alkoxycarbonylgruppe mit insgesamt 2 bis 4 Kohlenstoffatomen,

$R_5$ die Methylgruppe,

$R_6$ und $R_7$ je ein Wasserstoffatom bedeuten und deren physiologisch verträgliche Salze mit anorganischen oder organischen Säuren und auch Basen, wenn $R_4$ die Carboxylgruppe darstellt.

5. 2-[3-(4-Chlorphenyl)-1,2-dimethyl-1H-indol-5-yloxy]-2-methyl-propansäure und dessen Salze mit anorganischen oder organischen Basen und Säuren.

6. 2-[1-Butyl-3-(4-fluorphenyl)-2-methyl-1H-indol-5-yloxy]-2-methyl-propansäure und dessen Salze mit anorganischen oder organischen Basen und Säuren.

7. 2-Methyl-2-[2-methyl-1-propyl-3-(4-pyridyl)-1H-indol-5-yloxy]propansäure und dessen Salze mit anorganischen oder organischen Basen und Säuren.

8. Arzneimittel, enthaltend eine verbindung gemäß den Ansprüchen 1 bis 7 neben einem oder mehreren inerten Trägerstoffen und/oder Verdünnungsmitteln.

9. Verwendung einer Verbindung gemäß den Ansprüchen 1 bis 7 zur Herstellung eines Arzneimittels zur Bekämpfung von hyperlipidämien und der Atherosklerose auf nichtchemischem Wege.

10. Verfahren zur Herstellung von neuen Indolderivaten der allgemeinen Formel

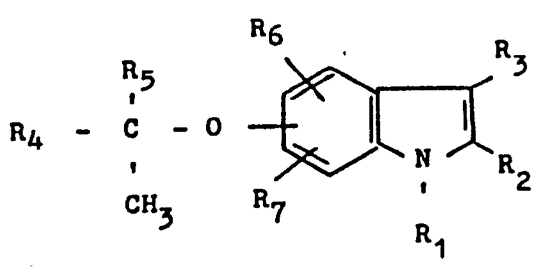

(I)

in der

$R_1$ ein Wasserstoffatom, eine geradkettige oder verzweigte Alkylgruppe mit 1 bis 12 Kohlenstoffatomen oder eine Alkenylgruppe mit 3 bis 5 Kohlenstoffatomen,

$R_2$ eine Alkylgruppe mit 1 bis 3 Kohlenstoffatomen, dir durch eine Phenylgruppe substituiert sein kann, eine gegebenenfalls durch ein Halogenatom, eine Methoxy-, Amino-, Nitro- oder Acetamidogruppe substituierte Phenylgruppe, eine Pyridylgruppe oder $R_1$ zusammen mit $R_2$ eine Alkylengruppe mit 3 bis 5 Kohlenstoffatomen,

$R_3$ eine Alkylgruppe mit 1 bis 3 Kohlenstoffatomen, die durch eine gegebenenfalls durch eine Methyl-, Methoxy- oder Trifluormethylgruppe oder ein Halogenatom substituierte Phenylgruppe oder eine Pyridylgruppe substituiert sein kann, eine gegebenenfalls durch eine Methyl-, Methoxy- oder Trifluormethylgruppe oder ein Halogenatom substituierte Phenylgruppe, eine Pyridyl-, Pyridyl-N-oxid- oder Chinolylgruppe oder einer der Reste $R_2$ oder $R_3$ auch ein Wasserstoffatom, wenn der andere der Reste $R_2$ oder $R_3$ jeweils einen der bei der Definition der Reste $R_2$ oder $R_3$ erwähnten aromatischen, araliphatischen, heteroaromatischen oder heteroaraliphatischen Rest darstellt,

$R_4$ eine Carboxyl- oder Nitrilgruppe, eine Trialkoxymethylgruppe, in der jeder Alkoxyrest 1 bis 3 Kohlenstoffatomen enthalten kann, eine Carbalkoxygruppe mit insgesamt 2 bis 8 Kohlenstoffatomen, eine gegebenenfalls durch Alkylgruppen mit 1 bis 7 Kohlenstoffatomen, Cycloalkylgruppen mit 3 bis 7 Kohlenstoffatomen oder Alkenylgruppen mit 3 bis 7 Kohlenstoffatomen mono- oder disubstituierte Aminocarbonylgruppe, wobei die Substituenten der Aminogruppe gleich oder verschieden sein können, eine Hydroxymethyl-, Piperidinocarbonyl-, Morpholinocarbonyl- oder Thiomorpholinocarbonylgruppe,

$R_5$ eine Alkylgruppe mit 1 bis 3 Kohlenstoffatomen,

$R_6$ ein Wasserstoffatom, eine Methyl- oder Benzylgruppe und

$R_7$ ein Wasserstoffatom oder die Methylgruppe bedeuten, sowie von deren physiologisch verträglichen Salzen mit anorganischen oder organischen Säuren und auch Basen, wenn $R_4$ die Carboxylgruppe darstellt, dadurch gekennzeichnet daß

a) ein Hydroxyindol der allgemeinen Formel

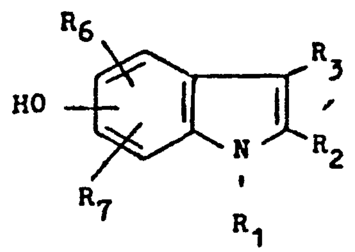

(II)

in der

$R_1$, $R_2$, $R_3$, $R_6$ und $R_7$ wie eingangs definiert sind, bzw. dessen Alkalisalze mit einer Verbindung der allgemeinen Formel

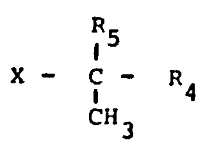

(III)

in der

$R_4$ und $R_5$ wie eingangs definiert sind und

X eine nuckleophile Austrittsgruppe wie ein Halogenatom darstellt, umgesetzt wird oder

b) zur Herstellung von Verbindungen der allgemeinen Formel I, in der $R_4$ die Carboxylgruppe darstellt, ein Hydroxyindol der allgemeinen Formel

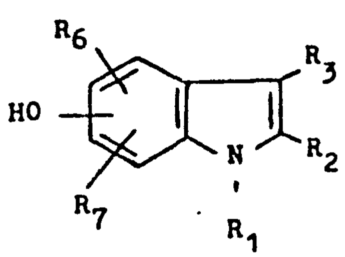

(II)

in der

$R_1$, $R_2$, $R_3$, $R_6$ und $R_7$ wie eingangs definiert sind, bzw. dessen Alkalisalze mit einem gegebenenfalls im Reaktionsgemisch hergestellten Alkohol der allgemeinen Formel

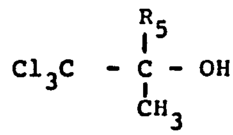

(IV)

in der

$R_5$ wie eingangs definiert ist, in Gegenwart einer anorganischen Base umgesetzt wird oder

c) zur Herstellung von Verbindungen der allgemeinen Formel I, in der $R_1$ kein Wasserstoffatom darstellt, ein Indolderivat der allgemeinen Formel

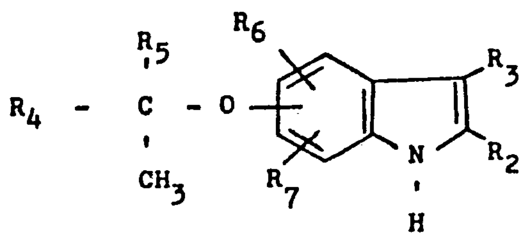

(VI)

in der

$R_2$ bis $R_7$ wie eingangs definiert sind, bzw. dessen Alkalisalze mit einer Verbindung der allgemeinen Formel

$$R_1' - Z \qquad \text{(VII)}$$

in der

$R_1'$ mit Ausnahme von Wasserstoff die für $R_1$ eingangs erwähnten Bedeutungen besitzt und

Z eine nukleophile Austrittsgruppe wie ein Halogenatom oder einen Sulfonsäurerest darstellt, alkyliert wird oder

d) zur Herstellung von Verbindungen der allgemeinen Formel I, in der $R_4$ die Carboxylgruppe darstellt, ein Indolderivat der allgemeinen Formel

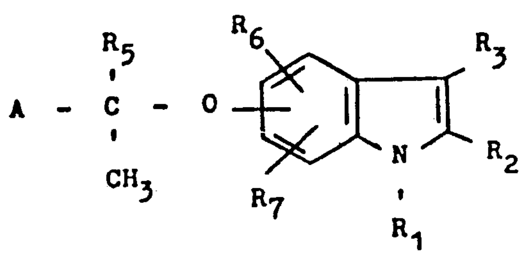

(VIII)

in der

$R_1$ bis $R_3$ und $R_5$ bis $R_7$ wie eingangs definiert sind und

A eine durch Hydrolyse in eine Carboxylgruppe überführbare Gruppe darstellt, hydrolysiert wird oder

e) zur Herstellung von Verbindungen der allgemeinen Formel I, in der $R_4$ die Carboxylgruppe darstellt, ein Indolderivat der allgemeinen Formel

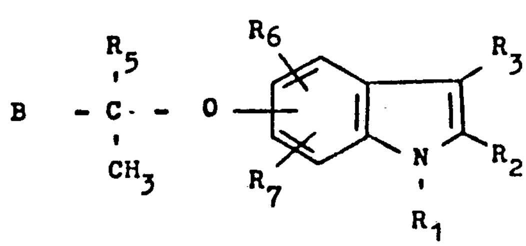

(IX)

in der

$R_1$ bis $R_3$ und $R_5$ bis $R_7$ wie eingangs definiert sind und

B eine durch Oxidation in eine Carboxylgruppe überführbare Gruppe darstellt, oxidiert wird,

und gewünschtenfalls anschließend eine erhaltene Verbindung der allgemeinen Formel I, in der $R_4$ die Carboxylgruppe darstellt, mittels Veresterung oder Amidierung in eine entsprechende Verbindung der allgemeinen Formel I, in der $R_4$ eine Carbalkoxygruppe mit insgesamt 2 bis 8 Kohlenstoffatomen, eine gegebenenfalls durch alkylgruppen mit 1 bis 7 Kohlenstoffatomen, Cycloalkylgruppen mit 3 bis 7 Kohlenstoffatomen oder Alkenylgruppen mit 3 bis 7 Kohlenstoffatomen mono- oder disubstituierte Aminocarbonylgruppe, wobei die Substituenten gleich oder verschieden sein können, eine Piperidinocarbonyl-, Morpholinocarbonyl- oder Thiomorpholinocarbonylgruppe darstellt, übergeführt wird

und/oder eine erhaltene Verbindung der allgemeinen Formel I, in der $R_4$ die Carboxylgruppe oder eine Carbalkoxygruppe mit insgesamt 2 bis 8 Kohlenstoffatomen darstellt, mittels Reduktion in eine entsprechende Verbindung der allgemeinen Formel I, in der $R_4$ die Hydroxymethylgruppe darstellt, übergeführt wird und/oder eine erhaltene Verbindung der allgemeinen Formel I, in der $R_2$ eine Nitrophenylgruppe darstellt, mittels Reduktion in eine entsprechende Verbindung der allgemeinen Formel I, in der $R_2$ eine Aminophenylgruppe darstellt, übergeführt wird

und/oder eine erhaltene Verbindung der allgemeinen Formel I, in der $R_2$ eine Aminophenylgruppe darstellt, mittels Acetylierung in eine entsprechende Verbindung der allgemeinen Formel I, in der $R_2$ eine Acetamidophenylgruppe darstellt, übergeführt wird

und/oder eine erhaltene Verbindung der allgemeinen Formel I in ihre physiologisch verträglichen Salze mit anorganischen oder organischen Säuren und auch Basen, falls $R_4$ die Carboxylgruppe darstellt, übergeführt wird.

**Ansprüche** (für den Vertragsstaat AT)

1. Verfahren zur Herstellung von neuen Indolderivaten der allgemeinen Formel

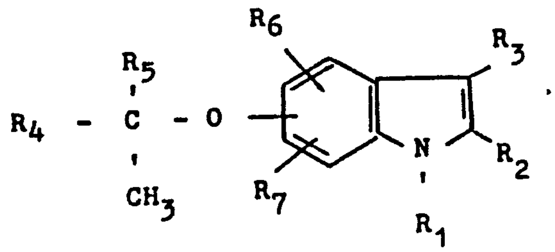

(I)

in der

$R_1$ ein Wasserstoffatom, eine geradkettige oder verzweigte Alkylgruppe mit 1 bis 12 Kohlenstoffatomen oder eine Alkenylgruppe mit 3 bis 5 Kohlenstoffatomen,

$R_2$ eine Alkylgruppe mit 1 bis 3 Kohlenstoffatomen, die durch eine Phenylgruppe substituiert sein kann, eine gegebenenfalls durch ein Halogenatom, eine Methoxy-, Amino-, Nitro- oder Acetamidogruppe substituierte Phenylgruppe, eine Pyridylgruppe oder $R_1$ zusammen mit $R_2$ eine Alkylengruppe mit 3 bis 5 Kohlenstoffatomen,

$R_3$ eine Alkylgruppe mit 1 bis 3 Kohlenstoffatomen, die durch eine gegebenenfalls durch eine Methyl-, Methoxy- oder Trifluormethylgruppe oder ein Halogenatom substituierte Phenylgruppe oder eine Pyridylgruppe substituiert sein kann, eine gegebenenfalls durch eine Methyl-, Methoxy- oder Trifluormethylgruppe oder ein Halogenatom substituierte Phenylgruppe, eine Pyridyl-, Pyridyl-N-oxid- oder Chinolylgruppe oder einer der Reste $R_2$ Oder $R_3$ auch ein Wasserstoffatom, wenn der andere der Reste $R_2$ oder $R_3$ jeweils einen der bei der Definition der Reste $R_2$ oder $R_3$ erwähnten aromatischen, araliphatischen, heteroaromatischen oder heteroaraliphatischen Rest darstellt,

$R_4$ eine Carboxyl- oder Nitrilgruppe, eine Trialkoxymethylgruppe, in der jeder Alkoxyrest 1 bis 3 Kohlenstoffatome enthalten kann, eine Carbalkoxygruppe mit insgesamt 2 bis 8 Kohlenstoffatomen, eine gegebenenfalls durch Alkylgruppen mit 1 bis 7 Kohlenstoffatomen, Cycloalkylgruppen mit 3 bis 7 Kohlenstoffatomen oder Alkenylgruppen mit 3 bis 7 Kohlenstoffatomen mono- oder disubstituierte Aminocarbonylgruppe, wobei die Substituenten der Aminogruppe gleich oder verschieden sein können, eine Hydroxymethyl-, Piperidinocarbonyl-, Morpholinocarbonyl- oder Thiomorpholinocarbonylgruppe,

$R_5$ eine Alkylgruppe mit 1 bis 3 Kohlenstoffatomen,

$R_6$ ein Wasserstoffatom, eine Methyl- oder Benzylgruppe und

$R_7$ ein Wasserstoffatom oder die Methylgruppe bedeuten, sowie von deren physiologisch verträglichen Salzen mit anorganischen oder organischen Säuren und auch Basen, wenn $R_4$ die Carboxylgruppe darstellt, dadurch gekennzeichnet, daß

a) ein Hydroxyindol der allgemeinenen Formel

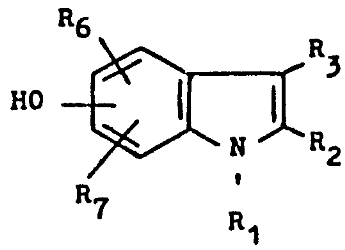

(II)

in der

$R_1$, $R_2$, $R_3$, $R_6$ und $R_7$ wie eingangs definiert sind, bzw. dessen Alkalisalze mit einer Verbindung der allgemeinen Formel

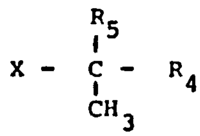

(III)

in der

$R_4$ und $R_5$ wie eingangs definiert sind und

X eine nukleophile Austrittsgruppe wie ein Halogenatom darstellt, umgesetzt wird oder

b) zur Herstellung von Verbindungen der allgemeinen Formel I, in der $R_4$ die Carboxylgruppe darstellt, ein Hydroxyindol der allgemeinen Formel

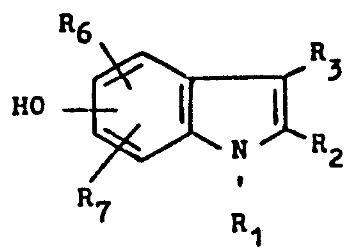

(II)

in der

$R_1$, $R_2$, $R_3$, $R_6$ und $R_7$ wie eingangs definiert sind, bzw. dessen Alkalisalze mit einem gegebenenfalls im Reaktionsgemisch hergestellten Alkohol der allgemeinen Formel

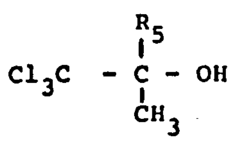

(IV)

in der

$R_5$ wie eingangs definiert ist, in Gegenwart einer anorganischen Base umgesetzt wird oder

c) zur Herstellung von Verbindungen der allgemeinen Formel I, in der $R_1$ kein Wasserstoffatom darstellt, ein Indolderivat der allgemeinen Formel

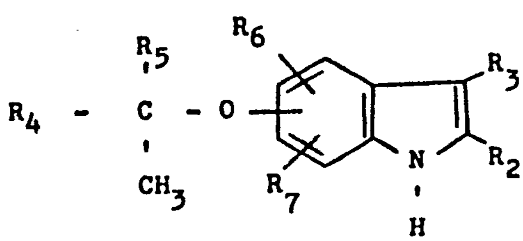

(VI)

in der

$R_2$ bis $R_7$ wie eingangs definiert sind, bzw. dessen Alkalisalze mit einer Verbindung der allgemeinen Formel

$$R_1' - Z \qquad \text{(VII)}$$

in der

$R_1'$ mit Ausnahme von Wasserstoff die für $R_1$ eingangs erwähnten Bedeutungen besitzt und

Z eine nukleophile Austrittsgruppe wie ein Halogenatom oder einen Sulfonsäurerest darstellt, alkyliert wird oder

d) zur Herstellung von Verbindungen der allgemeinen Formel I, in der $R_4$ die Carboxylgruppe darstellt, ein Indolderivat der allgemeinen Formel

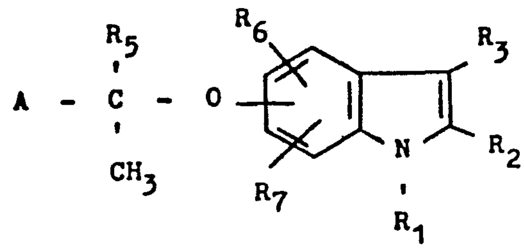

(VIII)

in der

$R_1$ bis $R_3$ und $R_5$ bis $R_7$ wie eingangs definiert sind und

A eine durch Hydrolyse in eine Carboxylgruppe überführbare Gruppe darstellt, hydrolysiert wird oder

e) zur Herstellung von Verbindungen der allgemeinen Formel I, in der $R_4$ die Carboxylgruppe darstellt, ein Indolderivat der allgemeinen Formel

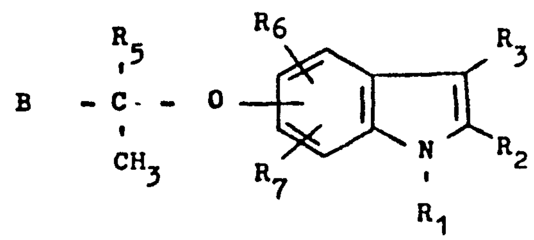

(IX)

in der

$R_1$ bis $R_3$ und $R_5$ bis $R_7$ wie eingangs definiert sind und

B eine durch Oxidation in eine Carboxylgruppe überführbare Gruppe darstellt, oxidiert wird

und gewünschtenfalls anschließend eine erhaltene Verbindung der allgemeinen Formel I, in der $R_4$ die Carboxylgruppe darstellt, mittels Veresterung oder Amidierung in eine entsprechende Verbindung der allgemeinen Formel I, in der $R_4$ eine Carbalkoxygruppe mit insgesamt 2 bis 8 Kohlenstoffatomen, eine gegebenenfalls durch Alkylgruppen mit 1 bis 7 Kohlenstoffatomen, Cycloalkylgruppen mit 3 bis 7 Kohlenstoffatomen oder Alkenylgruppen mit 3 bis 7 Kohlenstoffatomen mono- oder disubstituierte Aminocarbonylgruppe, wobei die Substituenten gleich oder verschieden sein können, eine Piperidinocarbonyl-, Morpholinocarbonyl- oder Thiomorpholinocarbonylgruppe darstellt, übergeführt wird

und/oder eine erhaltene Verbindung der allgemeinen Formel I, in der $R_4$ die Carboxylgruppe oder eine Carbalkoxygruppe mit insgesamt 2 bis 8 Kohlenstoffatomen darstellt, mittels Reduktion in eine entsprechende Verbindung der allgemeinen Formel I, in der $R_4$ die Hydroxymethylgruppe darstellt, übergeführt wird und/oder eine erhaltene Verbindung der allgemeinen Formel I, in der $R_2$ eine Nitrophenylgruppe darstellt, mittels Reduktion in eine entsprechende Verbindung der allgemeinen Formel I, in der $R_2$ eine Aminophenylgruppe darstellt, übergeführt wird

und/oder eine erhaltene Verbindung der allgemeinen Formel I, in der $R_2$ eine Aminophenylgruppe darstellt, mittels Acetylierung in eine entsprechende Verbindung der allgemeinen Formel I, in der $R_2$ eine Acetamidogruppe darstellt, übergeführt wird

und/oder eine erhaltene Verbindung der allgemeinen Formel I in ihre physiologisch verträglichen Salze mit anorganischen oder organischen Säuren und auch Basen, falls $R_4$ die Carboxylgruppe darstellt, übergeführt wird.

2. Verfahren nach Anspruch 1 zur Herstellung von neuen Indolderivaten der allgemeinen Formel

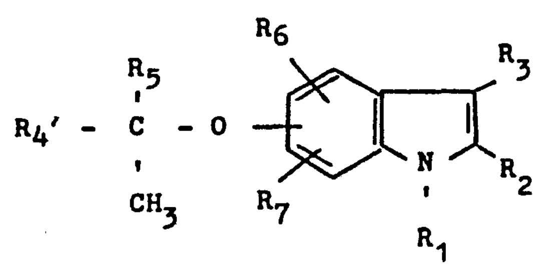

(Ia)

in der

$R_1$, $R_2$, $R_3$, $R_5$, $R_6$ und $R_7$ wie im Anspruch 1 definiert sind und

$R_4'$ eine Carboxylgruppe oder eine Carbalkoxygruppe mit insgesamt 2 bis 8 Kohlenstoffatomen bedeutet, sowie von deren physiologisch verträglichen Salzen mit anorganischen und organischen Säuren oder auch, wenn $R_4'$ eine Carboxylgruppe darstellt, mit anorganischen und organischen Basen, dadurch gekennzeichnet, daß

a) ein Hydroxyindol der allgemeinen Formel

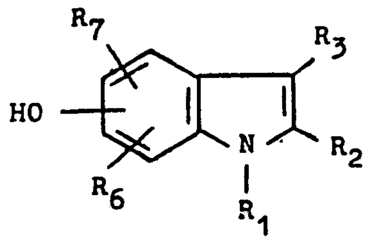

(II)

in der

$R_1$ bis $R_3$, $R_6$ und $R_7$ wie eingangs definiert sind, bzw. dessen Alkalisalze mit einer Verbindung der allgemeinen Formel

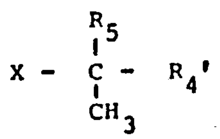

(III)

in der

$R_4'$ und $R_5$ wie eingangs definiert sind und

X eine nukleophile Austrittsgruppe wie ein Halogenatom darstellt, umgesetzt wird oder

b) zur Herstellung von Verbindungen der allgemeinen Formel I, in der $R_4'$ die Carboxylgruppe darstellt, ein Hydroxyindol der allgemeinen Formel

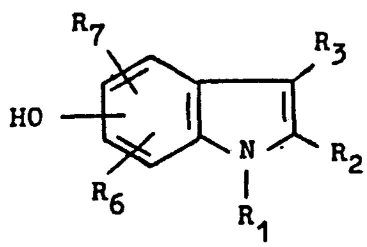

(II)

in der

$R_1$ bis $R_3$, $R_6$ und $R_7$ wie eingangs definiert sind, bzw. dessen Alkalisalze mit einem gegebenenfalls im Reaktionsgemisch hergestellten Alkohol der allgemeinen Formel

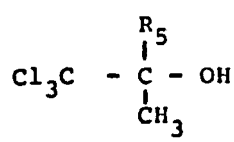

(IV)

in der

$R_5$ wie eingangs definiert ist, in Gegenwart einer anorganischen Base umgesetzt wird oder

c) zur Herstellung von Verbindungen der allgemeinen Formel I, in der $R_1$ kein Wasserstoffatom darstellt, ein Indolderivat der allgemeinen Formel

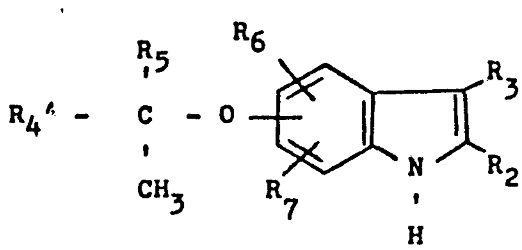

(VIa)

in der

$R_2$, $R_3$, $R_4'$ und $R_5$ bis $R_7$ wie eingangs definiert sind, bzw. dessen Alkalisalze mit einer Verbindung der allgemeinen Formel

$$R_1'—Z \quad (VII)$$

in der

$R_1'$ mit Ausnahme von Wasserstoff die für $R_1$ eingangs erwähnten Bedeutungen besitzt und

Z eine nukleophile Austrittsgruppe wie ein Halogenatom oder einen Sulfonsäurerest darstellt, alkyliert wird und gewünschtenfalls anschließend eine erhaltene Verbindung der allgemeinen Formel I, in der $R_4'$ eine Carbalkoxygruppe mit insgesamt 2 bis 8 Kohlenstoffatomen darstellt, mittels Hydrolyse in eine entsprechende Verbindung der allgemeinen Formel I, in der $R_4'$ eine Carboxylgruppe darstellt, übergeführt wird

und/oder eine erhaltene Verbindung der allgemeinen Formel I, in der $R_4'$ eine Carboxylgruppe darstellt, mittels Veresterung in eine entsprechende Verbindung der allgemeinen Formel I, in der $R_4'$ eine Carbalkoxygruppe mit insgesamt 2 bis 8 Kohlenstoffatomen darstellt, übergeführt wird und/oder eine erhaltene Verbindung der allgemeinen Formel I, in der $R_2$ eine Nitrophenylgruppe darstellt, mittels Reduktion in eine entsprechende Verbindung der allgemeinen Formel I, in der $R_2$ eine Aminophenylgruppe darstellt, übergeführt wird

und/oder eine erhaltene Verbindung der allgemeinen Formel I, in der $R_2$ eine Aminophenylgruppe darstellt, mittels Acetylierung in eine entsprechende Verbindung der allgemeinen Formel I, in der $R_2$ eine Acetamidophenylgruppe darstellt, übergeführt wird

und/oder eine erhaltene Verbindung der allgemeinen Formel I in ihre physiologisch verträglichen Salze mit anorganischen und organischen Säuren oder auch, falls $R_4'$ eine Carboxylgruppe darstellt, mit anorganischen und organischen Basen übergeführt wird.

Priorität : 13.03.1979 (P 29 09 779.8).

3. Verfahren gemäß den Ansprüchen 1 und 2, dadurch gekennzeichnet, daß die Umsetzung a) bei Temperaturen zwischen 0 und 200 °C, vorzugsweise jedoch bei Temperaturen zwischen der Raumtemperatur und der Siedetemperatur des verwendeten Lösungsmittels, z.B. bei Temperaturen zwischen 20 und 100 °C, durchgeführt wird.

4. Verfahren gemäß den Ansprüchen 1 und 2, dadurch gekennzeichnet, daß die Umsetzung b) in einem wasserfreien Keton der allgemeinen Formel

$$R_5—CO—CH_3 \quad (V)$$

in der

$R_5$ wie eingangs definiert ist, als Lösungsmittel durchgeführt wird.

5. Verfahren gemäß den Ansprüchen 1, 2 und 4, dadurch gekennzeichnet, daß die Umsetzung b) bei Temperaturen zwischen 0 °C und der Siedetemperatur des verwendeten Lösungsmittels, z.B. bei Temperaturen zwischen 0 und 100 °C, durchgeführt wird.

6. Verfahren gemäß den Ansprüchen 1, 2, 4 und 5, dadurch gekennzeichnet, daß eine Verbindung der allgemeinen Formel IV im Reaktionsgemisch durch Umsetzung eines entsprechenden Ketons der allgemeinen Formel V mit Chloroform in Gegenwart einer anorganischen Base hergestellt wird.

7. Verfahren gemäß den Ansprüchen 1 und 2, dadurch gekennzeichnet, daß die Alkylierung c) bei Temperaturen zwischen 0 und 200 °C, vorzugsweise jedoch bei Temperaturen zwischen Raumteperatur und der Siedetemperatur des verwendeten Lösungsmittels, z.B. bei Temperaturen zwischen 20 und 100 °C, durchgeführt wird.

8. Verfahren gemäß Anspruch 1, dadurch gekennzeichnet, daß die Umsetzung d) bei der Siedetemperatur des Reaktionsgemisches durchgeführt wird.

9. Verfahren gemäß Anspruch 1, dadurch gekennzeichnet, daß die Umsetzung e) bei Temperaturen zwischen 0 und 100 °C, vorzugsweise jedoch bei Temperaturen zwischen 20 und 50 °C, durchgeführt wird.

**Claims** (for the contracting States : BE, CH, DE, FR, GB, IT, LU, NL, SE)

1. Novel indole derivatives of general formula

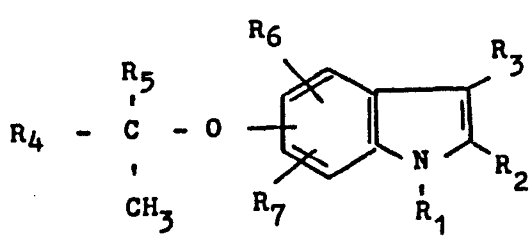

(I)

wherein

$R_1$ represents a hydrogen atom, a straight-chained or branched alkyl group with 1 to 12 carbon atoms or an alkenyl group with 3 to 5 carbon atoms,

$R_2$ represents an alkyl group with 1 to 3 carbon atoms, which may be substituted by a phenyl group, a phenyl group optionally substituted by a halogen atom, a methoxy, amino, nitro or acetamido group, a pyridyl group or $R_1$ together with $R_2$ represents an alkylene group with 3 to 5 carbon atoms,

$R_3$ represents an alkyl group with 1 to 3 carbon atoms, which may be substituted by a phenyl group optionally substituted by a methyl, methoxy or trifluoromethyl group or a halogen atom, or by a pyridyl group, a phenyl group optionally substituted by a methyl, methoxy or trifluoromethyl group or a halogen atom, a pyridyl, pyridyl-N-oxide or quinolyl group or one of the radicals $R_2$ or $R_3$ also represents a hydrogen atom, if the other radical represents an aromatic, araliphatic, heteroaromatic or heteroaraliphatic group mentioned in the definition of the groups $R_2$ or $R_3$;

$R_4$ represents a carboxyl or nitrile group, a trialkoxymethyl group wherein each alkoxy group may contain 1 to 3 carbon atoms, a carbalkoxy group with a total of 2 to 8 carbon atoms, an aminocarbonyl group optionally mono- or disubstituted by alkyl groups with 1 to 7 carbon atoms, cycloalkyl groups with 3 to 7 carbon atoms or alkenyl groups with 3 to 7 carbon atoms, whilst the substituents of the amino group may be identical or different, or a hydroxymethyl, piperidinocarbonyl, morpholinocarbonyl or thiomorpholinocarbonyl group;

$R_5$ represents an alkyl group with 1 to 3 carbon atoms;

$R_6$ represents a hydrogen atom, a methyl or benzyl group and

$R_7$ represents a hydrogen atom or the methyl group, and the physiologically acceptable salts thereof with inorganic or organic acids or also bases, if $R_4$ represents a carboxyl group.

2. Novel indole derivatives of general formula I according to claim 1, wherein

$R_1$ represents a hydrogen atom, an alkyl group with 1 to 12 carbon atoms or the allyl group,

$R_2$ represents a hydrogen atom, an alkyl group with 1 to 3 carbon atoms, or $R_1$ and $R_2$ together represent the propylene, butylene or pentylene group, and

$R_3$ represents a phenyl group optionally substituted by a fluorine, chlorine or bromine atom or by a methyl, methoxy or trifluoromethyl group, a 2-phenylethyl group optionally substituted by a chlorine atom or a methoxy group, or a benzyl, chlorobenzyl, pyridyl-(2), pyridyl-(4), quinolyl-(2) or 4-pyridylmethyl group, or

$R_2$ represents a phenyl group optionally substituted by a chlorine atom or by a methoxy, nitro, amino or acetamino group, or a benzyl or pyridyl-(4) group, and

$R_3$ represents a hydrogen atom or an alkyl group with 1 to 3 carbon atoms,

$R_4$ represents a carboxyl or nitrile group, a carbalkoxy group with a total of 2 to 6 carbon atoms or a hydroxymethyl group,

$R_5$ represents the methyl group,

$R_6$ represents a hydrogen atom or the methyl or benzyl group, and

$R_7$ represents a hydrogen atom or the methyl group, and the physiologically acceptable salts thereof with inorganic or organic acids and also bases, if $R_4$ represents a carboxyl group.

3. Novel indole derivatives of general formula I according to claim 1, wherein

$R_1$ represents an alkyl group with 1 to 4 carbon atoms,

$R_2$ represents a methyl group or $R_1$ and $R_2$ together represent the n-pentylene group, and

$R_3$ represents a phenyl group optionally substituted in the 3- or 4-position by a fluorine or chlorine atom or by a methoxy or trifluoromethyl group, or a 2-phenylethyl, pyridyl-(4) or quinolyl-(2) group, or

$R_2$ represents a phenyl group optionally substituted in the 3- or 4-position by a chlorine atom or by a methoxy, amino or acetamino group, or a pyridyl-(4) group, and

$R_3$ represents a hydrogen atom or a methyl group,

$R_4$ represents a carboxyl group or an alkoxycarbonyl group with a total of 2 to 4 carbon atoms,

$R_5$ represents the methyl group,

$R_6$ and $R_7$ each represent a hydrogen atom, and the physiologically acceptable salts thereof with inorganic or organic acids and also bases, if $R_4$ represents a carboxyl group.

4. Novel indole derivatives of general formula I according to claim 1, wherein

$R_1$ represents an alkyl group with 1 to 4 carbon atoms,

$R_2$ represents a methyl group or $R_1$ and $R_2$ together represent the n-pentylene group,

$R_3$ represents a phenyl group optionally substituted in the 3- or 4-position by a fluorine or chlorine atom or by a methoxy or trifluoromethyl group, or a 2-phenylethyl, pyridyl-(4) or quinolyl-(2) group,

$R_4$ represents a carboxyl group or an alkoxycarbonyl group with a total of 2 to 4 carbon atoms,

$R_5$ represents the methyl group,

$R_6$ and $R_7$ each represent a hydrogen atom, and the physiologically acceptable salts thereof with inorganic or organic acids and also bases, if $R_4$ represents the carboxyl group.

5. 2-[3-(4-Chlorophenyl)-1,2-dimethyl-1H-indol-5-yloxyl]-2-methyl-propanoic acid and the salts thereof with inorganic or organic bases and acids.

6. 2-[1-Butyl-3-(4-fluorophenyl)-2-methyl-1H-indol-5-yloxy]-2-methyl-propanoic acid and the salts thereof with inorganic or organic bases and acids.

7. 2-Methyl-2-[2-methyl-1-propyl-3-(4-pyridyl)-1H-indol-5-yloxy]-propanoic acid and the salts thereof

with inorganic or organic bases and acids.

8. Pharmaceutical compositions, containing a compound according to claims 1 to 7, besides one or more inert carriers and/or diluents.

9. Use of a compound according to claims 1 to 7 for the preparation of a pharmaceutical composition for the treatment of hyperlipaemia and atherosclerosis by a non-chemical method.

10. Process for the preparation of novel indole derivatives of general formula

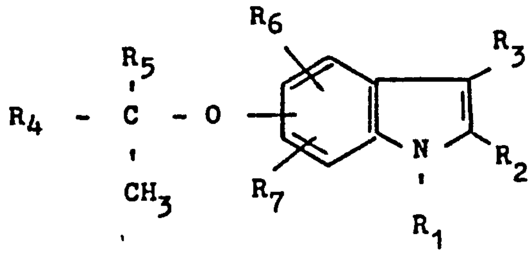

(I)

wherein

$R_1$ represents a hydrogen atom, a straight-chained or branched alkyl group with 1 to 12 carbon atoms or an alkenyl group with 3 to 5 carbon atoms,

$R_2$ represents an alkyl group with 1 to 3 carbon atoms, which can be substituted by a phenyl group ; a phenyl group optionally substituted by a halogen atom, a methoxy, amino, nitro or acetamido group, a pyridyl group or $R_1$ together with $R_2$ represents an alkylene group with 3 to 5 carbon atoms,

$R_3$ represents an alkyl group with 1 to 3 carbon atoms, which may be substituted by a phenyl group optionally substituted by a methyl, methoxy or trifluoromethyl group or a halogen atom, or by a pyridyl group, a phenyl group optionally substituted by a methyl, methoxy or trifluoromethyl group or a halogen atom, a pyridyl, pyridyl-N-oxide or quinolyl group or one of the radicals $R_2$ or $R_3$ also represents a hydrogen atom, if the other radical represents an aromatic, araliphatic, heteroaromatic or heteroaraliphatic group mentioned in the definitions of the groups $R_2$ or $R_3$,

$R_4$ represents a carboxyl or nitrile group, a trialkoxymethyl group wherein each alkoxy group may contain 1 to 3 carbon atoms, a carbalkoxy group with a total of 2 to 8 carbon atoms, an aminocarbonyl group optionally mono- or disubstituted by alkyl groups with 1 to 7 carbon atoms, cycloalkyl groups with 3 to 7 carbon atoms or alkenyl groups with 3 to 7 carbon atoms, whilst the substituents of the amino group may be identical or different, or a hydroxymethyl, piperidinocarbonyl, morpholinocarbonyl or thiomorpholinocarbonyl group,

$R_5$ represents an alkyl group with 1 to 3 carbon atoms,

$R_6$ represents a hydrogen atom, a methyl or benzyl group and

$R_7$ represents a hydrogen atom or the methyl group, and the physiologically acceptable salts thereof with inorganic or organic acids or also bases, if $R_4$ represents a carboxyl group, characterised in that

a) a hydroxyindole of general formula

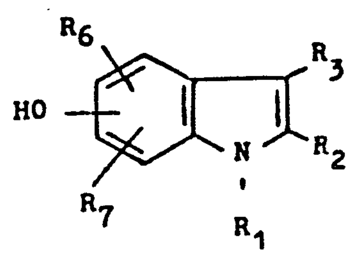

(II)

wherein

$R_1$, $R_2$, $R_3$, $R_6$ and $R_7$ are defined as hereinbefore, or an alkali salt thereof, is reacted with a compound of general formula

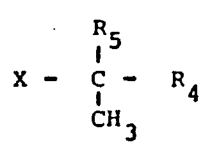

(III)

wherein

$R_4$ and $R_5$ are defined as hereinbefore

X represents a nucleophilically exchangeable group such as a halogen atom, or

b) for the preparation of compounds of general formula I, wherein $R_4$ represents a carboxyl group, a hydroxyindole of general formula

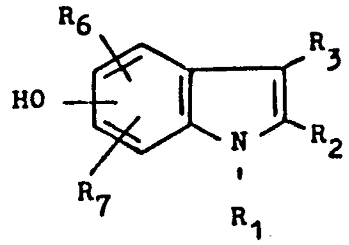

(II)

wherein

$R_1$, $R_2$, $R_3$, $R_6$ and $R_7$ are defined as hereinbefore, or an alkali salt thereof, is reacted with an alcohol of general formula IV,

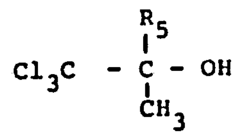

(IV)

optionally prepared in the reaction mixture, wherein

$R_5$ is as hereinbefore defined, in the presence of an inorganic base or

c) for the preparation of compounds of general formula I, wherein $R_1$ does not represent a hydrogen atom, an indole derivative of general formula

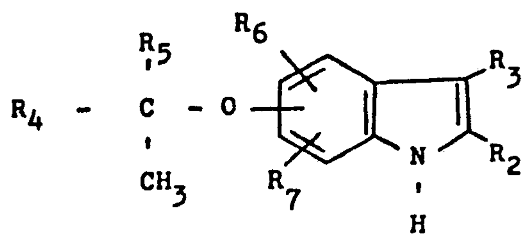

(VI)

wherein

$R_2$ to $R_7$ are defined as hereinbefore, or an alkali salt thereof, is alkylated with a compound of general formula

$$R_1' — Z \qquad (VII)$$

wherein

$R_1'$ has the meanings mentioned above for $R_1$, with the exception of hydrogen, and

Z represents a nucleophilically exchangeable group such as a halogen atom or a sulphonic acid radical, or

d) for the preparation of compounds of general formula I wherein $R_4$ represents the carboxyl group, an indole derivative of general formula

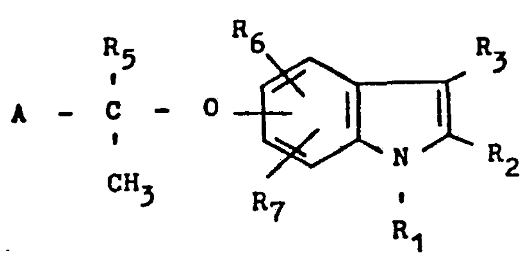

(VIII)

wherein

$R_1$ to $R_3$ and $R_5$ to $R_7$ are as hereinbefore defined and

A represents a group which can be converted by hydrolysis into a carboxyl group, is hydrolysed, or

e) for the preparation of compounds of general formula I wherein $R_4$ represents the carboxyl group, an indole derivative of general formula

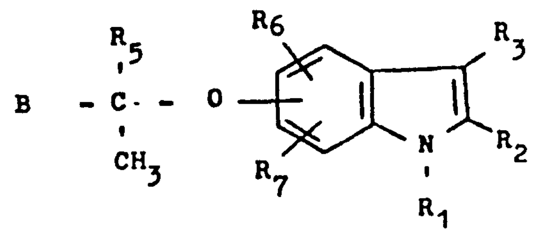

(IX)

wherein

$R_1$ to $R_3$ and $R_5$ to $R_7$ are as hereinbefore defined and

B represents a group which can be converted by oxidation into a carboxyl group, is oxidised, and if desired, a compound of general formula I obtained, wherein $R_4$ represents the carboxyl group, is subsequently converted by esterification or amidation into a corresponding compound of general formula I wherein $R_4$ represents a carbalkoxy group with a total of 2 to 8 carbon atoms, an aminocarbonyl group optionally mono- or disubstituted by alkyl groups with 1 to 7 carbon atoms, cycloalkyl groups with 3 to 7 carbon atoms or alkenyl groups with 3 to 7 carbon atoms, wherein the substituents may be identical or different, or a piperidinocarbonyl, morpholinocarbonyl or thiomorpholinocarbonyl group,

and/or a compound of general formula I obtained wherein $R_4$ represents the carboxyl group or a carbalkoxy group with a total of 2 to 8 carbon atoms, is converted by reduction into a corresponding compound of general formula I wherein $R_4$ represents the hydroxymethyl group,

and/or a compound of general formula I obtained wherein $R_2$ represents a nitrophenyl group, is converted by reduction into a corresponding compound of general formula I wherein $R_2$ represents an aminophenyl group,

and/or a compound of general formula I obtained wherein $R_2$ represents an aminophenyl group, is converted by acetylation into a corresponding compound of general formula I wherein $R_2$ represents an acetamido-phenyl group,

and/or a compound of general formula I obtained is converted into its physiologically acceptable salts with inorganic or organic acids and also bases, if $R_4$ represents a carboxyl group.

**Claims** (for the contracting State AT)

1. Process for the preparation of novel indole derivatives of general formula

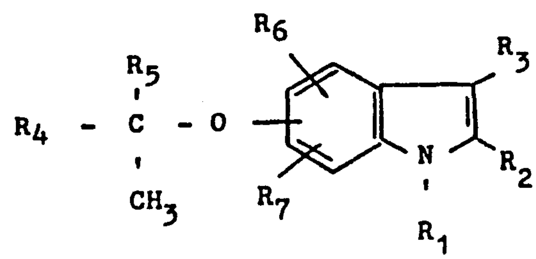

(I)

wherein

$R_1$ represents a hydrogen atom, a straight-chained or branched alkyl group with 1 to 12 carbon atoms or an alkenyl group with 3 to 5 carbon atoms,

$R_2$ represents an alkyl group with 1 to 3 carbon atoms, which can be substituted by a phenyl group ; a phenyl group optionally substituted by a halogen atom or by a methoxy, amino, nitro or acetamido group, a pyridyl group or $R_1$ together with $R_2$ represents an alkylene group with 3 to 5 carbon atoms,

$R_3$ represents an alkyl group with 1 to 3 carbon atoms, which may be substituted by a phenyl group optionally substituted by a methyl, methoxy or trifluoromethyl group or a halogen atom, or by a pyridyl group, a phenyl group optionally substituted by a methyl, methoxy or trifluoromethyl group or a halogen atom, a pyridyl, pyridyl-N-oxide or quinolyl group or one of the radicals $R_2$ or $R_3$ also represents a hydrogen atom, if the other radical $R_2$ or $R_3$ represents an aromatic, araliphatic, heteroaromatic or heteroaraliphatic group mentioned in the definition of the groups $R_2$ or $R_3$,

$R_4$ represents a carboxyl or nitrile group, a trialkoxymethyl group wherein each alkoxy group may contain 1 to 3 carbon atoms, a carbalkoxy group with a total of 2 to 8 carbon atoms, an aminocarbonyl group optionally mono- or disubstituted by alkyl groups with 1 to 7 carbon atoms, cycloalkyl groups with 3 to 7 carbon atoms or alkenyl groups with 3 to 7 carbon atoms, whilst the substituents of the amino group may be identical or different, or a hydroxymethyl, piperidinocarbonyl, morpholinocarbonyl or

thiomorpholinocarbonyl groups,

$R_5$ represents an alkyl group with 1 to 3 carbon atoms,

$R_6$ represents a hydrogen atom, a methyl or benzyl group and

$R_7$ represents a hydrogen atom or the methyl group, and the physiologically acceptable salts thereof with inorganic or organic acids or also bases, if $R_4$ represents a carboxyl group, characterised in that

a) a hydroxyindole of general formula

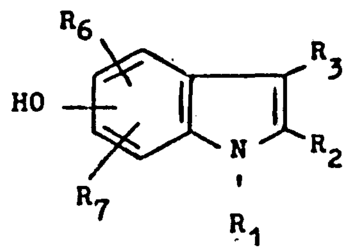

(II)

wherein

$R_1$, $R_2$, $R_3$, $R_6$ and $R_7$ are defined as hereinbefore, or an alkali salt thereof, is reacted with a compound of general formula

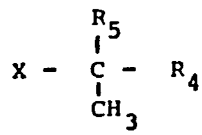

(III)

wherein

$R_4$ and $R_5$ are defined as hereinbefore and

X represents a nucleophilically exchangeable group such as a halogen atom, or

b) for the preparation of compounds of general formula I, wherein $R_4$ represents a carboxyl group, a hydroxyindole of general formula

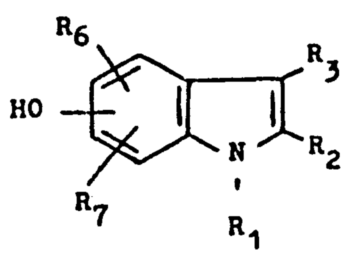

(II)

wherein

$R_1$, $R_2$, $R_3$, $R_6$ and $R_7$ are defined as hereinbefore, or an alkali salt thereof, is reacted with an alcohol of general formula IV,

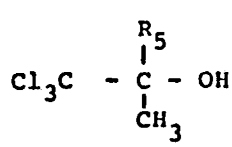

(IV)

optionally prepared in the reaction mixture, wherein

$R_5$ is as hereinbefore defined, in the presence of an inorganic base, or

c) for the preparation of compounds of general formula I, wherein $R_1$ does not represent a hydrogen atom, an indole derivative of general formula

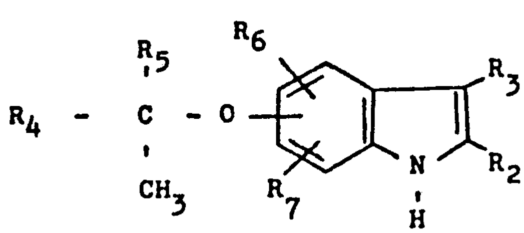

(VI)

wherein

$R_2$ to $R_7$ are defined as hereinbefore, or an alkali salt thereof, is alkylated with a compound of general formula

$$R_1' - Z \qquad (VII)$$

wherein

$R_1'$ has the meanings mentioned above for $R_1$ with the exception of hydrogen, and

Z represents a nucleophilically exchangeable group such as a halogen atom or a sulphonic acid radical, or

d) for the preparation of compounds of general formula I wherein $R_4$ represents the carboxyl group, an indole derivative of general formula

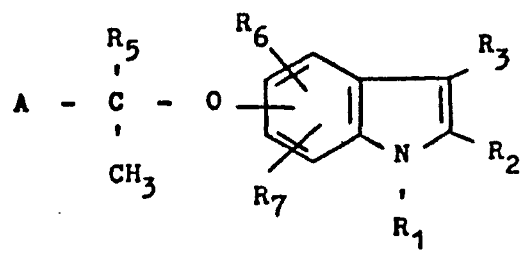

(VIII)

wherein

$R_1$ to $R_3$ and $R_5$ to $R_7$ are as hereinbefore defined and

A represents a group which can be converted by hydrolysis into a carboxyl group, is hydrolysed or

e) for the preparation of compounds of general formula I wherein $R_4$ represents the carboxyl group, an indole derivative of general formula

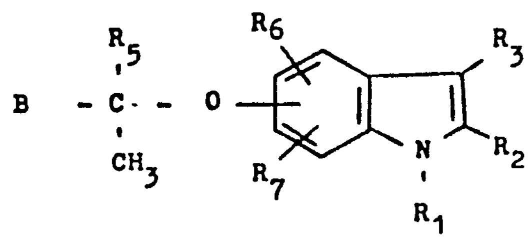

(IX)

wherein

$R_1$ to $R_3$ and $R_5$ to $R_7$ are as hereinbefore defined and

B represents a group which can be converted by oxidation into a carboxyl group, is oxidised, and if desired, a compound of general formula I obtained wherein $R_4$ represents the carboxyl group, is subsequently converted by esterification or amidation into a corresponding compound of general formula I wherein $R_4$ represents a carbalkoxy group with a total of 2 to 8 carbon atoms, an aminocarbonyl group optionally mono- or disubstituted by alkyl groups with 1 to 7 carbon atoms, cycloalkyl groups with 3 to 7 carbon atoms or alkenyl groups with 3 to 7 carbon atoms, wherein the substituents may be identical or different, or a piperidinocarbonyl, morpholinocarbonyl or thiomorpholinocarbonyl group,

and/or a compound of general formula I obtained, wherein $R_4$ represents the carboxyl group or a carbalkoxy group with a total of 2 to 8 carbon atoms, is converted by reduction into a corresponding compound of general formula I wherein $R_4$ represents the hydroxymethyl group,

and/or a compound of general formula I obtained, wherein $R_2$ represents a nitrophenyl group, is converted by reduction into a corresponding compound of general formula I wherein $R_2$ represents an aminophenyl group,

and/or a compound of general formula I obtained, wherein $R_2$ represents an aminophenyl group, is converted by acetylation into a corresponding compound of general formula I wherein $R_2$ represents an acetamido group,

and/or a compound of general formula I obtained is converted into its physiologically acceptable salts with inorganic or organic acids and also bases, if $R_4$ represents a carboxyl group.

2. Process according to claim 1 for the preparation of new indole derivatives of general formula

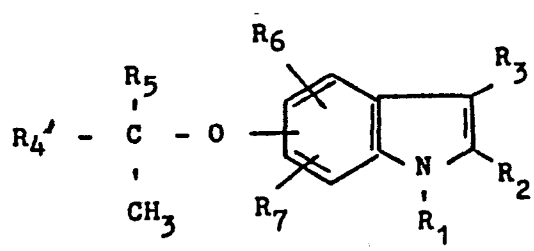

(Ia)

wherein

$R_1$, $R_2$, $R_3$, $R_5$, $R_6$ and $R_7$ are defined as in claim 1 and

$R_4'$ represents a carboxyl group or a carbalkoxy group with a total of 2 to 8 carbon atoms, and the physiologically acceptable salts thereof with inorganic and organic acids or, if $R_4'$ represents a carboxyl group, with inorganic and organic bases, characterised in that

a) a hydroxyindole of general formula

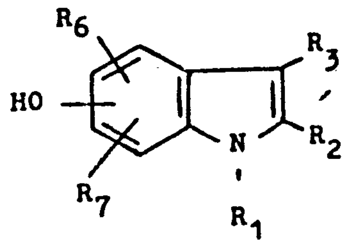

(II)

wherein

$R_1$ to $R_3$, $R_6$ and $R_7$ are as hereinbefore defined, or an alkali salt thereof, is reacted with a compound of general formula

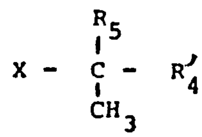

(III)

wherein

$R_4'$ and $R_5$ are as hereinbefore defined and X represents a nucleophilically exchangeable group such as a halogen atom, or

b) for the preparation of compounds of general formula I wherein $R_4'$ represents a carboxyl group, a hydroxyindole of general formula

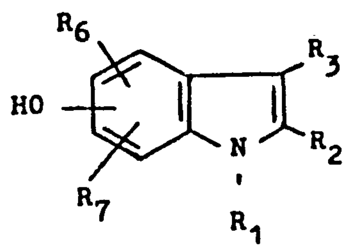

(II)

wherein

$R_1$ to $R_3$, $R_6$ and $R_7$ are as hereinbefore defined, or an alkali salt thereof, is reacted with an alcohol of general formula

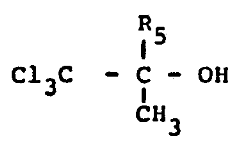

(IV)

optionally prepared in the reaction mixture, wherein

$R_5$ is as hereinbefore defined, in the presence of an inorganic base, or

c) for the preparation of compounds of general formula I wherein $R_1$ does not represent a hydrogen atom, an indole derivative of general formula

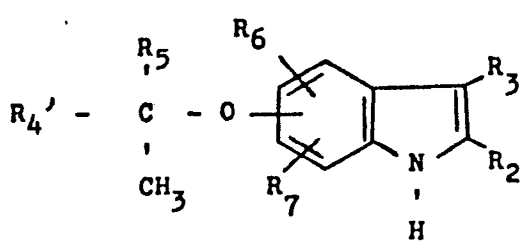

(VIa)

wherein

$R_2$, $R_3$, $R_4'$ and $R_5$ to $R_7$ are as hereinbefore defined, or an alkali salt thereof, is alkylated with a compound of general formula

$$R_1' - Z \quad (VII)$$

wherein

$R_1'$ has the meanings given above for $R_1$, with the exception of hydrogen, and

Z represents a nucleophilically exchangeable group such as a halogen atom or a sulphonic acid group,

and, if desired, a compound of general formula I obtained wherein $R_4'$ represents a carbalkoxy group with a total of 2 to 8 carbon atoms is subsequently converted by hydrolysis into a corresponding compound of general formula I wherein $R_4'$ represents a carboxyl group,

and/or a compound of general formula I obtained wherein $R_4'$ represents a carboxyl group is converted by esterification into a corresponding compound of general formula I wherein $R_4'$ represents a carbalkoxy group with a total of 2 to 8 carbon atoms,

and/or a compound of general formula I obtained wherein $R_2$ represents a nitrophenyl group is converted by reduction into a corresponding compound of general formula I wherein $R_2$ represents an aminophenyl group,

and/or a compound of general formula I obtained wherein $R_2$ represents an aminophenyl group is converted by acetylation into a corresponding compound of general formula I wherein $R_2$ represents an acetamidophenyl group,

and/or compound of general formula I obtained is converted into the physiologically acceptable salts thereof with inorganic and organic acids or, if $R_4'$ represents a carboxyl group, with inorganic and organic bases.

Priority : 13.03.1979 (P29 09 779.8).

3. Process according to claims 1 and 2, characterised in that the reaction (a) is effected at temperatures of between 0 and 200 °C, but preferably at temperatures between ambient temperature and the boiling temperature of the solvent used, e.g. at temperatures of between 20 and 100 °C.

4. Process according to claims 1 and 2, characterised in that the reaction (b) is effected in an anhydrous ketone of general formula

$$R_5 - CO - CH_3 \quad (V)$$

wherein

$R_5$ is as hereinbefore defined, as the solvent.

5. Process according to claims 1, 2 and 4, characterised in that the reaction (b) is effected at temperatures of between 0 °C and the boiling temperature of the solvent used, e.g. at temperatures of between 0 and 100 °C.

6. Process according to claims 1, 2, 4 and 5, characterised in that a compound of general formula IV is prepared in the reaction mixture by reacting a corresponding ketone of general formula V with chloroform in the presence of an inorganic base.

7. Process according to claims 1 and 2, characterised in that the alkylation (c) is effected at temperatures of between 0 and 200 °C, but preferably at temperatures of between ambient temperature and the boiling temperature of the solvent used, e.g. at temperatures of between 20 and 100 °C.

8. Process according to claim 1, characterised in that the reaction (d) is effected at the boiling temperature of the reaction mixture.

9. Process according to claim 1, characterised in that the reaction (e) is effected at temperatures of between 0 and 100 °C, but preferably at temperatures of between 20 and 50 °C.

**Revendications** (pour les Etats contractant : BE, CH, DE, FR, GB, IT, LU, NL, SE)

1. Nouveaux dérivés de l'indole de formule générale

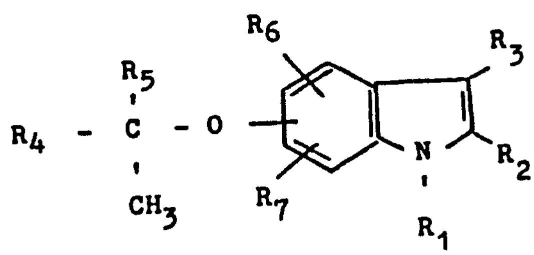

(I)

dans laquelle

$R_1$ représente un atome d'hydrogène, un groupe alcoyle rectiligne ou ramifié avec 1 à 12 atomes de

carbone ou un groupe alcényle avec 3 à 5 atomes de carbone,

$R_2$ représente un groupe alcoyle avec 1 à 3 atomes de carbone qui peut être substitué par un groupe phényle, un groupe phényle éventuellement substitué par un atome d'halogène, un groupe méthoxy, amino, nitro ou acétamido, un groupe pyridyle ou $R_1$ représente ensemble avec $R_2$ un groupe alcoylène avec 3 à 5 atomes de carbone,

$R_3$ représente un groupe alcoyle avec 1 à 3 atomes de carbone qui peut être substitué par un groupe phényle éventuellement substitué par un groupe méthyle, groupe méthoxy, groupe trifluorométhyle ou un atome d'halogène, ou par un groupe pyridyle, un groupe phényle éventuellement substitué par un groupe méthyle, groupe méthoxy, groupe trifluorométhyle ou un atome d'halogène, un groupe pyridyle, pyridyl-N-oxyde ou quinoléyle ou l'un des radicaux $R_2$ ou $R_3$ représente également un atome d'hydrogène lorsque l'autre des radicaux $R_2$ ou $R_3$ représente dans chaque cas l'un des radicaux aromatiques, araliphatiques, hétéroaromatiques ou hétéroaliphatiques mentionnés à propos de la définition des radicaux $R_2$ ou $R_3$,

$R_4$ représente un groupe carboxyle ou nitrile, un groupe trialcoxyméthyle dans lequel chaque radical alcoxy peut contenir 1 à 3 atomes de carbone, un groupe carbalcoxy avec en tout 2 à 8 atomes de carbone, un groupe aminocarbonyle éventuellement mono- ou disubstitué par des groupes alcoyle avec 1 à 7 atomes de carbone, des groupes cycloalcoyle avec 3 à 7 atomes de carbone, ou des groupes alcényle avec 3 à 7 atomes de carbone, les substituants du groupe amino pouvant être identiques ou différents, un groupe hydroxyméthyle, pipéridinocarbonyle, morpholinocarbonyle ou thiomorpholinocarbonyle,

$R_5$ représente un groupe alcoyle avec 1 à 3 atomes de carbone,

$R_6$ représente un atome d'hydrogène, un groupe méthyle ou benzyle et

$R_7$ représente un atome d'hydrogène ou le groupe méthyle, et leurs sels physiologiquement supportables avec des acides et également des bases, lorsque $R_4$ représente le groupe carboxyle, minéraux ou organiques.

2. Nouveaux dérivés de l'indole de formule générale I selon la revendication 1, dans laquelle

$R_1$ représente un atome d'hydrogène, un groupe alcoyle avec 1 à 12 atomes de carbone ou le groupe allyle,

$R_2$ représente un atome d'hydrogène, un groupe alcoyle avec 1 à 3 atomes de carbone ou $R_1$ et $R_2$ représentent ensemble le groupe propylène, butylène ou pentylène et

$R_3$ représente un groupe phényle éventuellement substitué par un atome de fluor, de chlore ou de brome, ou par un groupe méthyle, méthoxy ou trifluorométhyle, un groupe 2-phényléthyle éventuellement substitué par un atome de chlore ou un groupe méthoxy, un groupe benzyle, chlorobenzyle, pyridyle-(2), pyridyle-(4), quinoléyle-(2) ou 4-pyridylméthyle ou

$R_2$ représente un groupe phényle éventuellement substitué par un atome de chlore, par un groupe méthoxy, nitro amino ou acétamino, un groupe benzyle ou pyridyle-(4) et

$R_3$ représente un atome d'hydrogène ou un groupe alcoyle avec 1 à 3 atomes de carbone,

$R_4$ représente un groupe carboxyle ou nitrile, un groupe carbalcoxy avec en tout 2 à 6 atomes de carbone ou un groupe hydroxyméthyle,

$R_5$ représente le groupe méthyle,

$R_6$ représente un atome d'hydrogène, le groupe méthyle ou benzyle et

$R_7$ représente un atome d'hydrogène ou le groupe méthyle, et leurs sels physiologiquement supportables avec des acides et également des bases, lorsque $R_4$ représente le groupe carboxyle, minéraux ou organiques.

3. Nouveaux dérivés de l'indole de formule générale I selon la revendication 1, dans laquelle

$R_1$ représente un groupe alcoyle avec 1 à 4 atomes de carbone,

$R_2$ représente un groupe méthyle ou $R_1$ et $R_2$ représentent ensemble le groupe n-pentylène et

$R_3$ représente un groupe phényle éventuellement substitué en position 3 ou 4 par un atome de fluor ou de chlore, ou par un groupe méthoxy ou trifluorométhyle, un groupe 2-phényléthyle, pyridyle-(4) ou quinoléyle-(2) ou

$R_2$ représente un groupe phényle éventuellement substitué en position 3 ou 4 par un atome de chlore, par un groupe méthoxy, amino ou acétamino, ou un groupe pyridyle-(4) et

$R_3$ représente un atome d'hydrogène ou un groupe méthyle,

$R_4$ représente un groupe carboxyle ou un groupe alcoxycarbonyle avec en tout 2 à 4 atomes de carbone,

$R_5$ représente le groupe méthyle,

$R_6$ et $R_7$ représentent chacun un atome d'hydrogène, et leurs sels physiologiquement supportables avec des acides et également des bases, lorsque $R_4$ représente le groupe carboxyle, minéraux ou organiques.

4. Nouveaux dérivés de l'indole de formule générale I selon la revendication 1, dans laquelle

$R_1$ représente un groupe alcoyle avec 1 à 4 atomes de carbone,

$R_2$ représente un groupe méthyle ou $R_1$ et $R_2$ représentent ensemble le groupe n-pentylène,

$R_3$ représente un groupe phényle éventuellement substitué en position 3 ou 4 par un atome de fluor ou de chlore ou par un groupe méthoxy ou trifluorométhyle, un groupe 2-phényléthyle, pyridyle-(4) ou quinoléyle-(2),

$R_4$ représente un groupe carboxyle ou un groupe alcoxycarbonyle avec en tout 2 à 4 atomes de carbone,

$R_5$ représente le groupe méthyle,

$R_6$ et $R_7$ représentent chacun un atome d'hydrogène et leurs sels physiologiquement supportables avec des acides et également des bases, lorsque $R_4$ représente le groupe carboxyle, minéraux ou organiques.

5. L'acide 2-[3-(4-chlorophényl)-1,2-diméthyl-1H-indol-5-yloxy]-2-méthyl-propanoïque et ses sels avec des bases et acides minéraux ou organiques.

6. L'acide 2-[1-butyl-3-(4-fluorophényl)-2-méthyl-1H-indol-5-yloxy]-2-méthyl-propanoïque et ses sels avec des bases et acides minéraux ou organiques.

7. L'acide 2-méthyl-2-[2-méthyl-1-propyl-3-(4-pyridyl)-1H-indol-5-yloxy]propanoïque et ses sels avec des bases et acides minéraux ou organiques.

8. Médicament contenant un composé selon l'une des revendications 1 à 7 conjointement à 1 ou plusieurs excipients et/ou diluants inertes.

9. Utilisation d'un composé selon les revendications 1 à 7 pour la préparation d'un médicament pour combattre l'hyperlipidémie et l'artériosclérose par voie non chimique.

10. Procédé pour la préparation de nouveaux dérivés de l'indole de formule générale

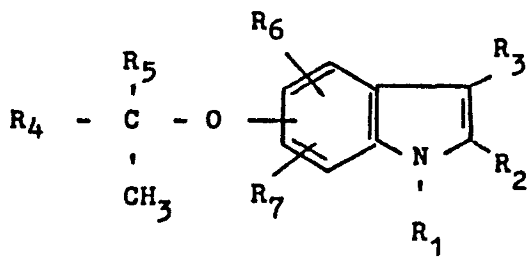

(I)

dans laquelle

$R_1$ représente un atome d'hydrogène, un groupe alcoyle rectiligne ou ramifié avec 1 à 12 atomes de carbone ou un groupe alcényle avec 3 à 5 atomes de carbone,

$R_2$ représente un groupe alcoyle avec 1 à 3 atomes de carbone qui peut être substitué par un groupe phényle, un groupe phényle éventuellement substitué par un atome d'halogène, un groupe méthoxy, amino, nitro ou acétamido, un groupe pyridyle ou $R_1$ représente ensemble avec $R_2$ un groupe alcoylène avec 3 à 5 atomes de carbone,

$R_3$ représente un groupe alcoyle avec 1 à 3 atomes de carbone qui peut être substitué par un groupe phényle éventuellement substitué par un groupe méthyle, groupe méthoxy, groupe trifluorométhyle ou un atome d'halogène ou par un groupe pyridyle, un groupe phényle éventuellement substitué par un groupe méthyle, groupe méthoxy, groupe trifluorométhyle ou un atome d'halogène, un groupe pyridyle, pyridyl-N-oxyde ou quinoléyle ou l'un des radicaux $R_2$ ou $R_3$ représente également un atome d'hydrogène lorsque l'autre des radicaux $R_2$ ou $R_3$ représente dans chaque cas l'un des radicaux aromatiques, araliphatiques, hétéroaromatiques ou hétéroaraliphatiques mentionnés à propos de la définition des radicaux $R_2$ ou $R_3$,

$R_4$ représente un groupe carboxyle ou nitrile, un groupe trialcoxyméthyle dans lequel chaque radical alcoxy peut contenir 1 à 3 atomes de carbone, un groupe carbalcoxy avec en tout 2 à 8 atomes de carbone, un groupe aminocarbonyle éventuellement mono- ou disubstitué par des groupes alcoyle avec 1 à 7 atomes de carbone, des groupes cycloalcoyle avec 3 à 7 atomes de carbone, ou des groupes alcényle avec 3 à 7 atomes de carbone, les substituants du groupe amino pouvant être identiques ou différents, un groupe hydroxyméthyle, pipéridinocarbonyle, morpholinocarbonyle ou thiomorpholinocarbonyle,

$R_5$ représente un groupe alcoyle avec 1 à 3 atomes de carbone,

$R_6$ représente un atome d'hydrogène, un groupe méthyle ou benzyle et

$R_7$ représente un atome d'hydrogène ou le groupe méthyle, ainsi que leurs sels physiologiquement supportables avec des acides et également des bases, lorsque $R_4$ représente le groupe carboxyle, minéraux ou organiques, caractérisé en ce que

a) on fait réagir un hydroxyindole de formule générale

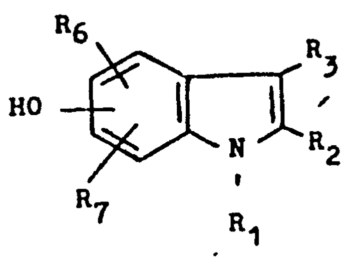

(II)

dans laquelle

$R_1$, $R_2$, $R_3$, $R_6$ et $R_7$ sont définis comme au début, ou respectivement ses sels alcalins avec un

composé de formule générale

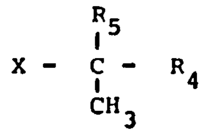

(III)

dans laquelle

$R_4$ et $R_5$ sont définis comme au début et

X représente un groupe partant nucléophile tel qu'un atome d'halogène ou

b) pour préparer des composés de formule générale I dans laquelle $R_4$ représente le groupe carboxyle, on fait réagir un hydroxyindole de formule générale

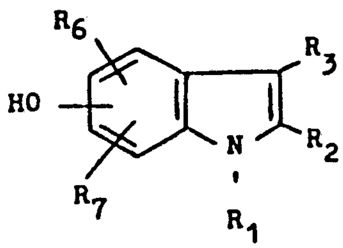

(II)

dans laquelle

$R_1$, $R_2$, $R_3$, $R_6$ et $R_7$ sont définis comme au début ou respectivement ses sels alcalins, avec un alcool éventuellement préparé dans le milieu réactionnel, de formule générale

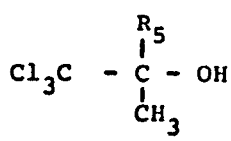

(IV)

dans laquelle

$R_5$ est défini comme au début, en présence d'une base minérale ou

c) pour préparer des composés de formule générale I dans laquelle $R_1$ ne représente pas un atome d'hydrogène, on alcoyle un dérivé d'indole de formule générale

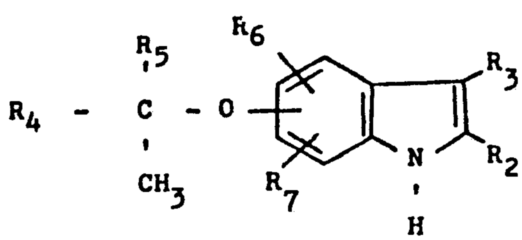

(VI)

dans laquelle

$R_2$ à $R_7$ sont définis comme au début, ou respectivement ses sels alcalins, avec un composé de formule générale

$R_1' — Z$ (VII)

dans laquelle

$R_1'$ possède les significations mentionnées au début pour $R_1$ à l'exception de l'atome d'hydrogène et

Z représente un groupe partant nucléophile tel qu'un atome d'hydrogène ou un reste d'acide sulfonique ou

d) pour préparer des composés de formule générale I dans laquelle $R_4$ représente le groupe carboxyle, on hydrolyse un dérivé d'indole de formule générale

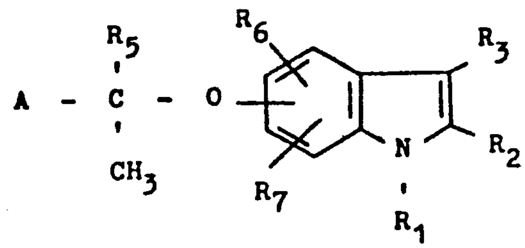

(VIII)

dans laquelle

$R_1$ à $R_3$ et $R_5$ à $R_7$ sont définis comme au début et

A représente un groupe transformable en un groupe carboxyle par hydrolyse ou

e) pour préparer des composés de formule générale I dans laquelle $R_4$ représente le groupe carboxyle, on oxyde un dérivé d'indole de formule générale

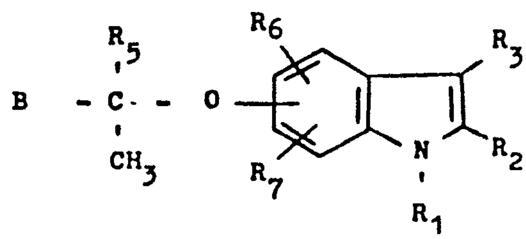

(IX)

dans laquelle

$R_1$ à $R_3$ et $R_5$ à $R_7$ sont définis comme au début et

B représente un groupe transformable en un groupe carboxyle par oxydation,

et si on le désire on transforme ensuite un composé obtenu de formule générale I dans laquelle $R_4$ représente le groupe carboxyle, par estérification ou amidation, en un composé correspondant de formule générale I dans laquelle $R_4$ représente un groupe carbalcoxy avec en tout 2 à 8 atomes de carbone, un groupe aminocarbonyle éventuellement mono- ou disubstitué par des groupes alcoyle avec 1 à 7 atomes de carbone, des groupes cycloalcoyle avec 3 à 7 atomes de carbone ou des groupes alcényle avec 3 à 7 atomes de carbone, les substituants pouvant être identiques ou différents, un groupe pipéridinocarbonyle, morpholinocarbonyle ou thiomorpholinocarbonyle

et/ou on transforme un composé obtenu de formule générale I dans laquelle $R_4$ représente le groupe carboxyle ou un groupe carbalcoxy avec en tout 2 à 8 atomes de carbone, par réduction, en un composé correspondant de formule générale I dans laquelle $R_4$ représente le groupe hydroxyméthyle

et/ou on transforme un composé obtenu de formule générale I dans laquelle $R_2$ représente un groupe nitrophényle, par réduction, en un composé correspondant de formule générale I dans laquelle $R_2$ représente un groupe aminophényle

et/ou on transforme un composé obtenu de formule générale I dans laquelle $R_2$ représente un groupe aminophényle, par acétylation, en un composé correspondant de formule générale I dans laquelle $R_2$ représente un groupe acétamidophényle

et/ou on transforme un composé obtenu de formule générale I en ses sels physiologiquement supportables avec des acides et également des bases, dans le cas où $R_4$ représente le groupe carboxyle, minéraux ou organiques.

**Revendications** (pour l'Etat contractant AT)

1. Procédé pour la préparation de nouveaux dérivés de l'indole de formule générale

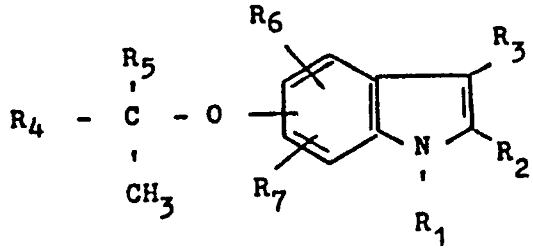

(I)

dans laquelle

$R_1$ représente un atome d'hydrogène, un groupe alcoyle rectiligne ou ramifié avec 1 à 12 atomes de carbone ou un groupe alcényle avec 3 à 5 atomes de carbone,

$R_2$ représente un groupe alcoyle avec 1 à 3 atomes de carbone qui peut être substitué par un groupe phényle, un groupe phényle éventuellement substitué par un atome d'halogène, un groupe méthoxy, amino-, nitro ou acétamido, un groupe pyridyle ou $R_1$ représente ensemble avec $R_2$ un groupe alcoylène avec 3 à 5 atomes de carbone,

$R_3$ représente un groupe alcoyle avec 1 à 3 atomes de carbone qui peut être substitué par un groupe phényle éventuellement substitué par un groupe méthyle, groupe méthoxy, groupe trifluorométhyle ou un atome d'halogène ou par un groupe pyridyle, un groupe phényle éventuellement substitué par un groupe méthyle, groupe méthoxy, groupe trifluorométhyle ou un atome d'halogène, un groupe pyridyle, pyridyl-N-oxyde ou quinoléyle ou l'un des radicaux $R_2$ ou $R_3$ représente également un atome d'hydrogène lorsque l'autre des radicaux $R_2$ ou $R_3$ représente dans chaque cas l'un des radicaux aromatiques, araliphatiques, hétéroaromatiques ou hétéroaraliphatiques mentionnés à propos de la définition des radicaux $R_2$ ou $R_3$,

$R_4$ représente un groupe carboxyle ou nitrile, un groupe trialcoxyméthyle dans lequel chaque radical alcoxy peut contenir 1 à 3 atomes de carbone, un groupe carbalcoxy avec en tout 2 à 8 atomes de carbone, un groupe aminocarbonyle éventuellement mono- ou disubstitué par des groupes alcoyle avec 1 à 7 atomes de carbone, des groupes cycloalcoyle avec 3 à 7 atomes de carbone, ou des groupes alcényle avec 3 à 7 atomes de carbone, les substituants du groupe amino pouvant être identiques ou différents, un groupe hydroxyméthyle, pipéridinocarbonyle, morpholinocarbonyle ou thiomorpholinocarbonyle,

$R_5$ représente un groupe alcoyle avec 1 à 3 atomes de carbone,

$R_6$ représente un atome d'hydrogène, un groupe méthyle ou benzyle et

$R_7$ représente un atome d'hydrogène ou le groupe méthyle, ainsi que leurs sels physiologiquement supportables avec des acides et également des bases, lorsque $R_4$ représente le groupe carboxyle, minéraux ou organiques, caractérisé en ce que

a) on fait réagir un hydroxyindole de formule générale

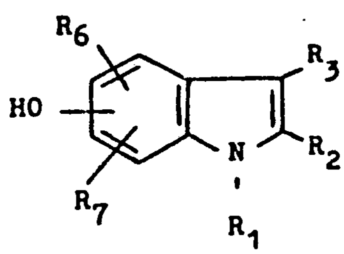

(II)

dans laquelle

$R_1$, $R_2$, $R_3$, $R_6$ et $R_7$ sont définis comme au début, ou respectivement ses sels alcalins avec un composé de formule générale

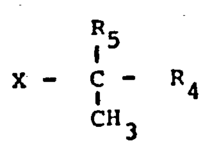

(III)

dans laquelle

$R_4$ et $R_5$ sont définis comme au début et

X représente un groupe partant nucléophile tel qu'un atome d'halogène ou

b) pour préparer des composés de formule générale I dans laquelle $R_4$ représente le groupe carboxyle, on fait réagir un hydroxyindole de formule générale

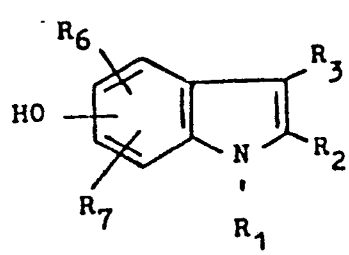

(II)

dans laquelle

$R_1$, $R_2$, $R_3$, $R_6$ et $R_7$ sont définis comme au début ou respectivement ses sels alcalins, avec un alcool éventuellement préparé dans le milieu réactionnel, de formule générale

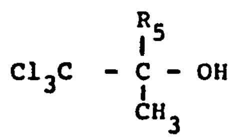

(IV)

dans laquelle

$R_5$ est défini comme au début, en présence d'une base minérale ou

c) pour préparer des composés de formule générale I dans laquelle $R_1$ ne représente pas un atome d'hydrogène, on alcoyle un dérivé d'indole de formule générale

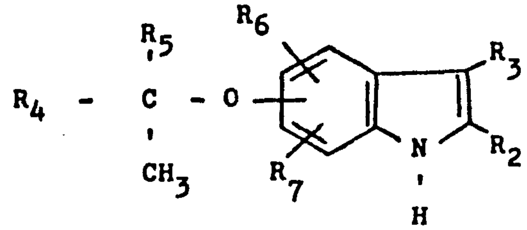

(VI)

dans laquelle

$R_2$ et $R_7$ sont définis comme au début, ou respectivement ses sels alcalins, avec un composé de formule générale

$$R_1' — Z \qquad (VII)$$

dans laquelle

$R_1'$ possède les significations mentionnées au début pour $R_1$ à l'exception de l'atome d'hydrogène et

Z représente un groupe partant nucléophile tel qu'un atome d'hydrogène ou un reste d'acide sulfonique ou

d) pour préparer des composés de formule générale I dans laquelle $R_4$ représente le groupe carboxyle, on hydrolyse un dérivé d'indole de formule générale

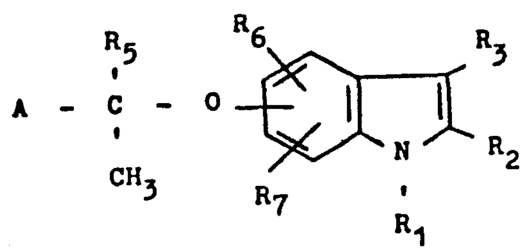

(VIII)

dans laquelle

$R_1$ à $R_3$ et $R_5$ à $R_7$ sont définis comme au début et

A représente un groupe transformable en un groupe carboxyle par hydrolyse ou

e) pour préparer des composés de formule générale I dans laquelle $R_4$ représente le groupe carboxyle, on oxyde un dérivé d'indole de formule générale

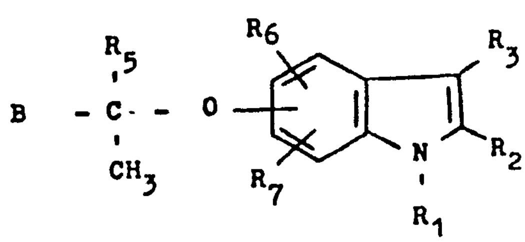

(IX)

dans laquelle

$R_1$ à $R_3$ et $R_5$ à $R_7$ sont définis comme au début et

B représente un groupe transformable en un groupe carboxyle par oxydation,

et si on le désire on transforme ensuite un composé obtenu de formule générale I dans laquelle $R_4$ représente le groupe carboxyle, par estérification ou amidation, en un composé correspondant de formule générale I dans laquelle $R_4$ représente un groupe carbalcoxy avec en tout 2 à 8 atomes de carbone, un groupe aminocarbonyle éventuellement mono- ou disubstitué par des groupes alcoyle avec 1 à 7 atomes de carbone, des groupes cycloalcoyle avec 3 à 7 atomes de carbone ou des groupes alcényle avec 3 à 7 atomes de carbone, les substituants pouvant être identiques ou différents, un groupe pipéridinocarbonyle, morpholinocarbonyle ou thiomorpholinocarbonyle

et/ou on transforme un composé obtenu de formule générale I dans laquelle $R_4$ représente le groupe carboxyle ou un groupe carbalcoxy avec en tout 2 à 8 atomes de carbone, par réduction, en un composé correspondant de formule générale I dans laquelle $R_4$ représente le groupe hydroxyméthyle

et/ou on transforme un composé obtenu de formule générale I dans laquelle $R_2$ représente un groupe nitrophényle, par réduction, en un composé correspondant de formule générale I dans laquelle $R_2$ représente un groupe aminophényle

et/ou on transforme un composé obtenu de formule générale I dans laquelle $R_2$ représente un groupe aminophényle, par acétylation, en un composé correspondant de formule générale I dans laquelle $R_2$ représente un groupe acétamidophényle

et/ou on transforme un composé obtenu de formule générale I en ses sels physiologiquement supportables avec des acides et également des bases, dans le cas où $R_4$ représente le groupe carboxyle, minéraux ou organiques.

2. Procédé selon la revendication 1 pour préparer de nouveaux dérivés de l'indole de formule générale

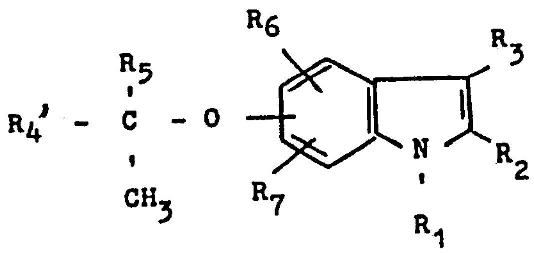

(Ia)

dans laquelle

$R_1$, $R_2$, $R_3$, $R_5$, $R_6$ et $R_7$ sont définis comme dans la revendication 1 et

$R_4'$ représente un groupe carboxyle ou un groupe carbalcoxy avec en tout 2 à 8 atomes de carbone, ainsi que leurs sels physiologiquement supportables avec des acides minéraux et organiques ou également, lorsque $R_4'$ représente un groupe carboxyle, avec des bases minérales et organiques, caractérisé en ce que

a) on fait réagir un hydroxyindole de formule générale

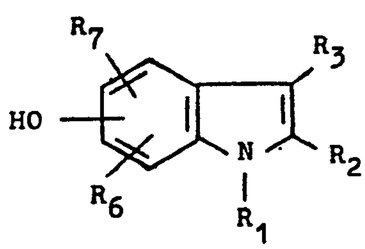

(II)

dans laquelle

$R_1$ à $R_3$, $R_6$ et $R_7$ sont définis comme au début, ou respectivement ses sels alcalins, avec un composé de formule générale

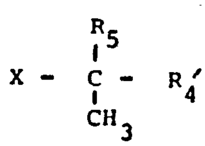

(III)

dans laquelle

$R_4'$ et $R_5$ sont définis comme au début et

X représente un groupe partant nucléophile tel qu'un atome d'halogène ou

b) pour préparer des composés de formule générale I dans laquelle $R_4'$ représente le groupe carboxyle, on fait réagir un hydroxyindole de formule générale

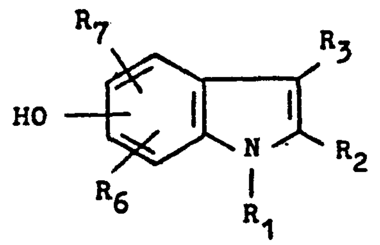

(II)

dans laquelle

$R_1$ à $R_3$, $R_6$ et $R_7$ sont définis comme au début, ou respectivement ses sels alcalins avec un alcool éventuellement préparé dans le milieu réactionnel, de formule générale

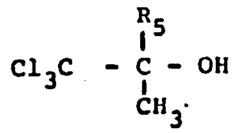

(IV)

dans laquelle

$R_5$ est défini comme au début, en présence d'une base minérale ou

c) pour préparer des composés de formule générale I dans laquelle $R_1$ ne représente pas un atome d'hydrogène, on alcoyle un dérivé d'indole de formule générale

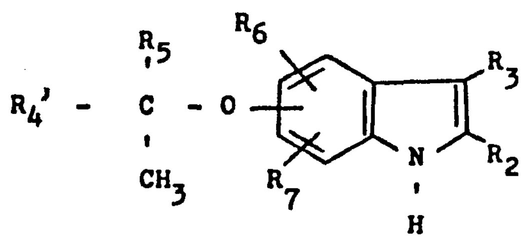

(VIa)

dans laquelle

$R_2$, $R_3$, $R_4'$ et $R_5$ à $R_7$ sont définis comme au début, ou respectivement ses sels alcalins, au moyen d'un composé de formule générale

$$R_1' - Z \qquad \text{(VII)}$$

dans laquelle

$R_1'$ possède les significations mentionnées au début pour $R_1$, à l'exception de l'hydrogène et

Z représente un groupe partant nucléophile tel qu'un atome d'halogène ou un reste d'acide sulfonique, et si on le désire on transforme ensuite un composé obtenu de formule générale I dans laquelle $R_4'$ représente un groupe carbalcoxy avec en tout 2 à 8 atomes de carbone, au moyen d'une hydrolyse, en un composé correspondant de formule générale I dans laquelle $R_4'$ représente un groupe carboxyle

et/ou on transforme un composé obtenu de formule générale I dans laquelle $R_4'$ représente un groupe carboxyle, au moyen d'une estérification, en un composé correspondant de formule générale I dans laquelle $R_4'$ représente un groupe carbalcoxy avec en tout 2 à 8 atomes de carbone

et/ou on transforme un composé obtenu de formule générale I dans laquelle $R_2$ représente un groupe nitrophényle, au moyen d'une réduction, en un composé correspondant de formule générale I dans laquelle $R_2$ représente un groupe aminophényle

et/ou on transforme un composé obtenu de formule générale I dans laquelle $R_2$ représente un groupe aminophényle, au moyen d'une acétylation, en un composé correspondant de formule générale I dans laquelle $R_2$ représente un groupe acétamidophényle

et/ou on transforme un composé obtenu de formule générale I en ses sels physiologiquement supportables avec des acides minéraux et organiques ou également, dans le cas où $R_4'$ représente un groupe carboxyle, au moyen de bases minérales et organiques.

Priorité : 13.03.1979 (29 09 779.8).

3. Procédé selon les revendications 1 et 2, caractérisé en ce que la réaction a) est effectuée à des températures entre 0 et 200 °C, de préférence cependant à des températures entre la température ambiante et la température d'ébullition du solvant utilisé, par exemple à des températures entre 20 et 100 °C.

4. Procédé selon les revendications 1 et 2, caractérisé en ce que la réaction b) est effectuée dans une cétone de formule générale

$$R_5 - CO - CH_3 \qquad (V)$$

dans laquelle

$R_5$ est défini comme au début, en tant que solvant.

5. Procédé selon les revendications 1, 2 et 4, caractérisé en ce que la réaction b) est effectuée à des températures entre 0 °C et la température d'ébullition du solvant utilisé, par exemple à des températures entre 0 et 100 °C.

6. Procédé selon les revendications 1, 2, 4 et 5, caractérisé en ce qu'on prépare un composé de formule générale IV dans le mélange réactionnel par réaction d'une cétone correspondante de formule générale V avec du chloroforme en présence d'une base minérale.

7. Procédé selon les revendications 1 et 2, caractérisé en ce que l'alcoylation c) est effectuée à des températures entre 0 et 200 °C, de préférence cependant à des températures entre la température ambiante et la température d'ébullition du solvant utilisé, par exemple à des températures entre 20 et 100 °C.

8. Procédé selon la revendication 1, caractérisé en ce que la réaction d) est effectuée à la température d'ébullition du mélange réactionnel.

9. Procédé selon la revendication 1, caractérisé en ce que la réaction e) est effectuée à des températures entre 0 et 100 °C, de préférence cependant à des températures entre 20 et 50 °C.